# EUROPEAN PATENT APPLICATION

(11) **EP 4 831 199 A1**
(43) Date of publication of application: **28.10.2026**
(21) Application number: 24906261.3
(22) Date of filing: 13.12.2024
(51) Int. Cl.: C07K 16/28, A61K 47/68, A61K 45/00, A61P 35/00

(54) **IMMUNE-STIMULATING ANTIBODY CONJUGATE, PREPARATION METHOD THEREFOR, AND USE THEREOF**

(30) Priority: 22.12.2023 CN 202311788893
(71) Applicant: Sichuan Kelun-Biotech Biopharmaceutical Co., Ltd., Chengdu, Sichuan 611138 (CN)
(72) Inventor: LIU, Jinming, Chengdu, Sichuan 611138 (CN); REN, Yun, Chengdu, Sichuan 611138 (CN); HE, Ting, Chengdu, Sichuan 611138 (CN); KANG, Xiwei, Chengdu, Sichuan 611138 (CN); YUAN, Xiaoxi, Chengdu, Sichuan 611138 (CN); HAN, Zhu, Chengdu, Sichuan 611138 (CN); TIAN, Qiang, Chengdu, Sichuan 611138 (CN); SONG, Hongmei, Chengdu, Sichuan 611138 (CN); GE, Junyou, Chengdu, Sichuan 611138 (CN)
(74) Representative: Santarelli
(86) International application number: PCT/CN2024/139222
(87) International publication number: WO 2025/130780

(57) **Abstract**

Provided are an immune-stimulating antibody conjugate, a preparation method therefor, and a use thereof. Specifically provided are an immune-stimulating antibody conjugate as shown in formula (I), a preparation method therefor, and a use thereof in the preparation of a drug for treating and/or preventing cancer. Also provided are a drug linker as shown in formula (II) and a compound as shown in formula III-1, formula III-2, formula IV-1 or formula IV-2, a preparation method therefor, and a use thereof in the preparation of an immune-stimulating antibody conjugate.

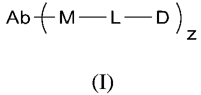

## Description

The present application claims priority to Chinese Application No. 202311788893.6, filed on December 22, 2023, which is incorporated herein by reference in its entirety.

### TECHNICAL FIELD

The present disclosure pertains to the field of pharmaceuticals and particularly relates to a class of immune-stimulating antibody conjugates, a preparation method therefor, a pharmaceutical composition thereof, and use thereof.

### BACKGROUND

STING (stimulator of interferon gene) is an important signaling molecule in immune responses. Upon stimulation and activation by ligands such as bacterial-derived cyclic dinucleotides (CDNs), it upregulates the IRF3 and NF-κB signaling pathways. Activated STING can recruit TBK1 in the cytoplasm and mediate the phosphorylation of IRF3 by TBK1, resulting in the generation of interferons and other cytokines, and it is extensively involved in various pathological and physiological processes such as tumor immunity, autoimmune inflammation, and autophagy.

At present, disclosed STING agonists can be divided into two main structural classes: cyclic dinucleotides and non-cyclic dinucleotides. Compared to conventional cyclic dinucleotide structures, non-cyclic dinucleotide STING agonists theoretically have superior stability and PK profiles, showing good application prospects in the pharmaceutical industry.

In antibody-drug conjugates (ADCs), antibodies are conjugated to small-molecule cytotoxic drugs by particular linkers. By using antibodies as carriers, ADCs enable targeted delivery of cytotoxic drugs to target cells, thereby reducing the systemic exposure of the cytotoxic drugs and improving safety. ADCs are a hot research topic in the area of targeted tumor therapy.

Based on ADC technology, a new class of agents known as immune-stimulating antibody conjugates (ISACs) has been developed. In ISACs, immunomodulators such as STING agonists are conjugated to antibodies by cleavable/non-cleavable linkers. By combining the precise location effects of antibodies with the long-lasting anti-tumor activity and immune memory effects of immunomodulators such as STING agonists, immune-stimulating antibody conjugates can safely elicit anti-tumor immune responses while enabling systemic administration.

### SUMMARY

In one aspect, the present disclosure provides a class of immune-stimulating antibody conjugates with non-cyclic dinucleotide STING agonists as payloads. The conjugates can efficiently exert the long-lasting anti-tumor activity and immune memory effects of the STING agonists through the precise location effects of antibodies, and have important significance for the prevention and treatment of tumors.

A first aspect of the present disclosure provides an immune-stimulating antibody conjugate of formula (I): 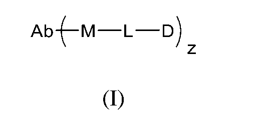 wherein
Ab is an antibody targeting a target antigen or an antigen-binding fragment thereof;
M is a linker part linking the antibody or the antigen-binding fragment thereof;
L is a linker linking M and D;
D is selected from structures of formula (D-I-1) and formula (D-I-2): 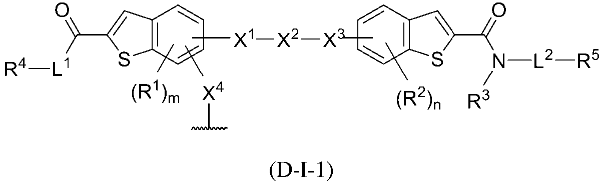 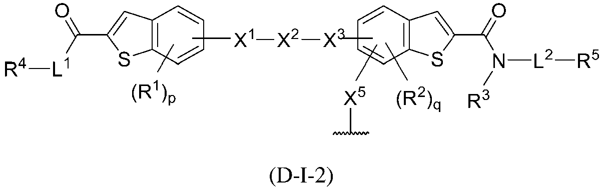
wherein 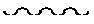 represents a point of attachment to L;
X¹ and X³ are identical or different and are each independently selected from a covalent bond, -O-, -S-, and -NR^{a}-;
X² is selected from C₁₋₆ alkylene, C₂₋₆ alkenylene, C₂₋₆ alkynylene, C₃₋₆ cycloalkyl, and 3-6 membered heterocyclyl, wherein the C₁₋₆ alkylene, C₂₋₆ alkenylene, C₂₋₆ alkynylene, C₃₋₆ cycloalkyl, and 3-6 membered heterocyclyl are each optionally substituted with one or more substituents independently selected from H, halogen, cyano, C₁₋₆ alkyl, -OR^{a}, and -NR^{a}R^{b};
X⁴ and X⁵ are each independently selected from -O- and -NR^{a}-;
L¹ is selected from a covalent bond and -(C(R⁶)₂)ⱼ-;
L² is selected from a covalent bond and -(C(R⁷)₂)ₖ-;
R¹ and R² are identical or different and are each independently selected from H, halogen, cyano, -OR^{a}, - NR^{a}R^{b}, -C(O)-OR^{a}, -C(O)-NR^{a}R^{b}, -NR^{a}-C(O)-R^{a}, C₁₋₆ alkyl, C₃₋₆ cycloalkyl, and 3-6 membered heterocyclyl, wherein the C₁₋₆ alkyl, C₃₋₆ cycloalkyl, and 3-6 membered heterocyclyl are each optionally substituted with one or more substituents independently selected from H, halogen, C₁₋₆ alkyl, -OR^{a}, and - NR^{a}R^{b};
R³ is selected from H and C₁₋₆ alkyl;
R⁴ is selected from H, -OR^{a}, -NR^{a}R^{b}, -C(O)-OR^{a}, -O-C(O)-R^{a}, -C(O)-NR^{a}R^{b}, -NR^{a}-C(O)-R^{a}, -O-C(O)-NR^{a}R^{b}, -NR^{a}-C(O)-OR^{a}, -NR^{a}-C(O)-NR^{a}R^{b}, C₁₋₆ alkyl, C₃₋₆ cycloalkyl, 3-6 membered heterocyclyl, C₆₋₁₀ aryl, and 5-10 membered heteroaryl, wherein the C₁₋₆ alkyl, C₃₋₆ cycloalkyl, 3-6 membered heterocyclyl, C₆₋₁₀ aryl, and 5-10 membered heteroaryl are each optionally substituted with one or more substituents independently selected from H, halogen, C₁₋₆ alkyl, -OR^{a}, -NR^{a}R^{b}, and -C(O)-OR^{a};
R⁵ is selected from H, C₁₋₆ alkyl, C₃₋₁₀ cycloalkyl, 3-10 membered heterocyclyl, -OR^{a}, and -C(O)-OR^{a}, wherein the C₁₋₆ alkyl, C₃₋₁₀ cycloalkyl, and 3-10 membered heterocyclyl are each optionally substituted with one or more substituents independently selected from H, halogen, C₁₋₆ alkyl, -OR^{a}, -NR^{a}R^{b}, and - C(O)-OR^{a};
each R⁶ is independently selected from H, -OR^{a}, -NR^{a}R^{b}, C₁₋₆ alkyl, C₂₋₆ alkenyl, C₂₋₆ alkynyl, C₃₋₆ cycloalkyl, and 3-6 membered heterocyclyl, wherein the C₁₋₆ alkyl, C₂₋₆ alkenyl, C₂₋₆ alkynyl, C₃₋₆ cycloalkyl, and 3-6 membered heterocyclyl are each optionally substituted with one or more substituents independently selected from H, halogen, C₁₋₆ alkyl, -OR^{a}, -NR^{a}R^{b}, and -C(O)-OR^{a}; or
two R⁶ groups on different carbon atoms, together with the carbon atoms therebetween, form C₃₋₆ cycloalkyl or 3-6 membered heterocyclyl; or
two R⁶ groups on the same carbon atom, together with the carbon atom to which they are both attached, form C₃₋₆ cycloalkyl or 3-6 membered heterocyclyl;
each R⁷ is independently selected from H, -OR^{a}, -NR^{a}R^{b}, C₁₋₆ alkyl, C₂₋₆ alkenyl, C₂₋₆ alkynyl, C₃₋₆ cycloalkyl, and 3-6 membered heterocyclyl, wherein the C₁₋₆ alkyl, C₂₋₆ alkenyl, C₂₋₆ alkynyl, C₃₋₆ cycloalkyl, and 3-6 membered heterocyclyl are each optionally substituted with one or more substituents independently selected from H, halogen, C₁₋₆ alkyl, -OR^{a}, -NR^{a}R^{b}, and -C(O)-OR^{a}; or
two R⁷ groups on the same carbon atom, together with the carbon atom to which they are both attached, form C₃₋₆ cycloalkyl or 3-6 membered heterocyclyl; or any one R⁷ and R³, together with the atoms therebetween, form 3-10 membered heterocyclyl;
R^{a} and R^{b} are each independently selected from H, C₁₋₆ alkyl, C₃₋₆ cycloalkyl, and 3-6 membered heterocyclyl, wherein the C₁₋₆ alkyl, C₃₋₆ cycloalkyl, and 3-6 membered heterocyclyl are each optionally substituted with one or more substituents independently selected from H, halogen, C₁₋₆ alkyl, hydroxy, amino, and carboxyl;
j and k are each independently selected from 1, 2, and 3;
m and q are each independently selected from 0, 1, and 2;
n and p are each independently selected from 0, 1, 2, and 3;
z is selected from 1-10.

A second aspect of the present disclosure provides an immune-stimulating antibody conjugate selected from the following structures: 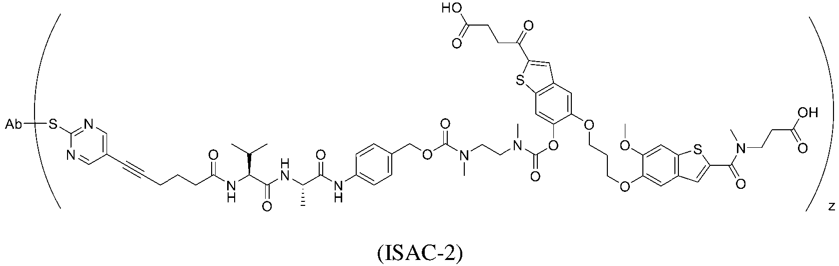 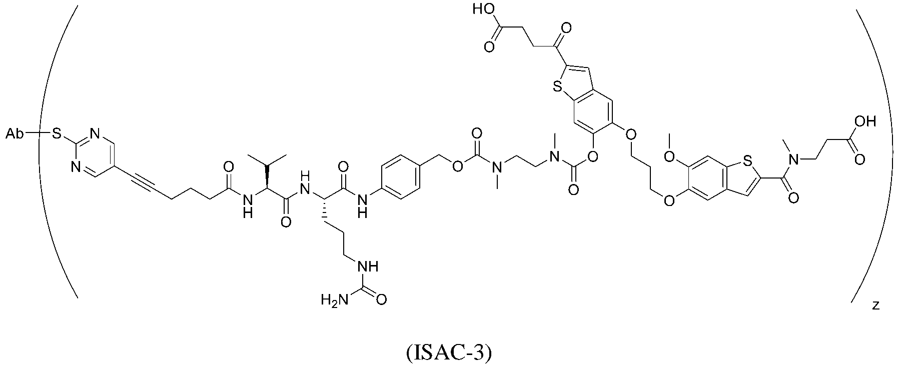 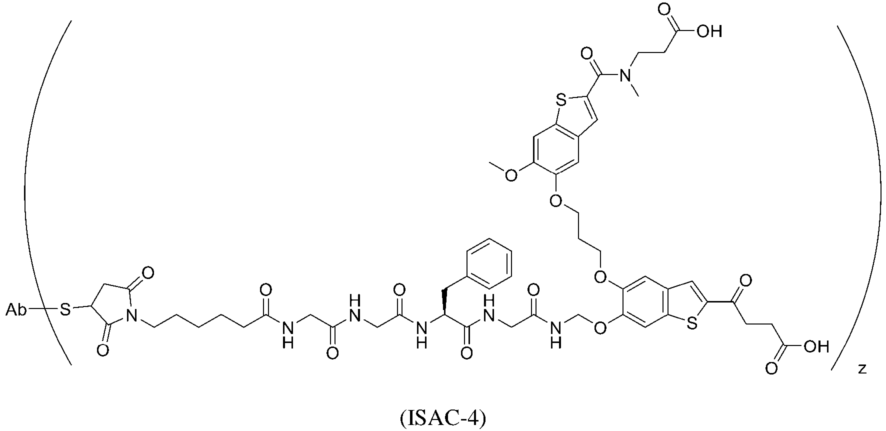
each z is selected from 1 to 10;
Ab is selected from an anti-Her-2 antibody.

A third aspect of the present disclosure provides a drug-linker compound of formula (II) or a pharmaceutically acceptable salt, ester, stereoisomer, tautomer, polymorph, solvate, N-oxide, isotopically labeled compound, metabolite, or prodrug thereof:

M¹-L-D (II)

wherein
M¹ is selected from 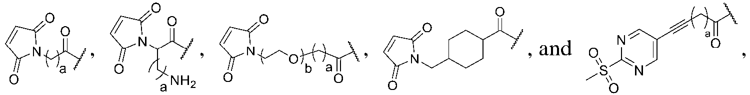
wherein each a is independently selected from integers of 1-6, and b is selected from integers of 1-10;
L and D are as defined above.

A fourth aspect of the present disclosure provides a compound represented by formula III-1 or III-2 or a pharmaceutically acceptable salt, ester, stereoisomer, tautomer, polymorph, solvate, N-oxide, isotopically labeled compound, metabolite, or prodrug thereof: 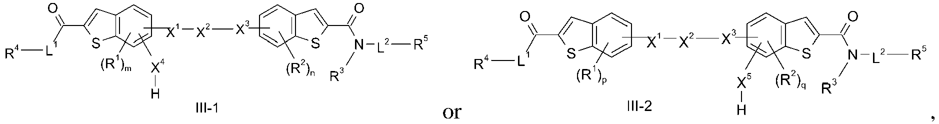 wherein R¹, R², R³, R⁴, R⁵, L¹, L², X¹, X², X³, X⁴, X⁵, m, n, p, and q are as defined above.

A fifth aspect of the present disclosure provides a compound represented by formula IV-1 or IV-2 or a pharmaceutically acceptable salt, ester, stereoisomer, tautomer, polymorph, solvate, N-oxide, isotopically labeled compound, metabolite, or prodrug thereof: 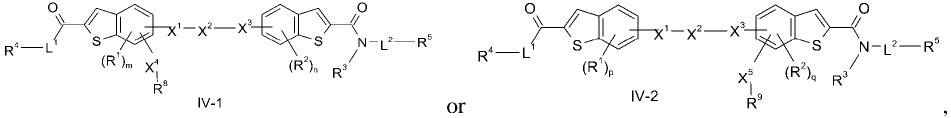
wherein R¹, R², R³, R⁴, R⁵, L¹, L², X¹, X², X³, X⁴, X⁵, m, n, p, and q are as defined above;
R⁸ and R⁹ are each independently selected from -C₁₋₆ alkylene-NR¹²-C(=O)-C₁₋₆ alkylene-NR¹⁰R¹¹, - C(=O)O-C₆₋₁₀ aryl, -C(=O)NR¹²-C₁₋₆ alkylene-NR¹⁰R¹¹, -C₁₋₆ alkylene-O-C₁₋₆ alkyl, and -C₁₋₆ alkylene-C(=O)-C₆₋₁₀ aryl, wherein the aryl is optionally substituted with amino or nitro;
R¹⁰ and R¹¹ are each independently selected from H, C₁₋₆ alkyl, Fmoc, and Boc;
R¹² is selected from H and C₁₋₆ alkyl.

A sixth aspect of the present disclosure provides a composition of an immune-stimulating antibody conjugate, comprising the immune-stimulating antibody conjugate described above, wherein the composition has a DAR value of 1.0-10.0.

A seventh aspect of the present disclosure provides a pharmaceutical composition comprising a prophylactically and/or therapeutically effective amount of the immune-stimulating antibody conjugate according to the first or second aspect of the present disclosure, the drug-linker compound according to the third aspect of the present disclosure or the compound or the pharmaceutically acceptable salt, ester, stereoisomer, tautomer, polymorph, solvate, N-oxide, isotopically labeled compound, metabolite, or prodrug thereof according to the fourth or fifth aspect of the present disclosure, or the composition of the immune-stimulating antibody conjugate according to the sixth aspect of the present disclosure, and one or more pharmaceutically acceptable auxiliary materials.

An eighth aspect of the present disclosure provides use of the immune-stimulating antibody conjugate of formula (I) according to the first or second aspect of the present disclosure, the drug-linker compound according to the third aspect of the present disclosure or the compound or the pharmaceutically acceptable salt, ester, stereoisomer, tautomer, polymorph, solvate, N-oxide, isotopically labeled compound, metabolite, or prodrug thereof according to the fourth or fifth aspect of the present disclosure, the composition of the immune-stimulating antibody conjugate according to the sixth aspect of the present disclosure, or the pharmaceutical composition according to the seventh aspect of the present disclosure, in the preparation of a medicament for treating and/or preventing cancer (e.g., HER2-positive cancer, such as HER2-positive gastric cancer, breast cancer, or non-small cell lung cancer).

A ninth aspect of the present disclosure provides the immune-stimulating antibody conjugate of formula (I) according to the first or second aspect of the present disclosure, the drug-linker compound according to the third aspect of the present disclosure or the compound or the pharmaceutically acceptable salt, ester, stereoisomer, tautomer, polymorph, solvate, N-oxide, isotopically labeled compound, metabolite, or prodrug thereof according to the fourth or fifth aspect of the present disclosure, the composition of the immune-stimulating antibody conjugate according to the sixth aspect of the present disclosure, or the pharmaceutical composition according to the seventh aspect of the present disclosure, for use in the treatment and/or prevention of cancer (e.g., HER2-positive cancer, such as HER2-positive gastric cancer, breast cancer, or non-small cell lung cancer).

A tenth aspect of the present disclosure provides a method for treating and/or preventing cancer (e.g., HER2-positive cancer, such as HER2-positive gastric cancer, breast cancer, or non-small cell lung cancer), comprising administering to an individual in need thereof a therapeutically and/or prophylactically effective amount of the immune-stimulating antibody conjugate of formula (I) according to the first or second aspect of the present disclosure, the drug-linker compound according to the third aspect of the present disclosure or the compound or the pharmaceutically acceptable salt, ester, stereoisomer, tautomer, polymorph, solvate, N-oxide, isotopically labeled compound, metabolite, or prodrug thereof according to the fourth or fifth aspect of the present disclosure, the composition of the immune-stimulating antibody conjugate according to the sixth aspect of the present disclosure, or the pharmaceutical composition according to the seventh aspect of the present disclosure.

An eleventh aspect of the present disclosure provides use of the drug-linker compound according to the third aspect of the present disclosure or the compound or the pharmaceutically acceptable salt, ester, stereoisomer, tautomer, polymorph, solvate, *N*-oxide, isotopically labeled compound, metabolite, or prodrug thereof according to the fourth or fifth aspect of the present disclosure in the preparation of an antibody-drug conjugate (e.g., the immune-stimulating antibody conjugate of the present disclosure).

### Definitions

Unless otherwise defined below, all technical and scientific terms used herein are intended to have the same meaning as commonly understood by those skilled in the art. Reference to the techniques used herein is intended to refer to techniques commonly understood in the art, including those variations of or equivalent alternatives to the techniques that are apparent to those skilled in the art. Although the following terms are believed to be well understood by those skilled in the art, the following definitions are set forth to better explain the present disclosure.

The term "antibody" refers to an immunoglobulin molecule that generally consists of two pairs of polypeptide chains, each pair having one light chain (LC) and one heavy chain (HC). Antibody light chains can be classified into κ (kappa) and λ (lambda) light chains. Heavy chains can be classified into µ, δ, γ, α, or ε, and isotypes of antibodies are defined as IgM, IgD, IgG, IgA, and IgE. In light chains and heavy chains, the variable region and constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain-CL. The constant domains are not directly involved in the binding of the antibody to the antigen, but exhibit a variety of effector functions, such as being able to mediate the binding of an immunoglobulin to host tissues or factors, including various cells of the immune system (e.g., effector cells) and the first component of the classical complement system (C1q). The VH and VL regions can be further subdivided into hypervariable regions (called complementarity determining regions (CDRs)) between which conservative regions called framework regions (FRs) are distributed. Each VH and VL consists of 3 CDRs and 4 FRs arranged from the amino-terminus to the carboxy-terminus in the following order: FR1, CDR1, FR2, CDR2, FR3, CDR3, FR4. The variable regions (VH and VL) of each heavy chain/light chain pair form an antigen-binding site. The assignment of amino acids in each region or domain can follow various numbering systems known in the art.

The term "complementarity determining region" or "CDR" refers to the amino acid residues in the variable regions of an antibody that are responsible for antigen binding. The variable regions of the heavy and light chains each contain three CDRs, designated CDR1, CDR2, and CDR3. The precise boundaries of these CDRs can be defined according to various numbering systems known in the art, for example, according to the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), the IMGT numbering system (Lefranc et al., Dev. Comparat. Immunol. 27:55-77, 2003), or the AbM numbering system (Martin ACR, Cheetham JC, Rees AR (1989) Modelling antibody hypervariable loops: A combined algorithm. Proc Natl Acad Sci USA 86:9268-9272). For a given antibody, those skilled in the art will readily identify CDRs defined by these numbering systems. Moreover, the correspondence between different numbering systems is well known to those skilled in the art (see, e.g., Lefranc et al., Dev. Comparat. Immunol. 27:55-77, 2003).

In the present disclosure, the CDRs contained in an antibody or an antigen-binding fragment thereof can be determined according to various numbering systems known in the art, for example, according to the Kabat, Chothia, IMGT, or AbM numbering system.

The term "framework region" or "FR" residues refers to those amino acid residues in the variable regions of an antibody that are not the CDR residues defined above.

The term "antigen-binding fragment", also known as "antigen-binding moiety", of an antibody refers to a polypeptide of a fragment of the antibody, e.g., a polypeptide of a fragment of a full-length antibody, that retains the ability to specifically bind to the same antigen as the full-length antibody does, and/or competes with the full-length antibody for specifically binding to the antigen. See generally, Fundamental Immunology, Ch. 7 (Paul, W., ed., 2nd Edition, Raven Press, N.Y. (1989), which is incorporated herein by reference in its entirety for all purposes. The antigen-binding fragment of the antibody can be produced by recombinant DNA technology or by enzymatic or chemical cleavage of an intact antibody. Nonlimiting examples of antigen-binding fragments include Fab fragments, Fab' fragments, F(ab)'₂ fragments, F(ab)'₃ fragments, Fd, Fv, scFv, di-scFv, (scFv)₂, disulfide-stabilized Fv proteins ("dsFv"), single-domain antibodies (sdAbs, nanobodies), and polypeptides that comprise at least a portion of an antibody that is sufficient to impart specific antigen-binding ability to the polypeptides. A review of engineered antibody variants is found in Holliger et al., 2005; Nat Biotechnol, 23: 1126-1136.

The term "Fd" means an antibody fragment consisting of a VH domain and a CH1 domain; the term "dAb fragment" means an antibody fragment consisting of a VH domain (Ward et al., Nature 341:544 546 (1989)); the term "Fab fragment" means an antibody fragment consisting of a VL domain, a VH domain, a CL domain, and a CH1 domain; the term "F(ab')₂ fragment" means an antibody fragment comprising two Fab fragments linked by a disulfide bridge in the hinge region; the term "Fab' fragment" means a fragment obtained by reducing the disulfide bond linking two heavy chain fragments in a F(ab')₂ fragment, and the fragment consists of an intact light chain and a heavy chain Fd fragment (consisting of a VH domain and a CH1 domain).

The term "Fv" means an antibody fragment consisting of the VL and VH domains of a single arm of an antibody. An Fv fragment is generally considered to be the smallest antibody fragment that can form an intact antigen-binding site. It is generally believed that the six CDRs impart antigen-binding specificity to the antibody. However, even one variable region (e.g., an Fd fragment, which contains only three CDRs specific to an antigen) is capable of recognizing and binding to an antigen, although its affinity may be lower than an intact binding site.

The term "Fc" means an antibody fragment formed by binding a second constant region and a third constant region of a first heavy chain of an antibody and a second constant region and a third constant region of a second heavy chain of the antibody via a disulfide bond. The Fc fragment of an antibody has a variety of different functions, but is not involved in antigen binding.

The term "scFv" refers to a single polypeptide chain comprising a VL domain and a VH domain, wherein the VL and VH are linked by a linker (see, e.g., Bird et al., Science 242:423-426 (1988); Huston et al., Proc. Natl. Acad. Sci. USA 85:5879-5883 (1988); and Pluckthun, The Pharmacology of Monoclonal Antibodies, Volume 113, Roseburg and Moore ed., Springer-Verlag, New York, pages 269-315 (1994)). Such scFv molecules may have the following general structure: NH₂-VL-linker-VH-COOH or NH₂-VH-linker-VL-COOH. An appropriate linker in the prior art consists of a repeated GGGGS amino acid sequence or a variant thereof. For example, a linker having the amino acid sequence (GGGGS)₄ may be used, and variants thereof may also be used (Holliger et al. (1993), Proc. Natl. Acad. Sci. USA, 90: 6444-6448). Other linkers that may be used in the present disclosure are described in Alfthan et al. (1995), Protein Eng., 8:725-731; Choi et al. (2001), Eur. J. Immunol., 31: 94-106; Hu et al. (1996), Cancer Res., 56:3055-3061; Kipriyanov et al. (1999), J. Mol. Biol., 293:41-56; and Roovers et al. (2001), Cancer Immunol. In some cases, a disulfide bond may also be present between the VH and VL of the scFv. In certain embodiments, the VH and VL domains may be situated relative to one another in any suitable arrangement. An example is an scFv comprising NH₂-VH-VH-COOH or NH₂VL-VL-COOH.

Each of the above antibody fragments retains the ability to specifically bind to the same antigen as the full-length antibody does, and/or competes with the full-length antibody for specifically binding to the antigen.

Herein, unless otherwise clearly defined in the context, when referring to the term "antibody", it includes not only intact antibodies but also antigen-binding fragments of antibodies.

Antigen-binding fragments (e.g., the above antibody fragments) of antibodies can be obtained from given antibodies (e.g., the antibodies provided by the present disclosure) by using conventional techniques known to those skilled in the art (e.g., recombinant DNA technology or enzyme catalysis or chemical cleavage), and the antigen-binding fragments of the antibodies are screened for specificity in a way identical to the way for intact antibodies.

The term "murine antibody" refers to an antibody obtained by fusing B cells and myeloma cells of an immunized mouse, selecting murine hybrid fusion cells that are capable of both infinite proliferation and antibody secretion, and then performing screening, antibody preparation, and antibody purification, or refers to an antibody secreted by plasma cells formed by differentiation and proliferation of B cells after an antigen invades a mouse.

The term "humanized antibody" refers to a genetically engineered non-human antibody that has an amino acid sequence modified to increase the homology to the sequence of a human antibody. Generally, all or part of the CDR regions of a humanized antibody are derived from a non-human antibody (donor antibody), and all or part of the non-CDR regions (e.g., variable region FRs and/or constant regions) are derived from a human immunoglobulin (receptor antibody). The humanized antibody generally retains the desired properties of the donor antibody, including but not limited to antigen specificity, affinity, reactivity, the ability to increase the activity of immune cells, the ability to enhance the immune response, etc. The donor antibody may be a mouse, rat, rabbit, or non-human primate (e.g., cynomolgus monkey) antibody having a desired property (e.g., antigen specificity, affinity, reactivity, the ability to increase the activity of immune cells, and/or the ability to enhance the immune response).

The twenty conventional amino acids involved herein are written in accordance with conventional usage. See, e.g., Immunology-A Synthesis (2nd Edition, E. S. Golub and D. R. Gren, Eds., Sinauer Associates, Sunderland, Mass. (1991)), which is incorporated herein by reference. In the present disclosure, amino acids are generally represented by single-letter and three-letter abbreviations known in the art. For example, alanine can be represented by A or Ala.

As used herein, the term "include", "comprise", "have", "contain", or "involve", as well as other variant forms thereof herein, is inclusive or open-ended and does not exclude other unrecited elements or method steps.

As used herein, the term "alkyl" is defined as a linear or branched saturated aliphatic hydrocarbon group. For example, as used herein, the term "C₁₋₆ alkyl" refers to a linear or branched group having 1 to 6 carbon atoms (e.g., methyl, ethyl, n-propyl, isopropyl, n-butyl, isobutyl, sec-butyl, tert-butyl, n-pentyl, or n-hexyl), and it is optionally substituted with one or more (such as 1 to 3) suitable substituents, such as halogens. The term "C₁₋₃ alkyl" refers to a linear or branched group having 1 to 3 carbon atoms (e.g., methyl, ethyl, n-propyl, or isopropyl), and it is optionally substituted with one or more (such as 1 to 3) suitable substituents, such as halogens.

As used herein, the term "alkoxy" refers to "alkyl-O-", and the "alkyl" is as defined above. For example, the term "C₁₋₆ alkoxy" refers to "C₁₋₆ alkyl-O-", and the "C₁₋₆ alkyl" is as defined above. The "C₁₋₃ alkoxy" described in the present disclosure refers to "C₁₋₃ alkyl-O-", and the "C₁₋₃ alkyl" is as defined above. Examples of C₁₋₆ alkoxy include methoxy, ethoxy, n-propoxy, isopropoxy, n-butoxy, isobutoxy, sec-butoxy, tert-butoxy, n-pentoxy, or n-hexoxy.

The "C₁₋₆ alkylene" described in the present disclosure refers to a divalent group formed by removing two hydrogen atoms from a linear or branched alkane containing 1-6 carbon atoms, including "C₁₋₅ alkylene", "C₁₋₄ alkylene", "C₁₋₃ alkylene", and "C₁₋₂ alkylene"; specific examples include, but are not limited to -CH₂-, -CH₂CH₂-, -CH₂CH₂CH₂-, -CH₂CH₂CH₂CH₂-, -CH₂CH₂CH₂CH₂CH₂-, - CH₂CH₂CH₂CH₂CH₂CH₂-, etc.

As used herein, the term "alkenyl" refers to a linear or branched aliphatic hydrocarbon group having one or more carbon-carbon double bonds. For example, the term "C₂₋₆ alkenyl" as used herein refers to an alkenyl group having 2-6 carbon atoms and one, two, or three carbon-carbon double bonds (e.g., ethenyl, 1-propenyl, 2-propenyl, 2-butenyl, 3-butenyl, 2-pentenyl, 3-pentenyl, 4-pentenyl, 2-hexenyl, 3-hexenyl, 4-hexenyl, 5-hexenyl, 2-methyl-2-propenyl, or 4-methyl-3-pentenyl), and it is optionally substituted with one or more (e.g., 1-3) substituents described herein.

The "C₂₋₆ alkenylene" described in the present disclosure refers to a divalent group formed by removing two hydrogen atoms from a linear or branched aliphatic hydrocarbon having one or more carbon-carbon double bonds.

As used herein, the term "alkynyl" refers to a linear or branched aliphatic hydrocarbon group having one or more carbon-carbon triple bonds. For example, the term "C₂₋₆ alkynyl" as used herein refers to an alkynyl group having 2-6 carbon atoms and one, two, or three carbon-carbon triple bonds (e.g., ethynyl, 1-propynyl, 2-propynyl, 2-butynyl, 3-butynyl, 2-pentynyl, 3-pentynyl, 4-pentynyl, 2-hexynyl, 3-hexynyl, 4-hexynyl, or 5-hexynyl), and it is optionally substituted with one or more (e.g., 1-3) substituents described herein.

The "C₂₋₆ alkynylene" described in the present disclosure refers to a divalent group formed by removing two hydrogen atoms from a linear or branched aliphatic hydrocarbon having one or more carbon-carbon triple bonds.

As used herein, the term "cycloalkyl" refers to a saturated or partially unsaturated non-aromatic monocyclic or polycyclic (such as bicyclic) hydrocarbon ring (e.g., a monocyclic ring, such as cyclopropyl, cyclobutyl, cyclopentyl, cyclohexyl, cycloheptyl, cyclooctyl, or cyclononyl, or a bicyclic ring, including spiro-ring, fused, or bridged systems, such as bicyclo[1.1.1]pentyl, bicyclo[2.2.1]heptyl, bicyclo[3.2.1]octyl, bicyclo[5.2.0]nonyl, or decahydronaphthyl), and it is optionally substituted with one or more (such as 1 to 3) suitable substituents. For example, the term "C₃₋₆ cycloalkyl" refers to a saturated or partially unsaturated non-aromatic monocyclic or polycyclic (such as bicyclic) hydrocarbon ring having 3 to 6 ring-forming carbon atoms (e.g., cyclopropyl, cyclobutyl, cyclopentyl, or cyclohexyl), and it is optionally substituted with one or more (such as 1 to 3) suitable substituents, for example, cyclopropyl substituted with methyl.

As used herein, the term "halogen" group is defined to include fluorine, chlorine, bromine, or iodine.

As used herein, the term "halo" refers to substitution with one or more (such as 1 to 3) identical or different halogen atoms.

As used herein, the term "haloalkyl" refers to an alkyl group substituted with one or more (such as 1 to 3) identical or different halogen atoms. For example, the term "C₁₋₆ haloalkyl" refers to a haloalkyl group having 1 to 6 carbon atoms, e.g., -CF₃, -C₂F₅, -CHF₂, -CH₂F, -CH₂CF₃, -CH₂Cl, or -CH₂CH₂CF₃.

As used herein, the term "heterocycle" or "heterocyclyl" refers to a saturated or partially unsaturated non-aromatic monocyclic or polycyclic group, for example, having 2, 3, 4, 5, 6, 7, 8, or 9 carbon atoms and one or more (e.g., 1, 2, 3, or 4) heteroatoms independently selected from N, O, and S(O)ₜ (wherein t is 0, 1, or 2) in the ring(s), e.g., 3-12 membered heterocyclyl, 3-7 membered heterocyclyl, 3-6 membered heterocyclyl, or 5-6 membered heterocyclyl, e.g., 5-10 membered nitrogen-containing heterocycyl, 6-10 membered oxygen-containing heterocycyl, 6-8 membered sulfur-containing heterocycyl, or 5-8 membered oxygen-containing heterocycyl. Representative examples of heterocyclyl include, but are not limited to, oxiranyl, aziridinyl, azetidinyl, oxetanyl, tetrahydrofuranyl, tetrahydropyrrolidinyl, hexahydro-1*H*-pyrroline, pyrrolidinonyl, imidazolidinyl, pyrazolidinyl, tetrahydropyranyl, tetrahydropyridinyl, piperidinyl, morpholinyl, dithianyl, thiomorpholinyl, piperazinyl, etc.

As used herein, the term "aryl" or "aromatic ring" refers to an all-carbon monocyclic or fused polycyclic aromatic group having a conjugated π-electron system. For example, the term "C₆₋₁₀ aryl" or "C₆₋₁₀ aromatic ring" refers to an aromatic group containing 6 to 10 carbon atoms, such as phenyl (ring) or naphthyl (ring). Aryl is optionally substituted with one or more (such as 1 to 3) suitable substituents (e.g., halogen, -OH, -CN, -NO₂, or C₁₋₆ alkyl).

Herein, the term "heteroaryl" refers to an aromatic cyclic group, wherein at least one ring atom is a heteroatom, such as a nitrogen atom, an oxygen atom, or a sulfur atom. Optionally, a ring atom (e.g., a carbon atom, a nitrogen atom, or a sulfur atom) in the cyclic structure may be substituted with oxo. Specific examples include, but are not limited to, 5-10 membered heteroaryl, 5-10 membered nitrogen-containing heteroaryl, 6-10 membered oxygen-containing heteroaryl, 6-8 membered nitrogen-containing heteroaryl, 5-8 membered oxygen-containing heteroaryl, etc., such as furanyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridinyl, 2-pyridonyl, 4-pyridonyl, pyrimidinyl, 1,4-dioxacyclohexadienyl, 2*H*-1,2-oxazinyl, 4*H*-1,2-oxazinyl, 6*H-*1,2-oxazinyl, 4*H*-1,3-oxazinyl, 6*H*-1,3-oxazinyl, 4*H*-1,4-oxazinyl, pyridazinyl, pyrazinyl, 1,2,3-triazinyl, 1,3,5-triazinyl, 1,2,4,5-tetrazinyl, azepinyl, 1,3-diazepinyl, azocinyl, etc.

Optionally, the hydrogen in the groups involved in the present disclosure may be substituted with deuterium.

The term "substituted" means that one or more (e.g., 1, 2, 3, or 4) hydrogen atoms on a specified atom are replaced with a selection from the indicated group, provided that the normal valency of the specified atom under the present circumstances is not exceeded and that the replacement results in a stable compound. A combination of substituents and/or variables is permissible only if the combination results in a stable compound.

If a substituent is described as being "optionally substituted with...", the substituent may be (1) unsubstituted or (2) substituted. If a carbon atom of a substituent is described as being optionally substituted with one or more of a list of substituents, then one or more hydrogen atoms on the carbon atom (to the extent of any hydrogen atom present) may be replaced individually and/or together with an independently selected substituent or not replaced. If a nitrogen atom of a substituent is described as being optionally substituted with one or more of a list of substituents, then one or more hydrogen atoms on the nitrogen atom (to the extent of any hydrogen atom present) may each be replaced with an independently selected substituent or not replaced.

If substituents are described as being "independently selected from" one set of groups, each substituent is selected independently of another. Thus, each substituent may be identical to or different from another (other) substituent.

As used herein, the term "one or more" means one or more than one, such as 2, 3, 4, 5, 6, 7, 8, 9, or 10, under reasonable conditions.

Unless otherwise specified, as used herein, the point of attachment of a substituent may be from any suitable position of the substituent.

When a bond of a substituent is shown to pass through a bond connecting two atoms in a ring, the substituent may be bonded to any ring-forming atom in the substitutable ring.

The present disclosure also includes all pharmaceutically acceptable isotopically labeled compounds, which are identical to the compounds of the present disclosure, except that one or more atoms are replaced by an atom having the same atomic number, but an atomic mass or mass number different from the atomic mass or mass number predominant in nature. Examples of isotopes suitable for incorporation into the compounds of the present disclosure include, but are not limited to, isotopes of hydrogen (e.g., ²H, ³H, deuterium D, and tritium T); isotopes of carbon (e.g., ¹¹C, ¹³C, and ¹⁴C); isotopes of chlorine (e.g., ³⁷Cl); isotopes of fluorine (e.g., ¹⁸F); isotopes of iodine (e.g., ¹²³I and ¹²⁵I); isotopes of nitrogen (e.g., ¹³N and ¹⁵N); isotopes of oxygen (e.g., ¹⁵O, ¹⁷O, and ¹⁸O); isotopes of phosphorus (e.g., ³²P); and isotopes of sulfur (e.g., ³⁵S). Certain isotopically labeled compounds of the present disclosure (e.g., those into which a radioactive isotope is incorporated) can be used in drug and/or substrate tissue distribution studies (e.g., analysis). The radioactive isotopes tritium (i.e., ³H) and carbon-14 (i.e., ¹⁴C) are particularly useful for this purpose because they can be easily incorporated and easily detected. Substitution with positron emitting isotopes (e.g., ¹¹C, ¹⁸F, ¹⁵O, and ¹³N) can be used to examine substrate receptor occupancy in positron emission tomography (PET) studies. The isotopically labeled compounds of the present disclosure can be prepared by processes analogous to those described in the accompanying routes and/or in the examples and preparations by using an appropriate isotopically labeled reagent in place of the non-labeled reagent previously employed. The pharmaceutically acceptable solvates of the present disclosure include those in which the crystallization solvent can be isotopically substituted, e.g., D₂O, acetone-*d*₆, or DMSO-*d*₆.

The term "stereoisomer" refers to an isomer formed due to at least one asymmetric center. In compounds having one or more (e.g., 1, 2, 3, or 4) asymmetric centers, racemic mixtures, single enantiomers, mixtures of diastereoisomers, and individual diastereoisomers may be produced. Particular individual molecules may also present as geometric isomers (cis/trans). Similarly, the compounds of the present disclosure may present as mixtures of two or more structurally different forms in rapid equilibrium (commonly referred to as tautomers). Representative examples of tautomers include keto-enol tautomers, phenol-keto tautomers, nitroso-oxime tautomers, imine-enamine tautomers, etc. It will be appreciated that the scope of the present application encompasses all such isomers or mixtures thereof in any proportion (e.g., 60%, 65%, 70%, 75%, 80%, 85%, 90%, 95%, 96%, 97%, 98%, or 99%).

The present disclosure encompasses all possible crystalline forms or polymorphs of the compounds of the present disclosure, which may be single polymorphs or mixtures of more than one polymorph in any ratio.

It will also be appreciated that certain compounds of the present disclosure can be present in free form for treatment or, where appropriate, in the form of a pharmaceutically acceptable derivative thereof. In the present disclosure, pharmaceutically acceptable derivatives include, but are not limited to, pharmaceutically acceptable salts, solvates, metabolites, or prodrugs; upon administration to a patient in need thereof, they are capable of providing, directly or indirectly, the compounds of the present disclosure or metabolites or residues thereof. Thus, when reference is made herein to "the compounds of the present disclosure", it is also intended to encompass the various derivative forms of the compounds described above.

The pharmaceutically acceptable salts of the compounds of the present disclosure include acid addition salts and base addition salts thereof. Suitable acid addition salts are formed from acids that form pharmaceutically acceptable salts. Suitable base addition salts are formed from bases that form pharmaceutically acceptable salts. A review of suitable salts is found in Stahl and Wermuth, "Handbook of Pharmaceutical Salts: Properties, Selection, and Use" (Wiley-VCH, 2002). Methods for preparing the pharmaceutically acceptable salts of the compounds of the present disclosure are known to those skilled in the art.

The compounds of the present disclosure may be present in the form of a solvate (preferably a hydrate), and the compounds of the present disclosure contain polar solvents as a structural element of the crystal lattice of the compounds. The amount of polar solvents, particularly water, may be present in a stoichiometric or non-stoichiometric ratio.

Those skilled in the art will appreciate that not all nitrogen-containing heterocyclic rings are capable of forming *N*-oxides, as nitrogen requires an available lone pair of electrons for oxidation to the oxides; those skilled in the art will recognize nitrogen-containing heterocyclic rings capable of forming *N*-oxides. Those skilled in the art will also recognize that tertiary amines are capable of forming *N*-oxides. Synthesis methods for preparing *N*-oxides of heterocycles and tertiary amines are well known to those skilled in the art and include the oxidation of heterocycles and tertiary amines with peroxy acids such as peroxyacetic acid and m-chloroperoxybenzoic acid (MCPBA), hydrogen peroxide, alkyl hydroperoxides such as tert-butyl hydroperoxide, sodium perborate, and dioxiranes such as dimethyldioxirane. These methods for preparing N-oxides have been extensively described and reviewed in the literature. See, e.g., T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

Also included within the scope of the present disclosure are metabolites of the compounds of the present disclosure, i.e., substances formed *in vivo* upon administration of the compounds of the present disclosure. Such products may result, for example, from the oxidation, reduction, hydrolysis, amidation, deamidation, esterification, enzymolysis, etc. of the administered compound. Thus, the present disclosure includes metabolites of the compounds of the present disclosure, including compounds prepared by the process of contacting the compounds of the present disclosure with a mammal for a time sufficient to produce metabolites thereof.

Further included within the scope of the present disclosure are prodrugs of the compounds of the present disclosure, which are certain derivatives of the compounds of the present disclosure that may themselves have little or no pharmacological activity and, when administered into or onto the body, can be converted to the compounds of the present disclosure having the desired activity, for example, by hydrolysis. Generally, such prodrugs will be functional group derivatives of the compounds, which are readily converted into desired therapeutically active compounds *in vivo.* Additional information on the use of prodrugs can be found in "Pro-drugs as Novel Delivery Systems", volume 14, ACS Symposium Series (T. Higuchi and V. Stella) and "Bioreversible Carriers in Drug Design", Pergamon Press, 1987 (E. B. Roche eds., American Pharmaceutical Association). The prodrugs of the present disclosure may be prepared by, for example, replacing appropriate functional groups present in the compounds of the present disclosure with certain moieties known to those skilled in the art as "pro-moieties", for example, as described in "Design of Prodrugs", H. Bundgaard (Elsevier, 1985).

The present disclosure further encompasses the compounds of the present disclosure containing a protective group. In any process of preparing the compounds of the present disclosure, it may be necessary and/or desirable to protect sensitive or reactive groups on any of the molecules concerned, thereby forming a chemically protected form of the compounds of the present disclosure. This can be achieved by conventional protective groups, as described, for example, in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, which are incorporated herein by reference. Protective groups can be removed at an appropriate subsequent stage using methods known in the art.

The term "about" means being within ±10%, preferably within ±5%, and more preferably within ±2%, of the stated numerical value.

The term "conjugate" refers to a substance obtained by linking a small-molecule drug to a targeting moiety. In some embodiments of the present disclosure, the small-molecule drug is linked to the targeting moiety by a linker. The linker can be cleaved in a particular environment (e.g., an intracellular low-pH environment or a slightly acidic tumor microenvironment) or under a particular action (e.g., the action of a lysosomal protease), thereby separating the small-molecule drug from the targeting moiety.

The term "linker" refers to a fragment that links a small-molecule drug and a targeting moiety.

The term "targeting moiety" refers to a moiety in a conjugate that is capable of specifically binding to a target (or a portion of the target) on the surface of a cell. Through the interaction of the targeting moiety with the target, the conjugate can be delivered to a particular cell population.

The term "fragment" refers to a portion of a compound molecule that remains after the compound molecule loses one or more atoms or groups of atoms (e.g., hydrogen atoms or hydroxy groups). For example, "compound fragment" refers to, after a compound described herein is linked to a linker in an immune-stimulating antibody conjugate, a portion of the compound that remains after the compound loses one hydrogen atom, hydroxy group, or the like.

The structure of the immune-stimulating antibody conjugate of the present application can be represented by general formula I, wherein z refers to the number of small-molecule drug fragments linked to each antibody molecule. In the process of preparing the immune-stimulating antibody conjugate, each antibody molecule may be linked to a different number of small-molecule drug fragments. Therefore, in general, the composition of the immune-stimulating antibody conjugate is a mixture of immune-stimulating antibody conjugates with different drug-to-antibody ratios. In practice, DAR is generally used to represent the average number of drugs linked to the antibody.

The linker may be linked to the antibody by various chemical bonds. For example, in some embodiments, the linker is linked by forming a thioether bond with a sulfhydryl group of the antibody. In some specific immune-stimulating antibody conjugates, -S- represents only a thioether bond formed by the linker and the sulfhydryl group of the antibody and does not mean that -S- is part of the linker.

### Immune-Stimulating Antibody Conjugate

One objective of the present disclosure is to provide an immune-stimulating antibody conjugate of formula (I): 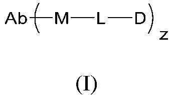 wherein
Ab is an antibody targeting a target antigen or an antigen-binding fragment thereof;
M is a linker part linking the antibody or the antigen-binding fragment thereof;
L is a linker linking M and D;
D is selected from structures of formula (D-I-1) and formula (D-I-2): 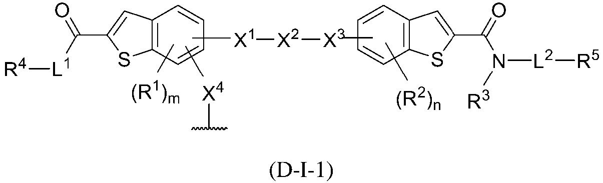 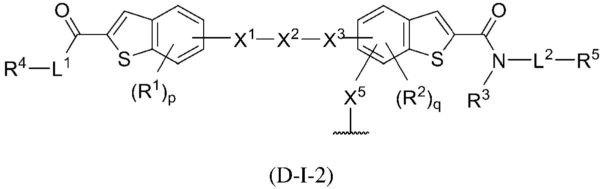
wherein 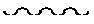 represents a point of attachment to L;
X¹ and X³ are identical or different and are each independently selected from a covalent bond, -O-, -S-, and -NR^{a}-;
X² is selected from C₁₋₆ alkylene, C₂₋₆ alkenylene, C₂₋₆ alkynylene, C₃₋₆ cycloalkyl, and 3-6 membered heterocyclyl, wherein the C₁₋₆ alkylene, C₂₋₆ alkenylene, C₂₋₆ alkynylene, C₃₋₆ cycloalkyl, and 3-6 membered heterocyclyl are each optionally substituted with one or more substituents independently selected from H, halogen, cyano, C₁₋₆ alkyl, -OR^{a}, and -NR^{a}R^{b};
X⁴ and X⁵ are each independently selected from -O- and -NR^{a}-;
L¹ is selected from a covalent bond and -(C(R⁶)₂)ⱼ-;
L² is selected from a covalent bond and -(C(R⁷)₂)ₖ-;
R¹ and R² are identical or different and are each independently selected from H, halogen, cyano, -OR^{a}, - NR^{a}R^{b}, -C(O)-OR^{a}, -C(O)-NR^{a}R^{b}, -NR^{a}-C(O)-R^{a}, C₁₋₆ alkyl, C₃₋₆ cycloalkyl, and 3-6 membered heterocyclyl, wherein the C₁₋₆ alkyl, C₃₋₆ cycloalkyl, and 3-6 membered heterocyclyl are each optionally substituted with one or more substituents independently selected from H, halogen, C₁₋₆ alkyl, -OR^{a}, and - NR^{a}R^{b};
R³ is selected from H and C₁₋₆ alkyl;
R⁴ is selected from H, -OR^{a}, -NR^{a}R^{b}, -C(O)-OR^{a}, -O-C(O)-R^{a}, -C(O)-NR^{a}R^{b}, -NR^{a}-C(O)-R^{a}, -O-C(O)-NR^{a}R^{b}, -NR^{a}-C(O)-OR^{a}, -NR^{a}-C(O)-NR^{a}R^{b}, C₁₋₆ alkyl, C₃₋₆ cycloalkyl, 3-6 membered heterocyclyl, C₆₋₁₀ aryl, and 5-10 membered heteroaryl, wherein the C₁₋₆ alkyl, C₃₋₆ cycloalkyl, 3-6 membered heterocyclyl, C₆₋₁₀ aryl, and 5-10 membered heteroaryl are each optionally substituted with one or more substituents independently selected from H, halogen, C₁₋₆ alkyl, -OR^{a}, -NR^{a}R^{b}, and -C(O)-OR^{a};
R⁵ is selected from H, C₁₋₆ alkyl, C₃₋₁₀ cycloalkyl, 3-10 membered heterocyclyl, -OR^{a}, and -C(O)-OR^{a}, wherein the C₁₋₆ alkyl, C₃₋₁₀ cycloalkyl, and 3-10 membered heterocyclyl are each optionally substituted with one or more substituents independently selected from H, halogen, C₁₋₆ alkyl, -OR^{a}, -NR^{a}R^{b}, and - C(O)-OR^{a};
each R⁶ is independently selected from H, -OR^{a}, -NR^{a}R^{b}, C₁₋₆ alkyl, C₂₋₆ alkenyl, C₂₋₆ alkynyl, C₃₋₆ cycloalkyl, and 3-6 membered heterocyclyl, wherein the C₁₋₆ alkyl, C₂₋₆ alkenyl, C₂₋₆ alkynyl, C₃₋₆ cycloalkyl, and 3-6 membered heterocyclyl are each optionally substituted with one or more substituents independently selected from H, halogen, C₁₋₆ alkyl, -OR^{a}, -NR^{a}R^{b}, and -C(O)-OR^{a}; or
two R⁶ groups on different carbon atoms, together with the carbon atoms therebetween, form C₃₋₆ cycloalkyl or 3-6 membered heterocyclyl; or
two R⁶ groups on the same carbon atom, together with the carbon atom to which they are both attached, form C₃₋₆ cycloalkyl or 3-6 membered heterocyclyl;
each R⁷ is independently selected from H, -OR^{a}, -NR^{a}R^{b}, C₁₋₆ alkyl, C₂₋₆ alkenyl, C₂₋₆ alkynyl, C₃₋₆ cycloalkyl, and 3-6 membered heterocyclyl, wherein the C₁₋₆ alkyl, C₂₋₆ alkenyl, C₂₋₆ alkynyl, C₃₋₆ cycloalkyl, and 3-6 membered heterocyclyl are each optionally substituted with one or more substituents independently selected from H, halogen, C₁₋₆ alkyl, -OR^{a}, -NR^{a}R^{b}, and -C(O)-OR^{a}; or
two R⁷ groups on the same carbon atom, together with the carbon atom to which they are both attached, form C₃₋₆ cycloalkyl or 3-6 membered heterocyclyl; or any one R⁷ and R³, together with the atoms therebetween, form 3-10 membered heterocyclyl;
R^{a} and R^{b} are each independently selected from H, C₁₋₆ alkyl, C₃₋₆ cycloalkyl, and 3-6 membered heterocyclyl, wherein the C₁₋₆ alkyl, C₃₋₆ cycloalkyl, and 3-6 membered heterocyclyl are each optionally substituted with one or more substituents independently selected from H, halogen, C₁₋₆ alkyl, hydroxy, amino, and carboxyl;
j and k are each independently selected from 1, 2, and 3;
m and q are each independently selected from 0, 1, and 2;
n and p are each independently selected from 0, 1, 2, and 3;
z is selected from 1-10.

In some embodiments of the present disclosure, M is selected from the following structures: 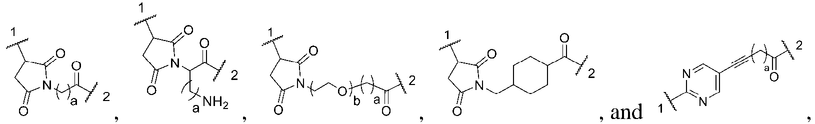 wherein position 1 of M is linked to Ab, and position 2 is linked to L; each a is independently selected from integers of 1-6 (e.g., 1, 2, 3, 4, 5, or 6), and b is selected from integers of 1-10 (e.g., 1, 2, 3, 4, 5, 6, 7, 8, 9, or 10).

In some embodiments of the present disclosure, M is selected from the following structures: 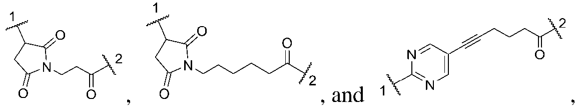 wherein position 1 of M is linked to Ab, and position 2 is linked to L.

In some embodiments of the present disclosure, M is selected from the following structures: 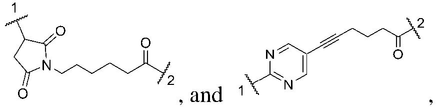 wherein position 1 of M is linked to Ab, and position 2 is linked to L.

In some embodiments of the present disclosure, M is 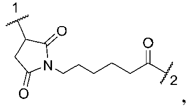 wherein position 1 of M is linked to Ab, and position 2 is linked to L.

In some embodiments of the present disclosure, M is 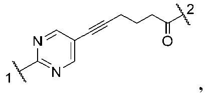 wherein position 1 of M is linked to Ab, and position 2 is linked to L.

In some embodiments of the present disclosure, L is selected from a non-cleavable linker and a cleavable linker, wherein the cleavable linker is cleaved by an enzyme present in a pathological environment, and the enzyme is selected from a protease, a phosphatase, a pyrophosphatase, *β*-glucuronidase, *β-*galactosidase, and a sulfatase.

In some embodiments of the present disclosure, L is -L^{a}-L^{b}-L^{c}-, wherein:
L^{a} is a covalent bond or is selected from 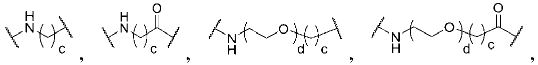 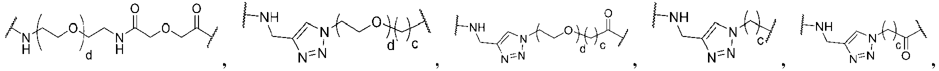 and 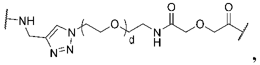
wherein each c is independently selected from integers of 1-6, and each d is independently selected from integers of 1-10;
L^{b} is a covalent bond or is selected from an amino acid fragment and a peptide fragment formed from two or more amino acids, wherein the amino acids are selected from Val, Cit, Glu, Lys, Arg, Phe, Leu, Gly, Ala, and Asn;
L^{c} is a covalent bond or -NH-CH₂-, or is selected from the following structures:

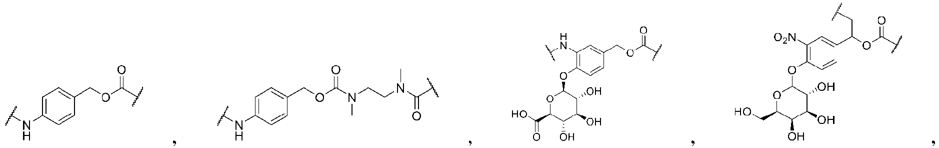

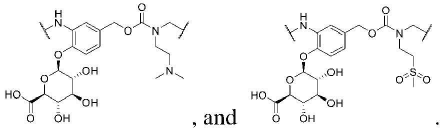

In some embodiments of the present disclosure, L^{a} is a covalent bond or

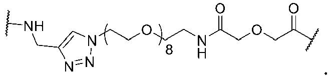

In some embodiments of the present disclosure, L^{a} is a covalent bond.

In some embodiments of the present disclosure, L^{b} is a covalent bond or a peptide fragment formed from two or more amino acids, wherein the amino acids are selected from Val, Cit, Phe, Gly, and Ala.

In some embodiments of the present disclosure, L^{b} is a covalent bond or is selected from the following structures:

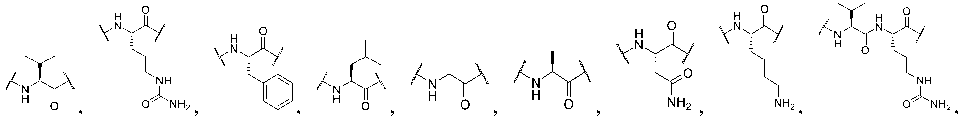

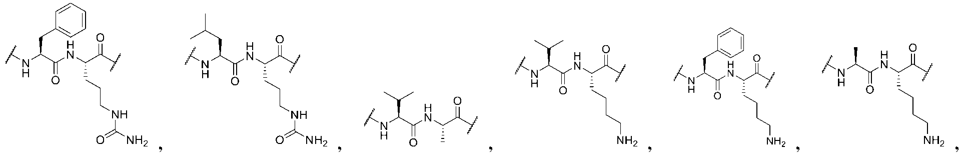

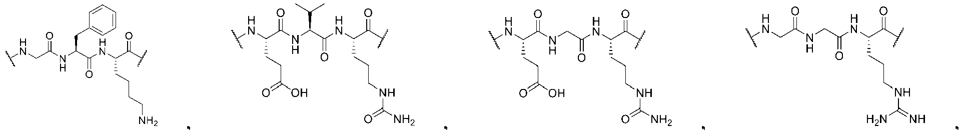

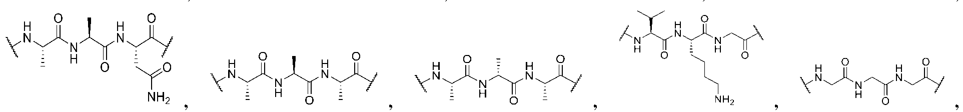

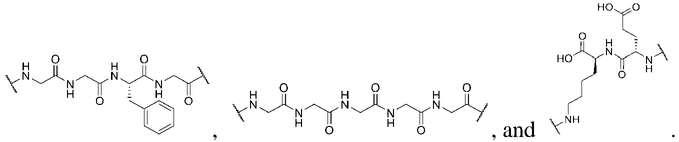

In some embodiments of the present disclosure, L^{b} is selected from the following structures:

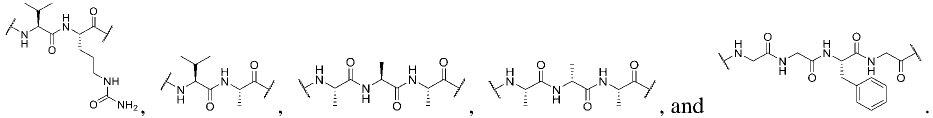

In some embodiments of the present disclosure, L^{c} is a covalent bond or -NH-CH₂-, or is selected from the following structures:

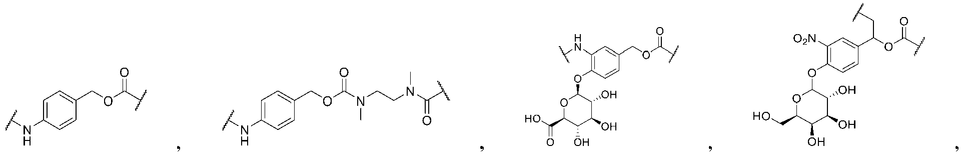

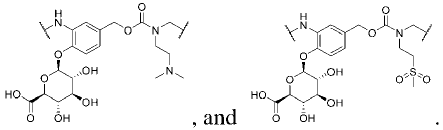

In some embodiments of the present disclosure, L^{c} is selected from -NH-CH₂- and

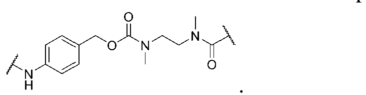

In some embodiments of the present disclosure, L is selected from the following structures: 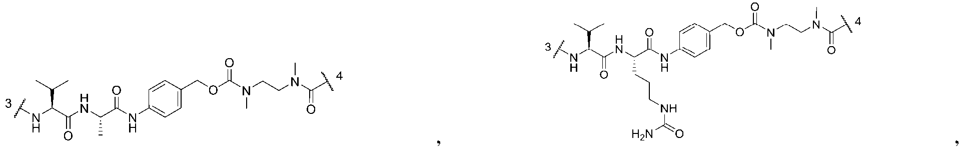 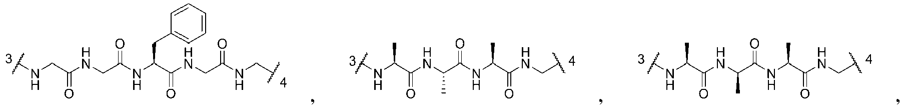 and 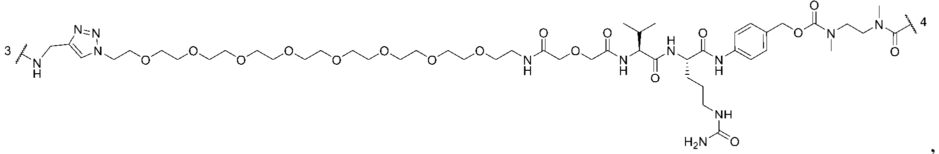 wherein position 3 of L is linked to M, and position 4 is linked to D.

In some embodiments of the present disclosure, X¹ and X³ in formula (D-I-1) and formula (D-I-2) are each independently selected from -O- and -S-.

In some embodiments of the present disclosure, X¹ and X³ in formula (D-I-1) and formula (D-I-2) are both -O-.

In some embodiments of the present disclosure, X² in formula (D-I-1) and formula (D-I-2) is selected from C₁₋₆ alkylene, e.g., C₂₋₄ alkylene.

In some embodiments of the present disclosure, X² in formula (D-I-1) and formula (D-I-2) is selected from -CH₂-, -(CH₂)₂-, -(CH₂)₃-, -(CH₂)₄-, and -(CH₂)₅-.

In some embodiments of the present disclosure, D is selected from structures of formula (D-II-1) and formula (D-II-2): 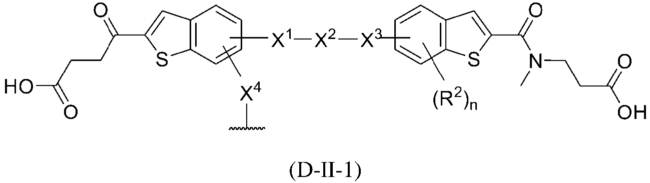 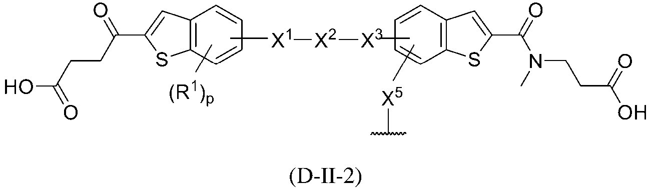 wherein 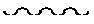 represents a point of attachment to L, and the groups R¹, R², X¹, X², X³, X⁴, and X⁵, n, and p are as defined above.

In some embodiments of the present disclosure, D is selected from structures of formula (D-III-1), formula (D-III-2), formula (D-III-3), and formula (D-III-4): 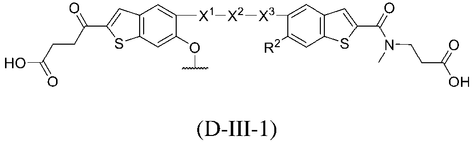 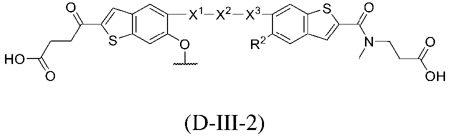 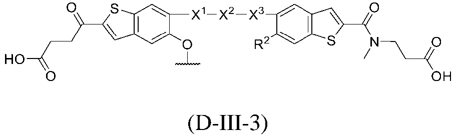 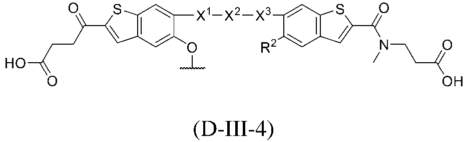 wherein 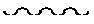 represents a point of attachment to L, and the groups R², X¹, X², and X³ are as defined above.

In some embodiments of the present disclosure, D is selected from the following structures: 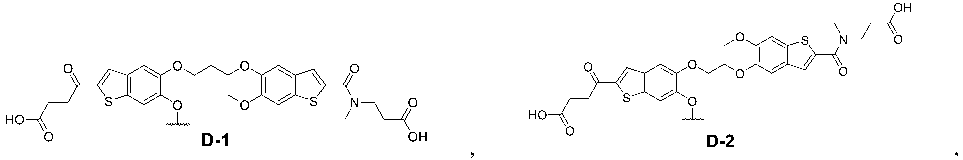 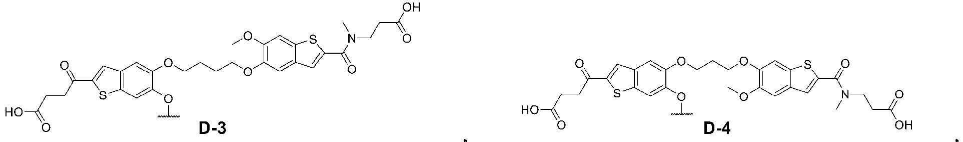 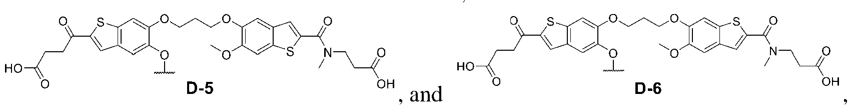 wherein 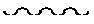 represents a point of attachment to L.

In some embodiments of the present disclosure, D is the following structure:

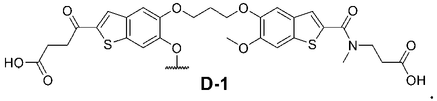

In some embodiments of the present disclosure, Ab is an antibody or an antigen-binding fragment thereof; the antibody is selected from a monoclonal antibody, a polyclonal antibody, a linear antibody, a bispecific antibody, a multispecific antibody, a chimeric antibody, a murine antibody, a humanized antibody, a fully human antibody, and a fusion protein comprising an antigen-binding moiety of an antibody; the antigen-binding fragment is selected from Fab, Fab', F(ab')2, Fv, disulfide-linked Fv, and scFv.

In some embodiments of the present disclosure, the antibody or the antigen-binding fragment thereof is an anti-Her-2 antibody.

In some embodiments of the present disclosure, the antibody or the antigen-binding fragment thereof comprises:
(1) the following heavy chain variable region (VH) and/or light chain variable region (VL):
   a heavy chain variable region (VH) comprising the following 3 CDRs: a CDR-H1 whose sequence is SEQ ID NO: 3 or a variant thereof, a CDR-H2 whose sequence is SEQ ID NO: 4 or a variant thereof, and a CDR-H3 whose sequence is SEQ ID NO: 5 or a variant thereof; and/or a light chain variable region (VL) comprising the following 3 CDRs: a CDR-L1 whose sequence is SEQ ID NO: 6 or a variant thereof, a CDR-L2 whose sequence is SEQ ID NO: 7 or a variant thereof, and a CDR-L3 whose sequence is SEQ ID NO: 8 or a variant thereof;
(2) the following heavy chain variable region (VH) and/or light chain variable region (VL):
   a heavy chain variable region (VH) comprising the following 3 CDRs: a CDR-H1 whose sequence is SEQ ID NO: 9 or a variant thereof, a CDR-H2 whose sequence is SEQ ID NO: 10 or a variant thereof, and a CDR-H3 whose sequence is SEQ ID NO: 5 or a variant thereof; and/or a light chain variable region (VL) comprising the following 3 CDRs: a CDR-L1 whose sequence is SEQ ID NO: 6 or a variant thereof, a CDR-L2 whose sequence is SEQ ID NO: 7 or a variant thereof, and a CDR-L3 whose sequence is SEQ ID NO: 8 or a variant thereof;
(3) the following heavy chain variable region (VH) and/or light chain variable region (VL):
   a heavy chain variable region (VH) comprising the following 3 CDRs: a CDR-H1 whose sequence is SEQ ID NO: 11 or a variant thereof, a CDR-H2 whose sequence is SEQ ID NO: 12 or a variant thereof, and a CDR-H3 whose sequence is SEQ ID NO: 13 or a variant thereof; and/or a light chain variable region (VL) comprising the following 3 CDRs: a CDR-L1 whose sequence is SEQ ID NO: 14 or a variant thereof, a CDR-L2 whose sequence is SEQ ID NO: 15 or a variant thereof, and a CDR-L3 whose sequence is SEQ ID NO: 8 or a variant thereof; or
(4) the following heavy chain variable region (VH) and/or light chain variable region (VL):
   a heavy chain variable region (VH) comprising the following 3 CDRs: a CDR-H1 whose sequence is SEQ ID NO: 16 or a variant thereof, a CDR-H2 whose sequence is SEQ ID NO: 17 or a variant thereof, and a CDR-H3 whose sequence is SEQ ID NO: 5 or a variant thereof; and/or a light chain variable region (VL) comprising the following 3 CDRs: a CDR-L1 whose sequence is SEQ ID NO: 6 or a variant thereof, a CDR-L2 whose sequence is SEQ ID NO: 7 or a variant thereof, and a CDR-L3 whose sequence is SEQ ID NO: 8 or a variant thereof.

In some embodiments of the present disclosure, the antibody or the antigen-binding fragment thereof comprises:
(1) the following heavy chain variable region (VH) and/or light chain variable region (VL), wherein the CDRs are defined according to the Chothia numbering system:
   a heavy chain variable region (VH) comprising the following 3 CDRs: a CDR-H1 whose sequence is SEQ ID NO: 3 or a variant thereof, a CDR-H2 whose sequence is SEQ ID NO: 4 or a variant thereof, and a CDR-H3 whose sequence is SEQ ID NO: 5 or a variant thereof; and/or a light chain variable region (VL) comprising the following 3 CDRs: a CDR-L1 whose sequence is SEQ ID NO: 6 or a variant thereof, a CDR-L2 whose sequence is SEQ ID NO: 7 or a variant thereof, and a CDR-L3 whose sequence is SEQ ID NO: 8 or a variant thereof;
(2) the following heavy chain variable region (VH) and/or light chain variable region (VL), wherein the CDRs are defined according to the Kabat numbering system:
   a heavy chain variable region (VH) comprising the following 3 CDRs: a CDR-H1 whose sequence is SEQ ID NO: 9 or a variant thereof, a CDR-H2 whose sequence is SEQ ID NO: 10 or a variant thereof, and a CDR-H3 whose sequence is SEQ ID NO: 5 or a variant thereof; and/or a light chain variable region (VL) comprising the following 3 CDRs: a CDR-L1 whose sequence is SEQ ID NO: 6 or a variant thereof, a CDR-L2 whose sequence is SEQ ID NO: 7 or a variant thereof, and a CDR-L3 whose sequence is SEQ ID NO: 8 or a variant thereof;
(3) the following heavy chain variable region (VH) and/or light chain variable region (VL), wherein the CDRs are defined according to the IMGT numbering system:
   a heavy chain variable region (VH) comprising the following 3 CDRs: a CDR-H1 whose sequence is SEQ ID NO: 11 or a variant thereof, a CDR-H2 whose sequence is SEQ ID NO: 12 or a variant thereof, and a CDR-H3 whose sequence is SEQ ID NO: 13 or a variant thereof; and/or a light chain variable region (VL) comprising the following 3 CDRs: a CDR-L1 whose sequence is SEQ ID NO: 14 or a variant thereof, a CDR-L2 whose sequence is SEQ ID NO: 15 or a variant thereof, and a CDR-L3 whose sequence is SEQ ID NO: 8 or a variant thereof; or
(4) the following heavy chain variable region (VH) and/or light chain variable region (VL), wherein the CDRs are defined according to the AbM numbering system:
   a heavy chain variable region (VH) comprising the following 3 CDRs: a CDR-H1 whose sequence is SEQ ID NO: 16 or a variant thereof, a CDR-H2 whose sequence is SEQ ID NO: 17 or a variant thereof, and a CDR-H3 whose sequence is SEQ ID NO: 5 or a variant thereof; and/or a light chain variable region (VL) comprising the following 3 CDRs: a CDR-L1 whose sequence is SEQ ID NO: 6 or a variant thereof, a CDR-L2 whose sequence is SEQ ID NO: 7 or a variant thereof, and a CDR-L3 whose sequence is SEQ ID NO: 8 or a variant thereof.

In some embodiments of the present disclosure, the antibody or the antigen-binding fragment thereof comprises: a VH set forth in SEQ ID NO: 18 or a variant thereof, and/or a VL set forth in SEQ ID NO: 19 or a variant thereof.

In some embodiments of the present disclosure, the antibody or the antigen-binding fragment thereof comprises: a heavy chain comprising a VH set forth in SEQ ID NO: 18 or a variant thereof and a heavy chain constant region (CH) set forth in SEQ ID NO: 20 or a variant thereof, and/or a light chain comprising a VL set forth in SEQ ID NO: 19 or a variant thereof and a light chain constant region (CL) set forth in SEQ ID NO: 21 or a variant thereof.

In some embodiments of the present disclosure, the anti-Her-2 antibody is trastuzumab.

In some embodiments of the present disclosure, the amino acid sequence of a heavy chain of trastuzumab is set forth in SEQ ID NO: 1, and the amino acid sequence of a light chain of trastuzumab is set forth in SEQ ID NO: 2.

The immune-stimulating antibody conjugate described in the present disclosure encompasses immune-stimulating antibody conjugates of formula (I) obtained by arbitrarily combining the preferred groups described above.

According to some embodiments of the present disclosure, the immune-stimulating antibody conjugate of the present disclosure is selected from: 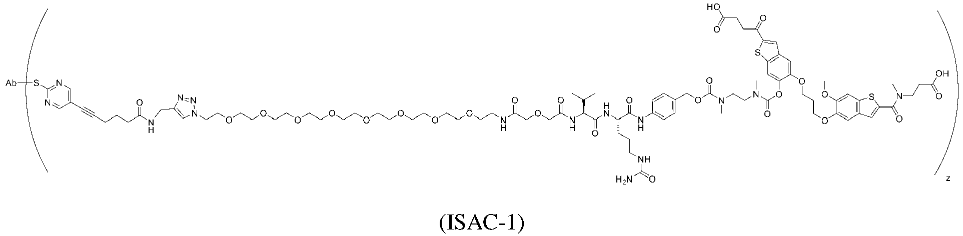 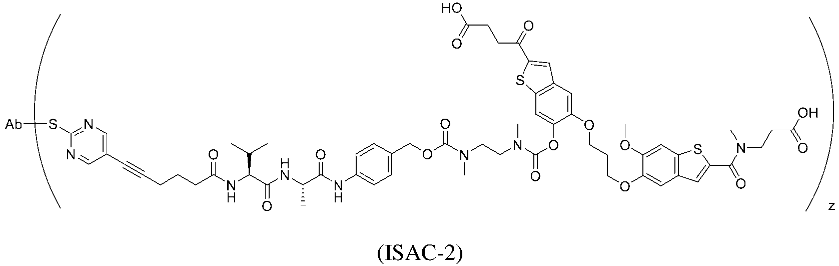 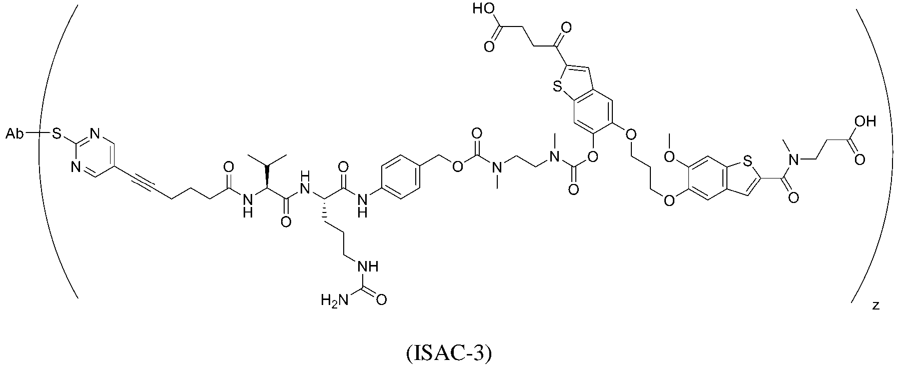 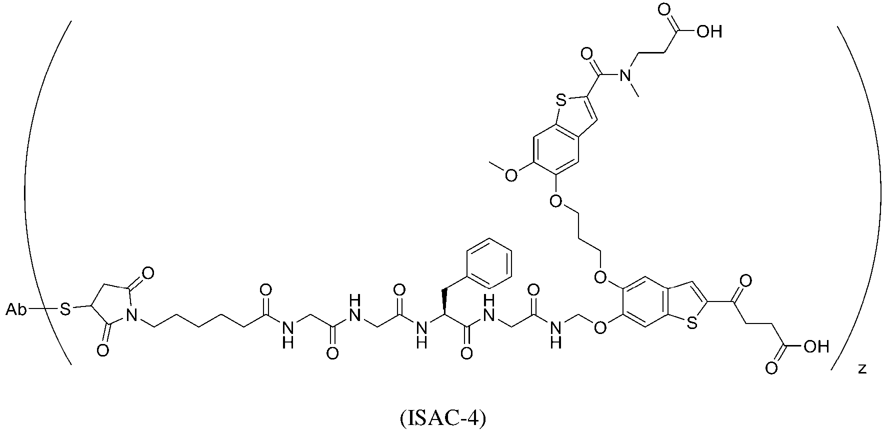 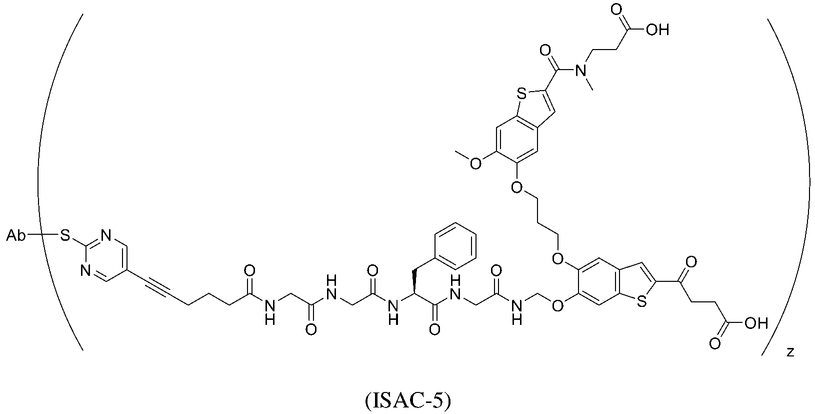 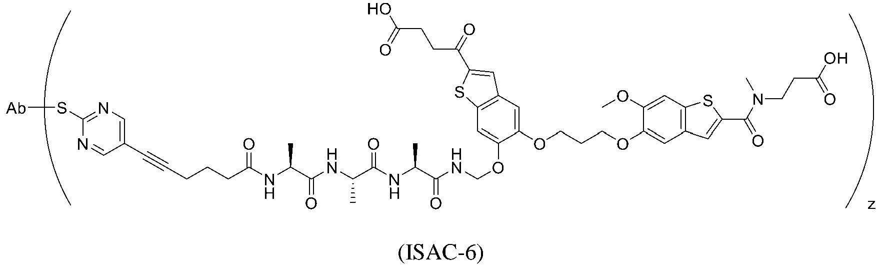 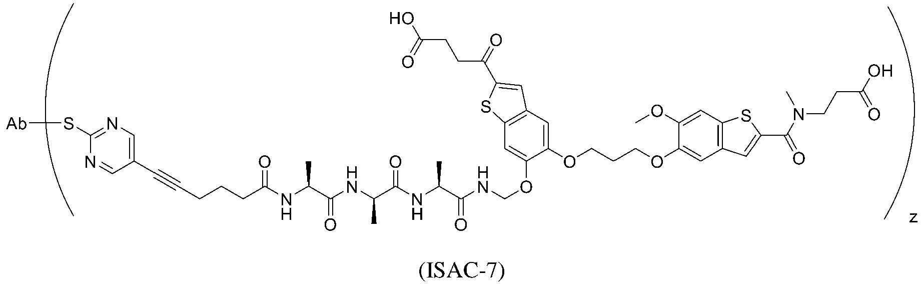 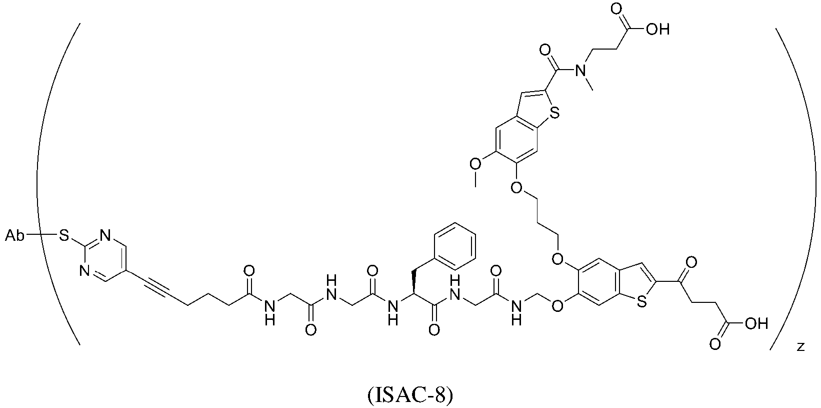 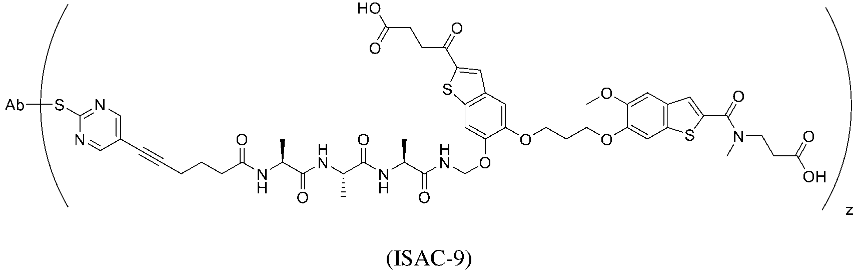 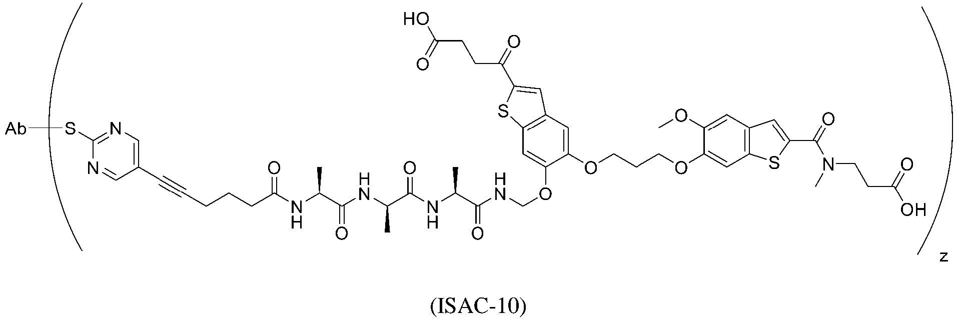 wherein z is selected from 1-10 (e.g., 1, 2, 3, 4, 5, 6, 7, 8, 9, or 10).

In some embodiments of the present disclosure, Ab is trastuzumab.

The present disclosure provides the following immune-stimulating antibody conjugates: 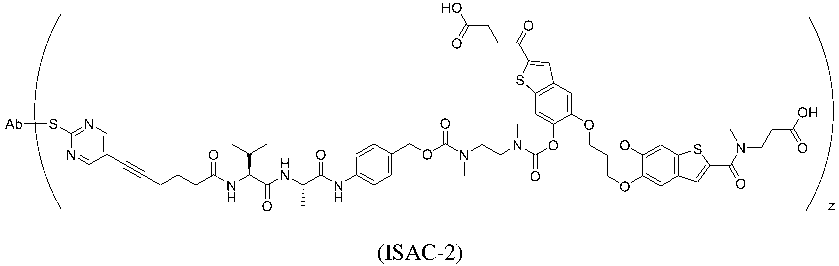 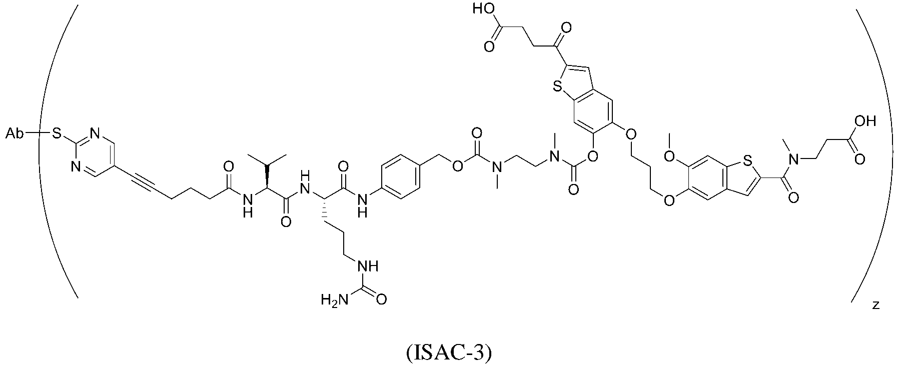 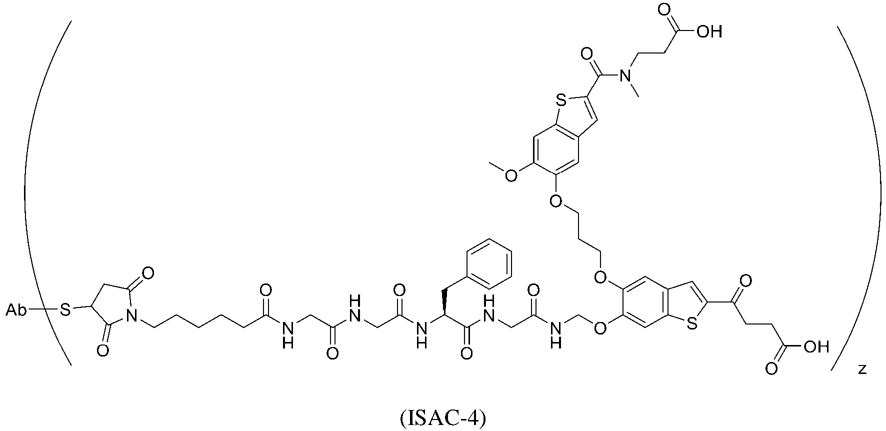 wherein each z is selected from 1 to 10; Ab is selected from an anti-Her-2 antibody.

The present disclosure provides the following immune-stimulating antibody conjugates: 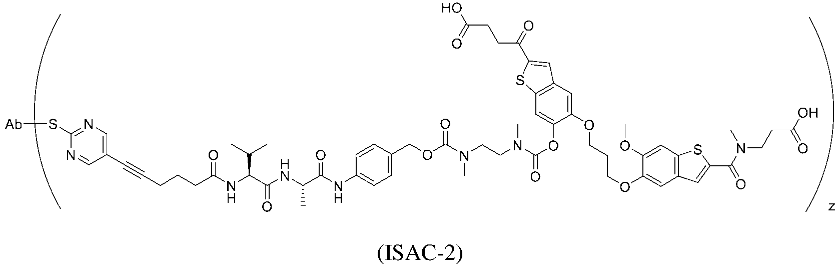 wherein each z is selected from 1 to 10; Ab is selected from an anti-Her-2 antibody.

The present disclosure provides the following immune-stimulating antibody conjugates: 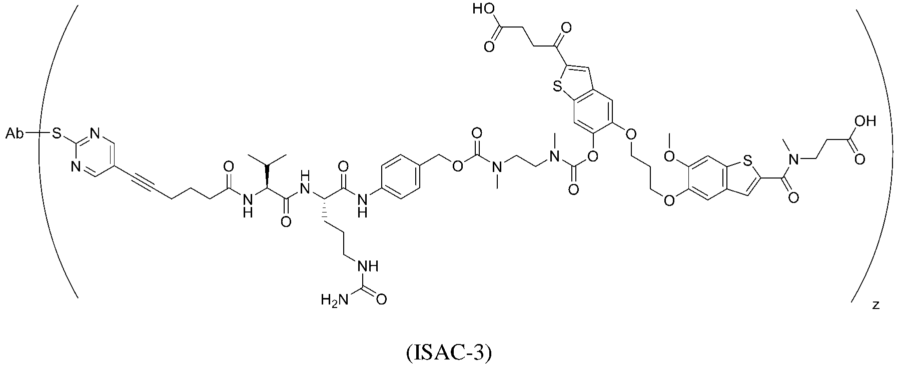 wherein each z is selected from 1 to 10; Ab is selected from an anti-Her-2 antibody.

The present disclosure provides the following immune-stimulating antibody conjugates: 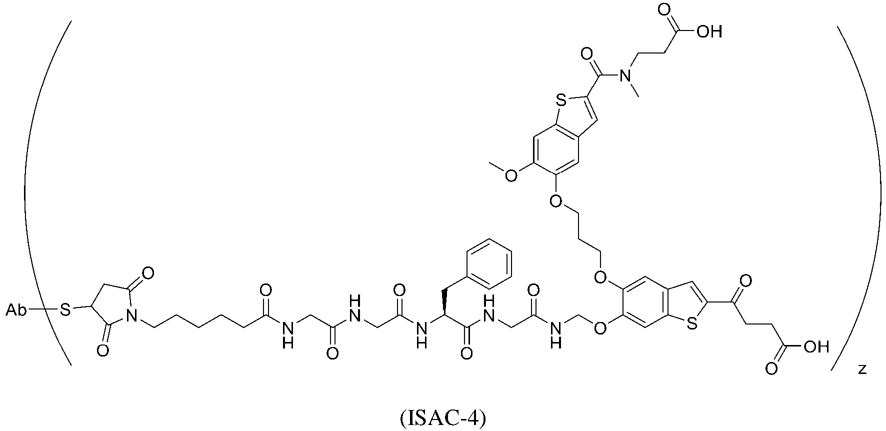 wherein each z is selected from 1 to 10; Ab is selected from an anti-Her-2 antibody.

In some embodiments of the present disclosure, Ab comprises a VH set forth in SEQ ID NO: 18 and/or a VL set forth in SEQ ID NO: 19.

In some embodiments of the present disclosure, Ab comprises a heavy chain comprising a VH set forth in SEQ ID NO: 18 and a CH set forth in SEQ ID NO: 20, and/or a light chain comprising a VL set forth in SEQ ID NO: 19 and a CL set forth in SEQ ID NO: 21.

In some embodiments of the present disclosure, Ab is trastuzumab.

The present disclosure provides a composition of an immune-stimulating antibody conjugate, comprising the immune-stimulating antibody conjugate described above, wherein the composition has a DAR value of 1.0-10.0, e.g., 1.0-2.0, 1.0-3.0, 1.0-3.5, 1.0-4.0, 1.0-4.5, 1.0-5.0, 1.0-5.5, 1.0-6.0, 1.0-6.5, 1.0-7.0, 1.0-7.5, 1.0-8.0, 1.0-8.5, 1.0-9.0, 1.0-9.5, 2.0-3.0, 2.0-3.5, 2.0-4.0, 2.0-4.5, 2.0-5.0, 2.0-5.5, 2.0-6.0, 2.0-6.5, 2.0-7.0, 2.0-7.5, 2.0-8.0, 2.0-8.5, 2.0-9.0, 2.0-9.5, 2.0-10.0, 3.0-3.5, 3.0-4.0, 3.0-4.5, 3.0-5.0, 3.0-5.5, 3.0-6.0, 3.0-6.5, 3.0-7.0, 3.0-7.5, 3.0-8.0, 3.0-8.5, 3.0-9.0, 3.0-9.5, 3.0-10.0, 3.5-4.0, 3.5-4.5, 3.5-5.0, 4.0-4.5, 4.5-5.0, 4.0-5.0, 4.0-5.5, 4.0-6.0, 4.0-6.5, 4.0-7.0, 4.0-7.5, 4.0-8.0, 4.0-8.5, 4.0-9.0, 4.0-9.5, 4.0-10.0, 5.0-5.5, 5.0-6.0, 5.0-6.5, 5.0-7.0, 5.0-7.5, 5.0-8.0, 5.0-8.5, 5.0-9.0, 5.0-9.5, 5.0-10.0, 6.0-6.5, 6.0-7.0, 6.0-7.5, 6.0-8.0, 6.0-8.5, 6.0-9.0, 6.0-9.5, 6.0-10.0, 7.0-7.5, 7.0-8.0, 7.0-8.5, 7.0-9.0, 7.0-9.5, 7.0-10.0, 8.0-8.5, 8.0-9.0, 8.0-9.5, or 8.0-10.0.

In some embodiments, the composition of the immune-stimulating antibody conjugate has a DAR value of 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0.

### Drug-Linker Compound

The present disclosure further provides a drug-linker compound of formula (II) or a pharmaceutically acceptable salt, ester, stereoisomer, tautomer, polymorph, solvate, N-oxide, isotopically labeled compound, metabolite, or prodrug thereof:

M¹-L-D (II)

wherein
M¹ is selected from 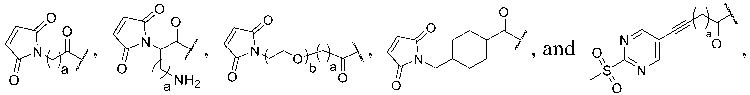
wherein each a is independently selected from integers of 1-6, and b is selected from integers of 1-10;
L and D are as defined above.

In some embodiments of the present disclosure, M¹ is selected from the following structures:

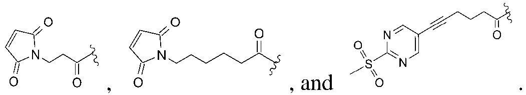

The drug-linker compound described in the present disclosure is selected from the following structures:

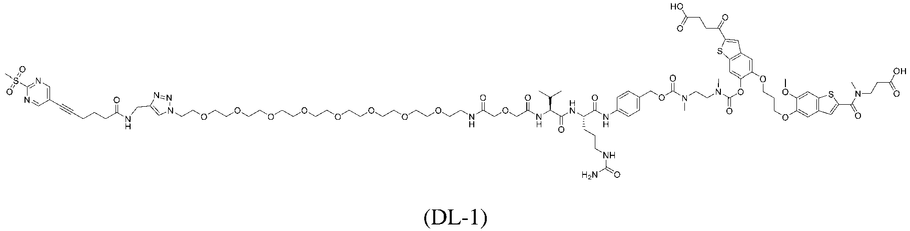

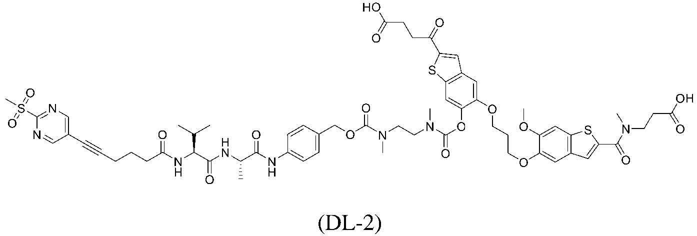

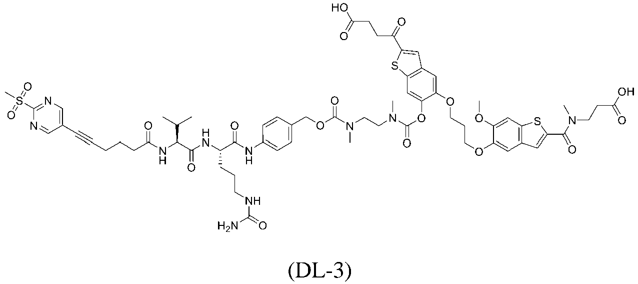

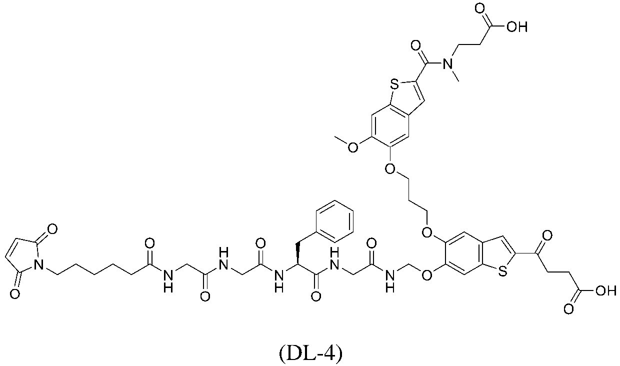

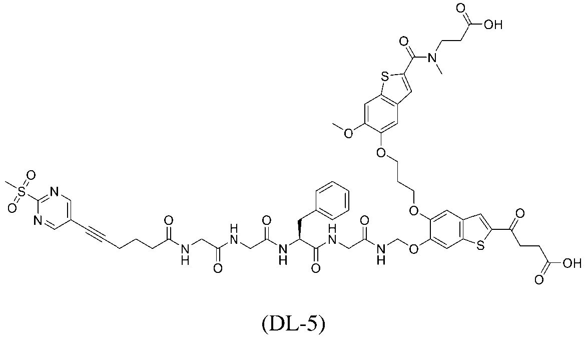

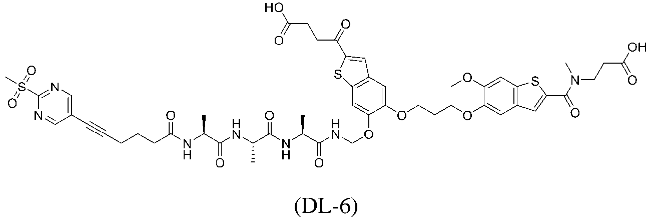

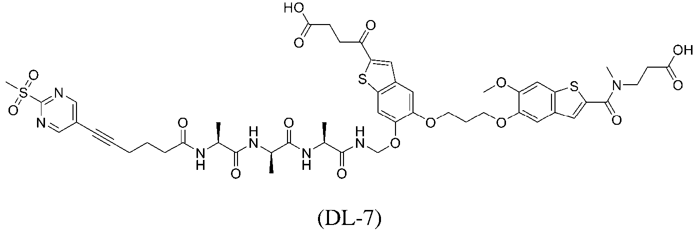

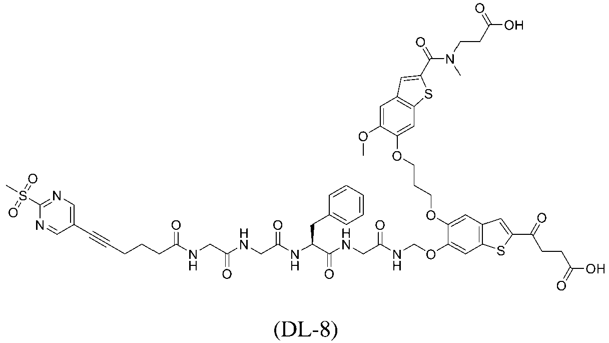

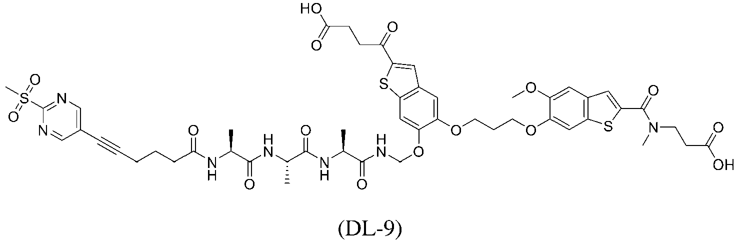

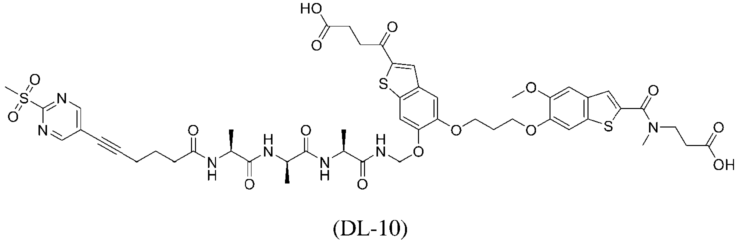

### Compound

The present disclosure further provides a compound represented by formula III-1 or III-2 or a pharmaceutically acceptable salt, ester, stereoisomer, tautomer, polymorph, solvate, N-oxide, isotopically labeled compound, metabolite, or prodrug thereof: 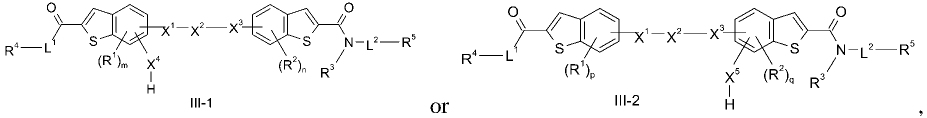 wherein R¹, R², R³, R⁴, R⁵, L¹, L², X¹, X², X³, X⁴, X⁵, m, n, p, and q are as defined above.

In some embodiments of the present disclosure, R⁴ and R⁵ are each independently selected from -C(O)-OH and -C(O)-O-C₁₋₆ alkyl.

In some embodiments of the present disclosure, R⁴ and R⁵ are each independently selected from -C(O)-OH, -C(O)-O-methyl, -C(O)-O-ethyl, -C(O)-O-n-propyl, -C(O)-O-isopropyl, and -C(O)-O-tert-butyl.

In some embodiments of the present disclosure, the compound of formula III-1 described above has a structure represented by formula III-1-1, and the compound of formula III-2 has a structure represented by formula III-2-1:

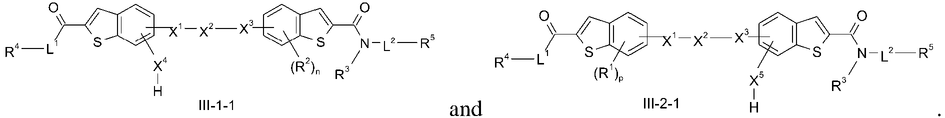

In some embodiments of the present disclosure, the compound of formula III-1 described above has a structure represented by formula III-1-2, and the compound of formula III-2 has a structure represented by formula III-2-2:

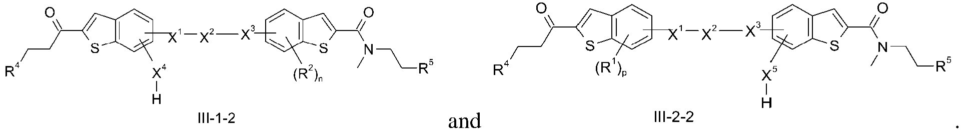

In some embodiments of the present disclosure, the compound of formula III-1 described above has structures represented by formula III-1-3-a to III-1-3-d, and the compound of formula III-2 has structures represented by formula III-2-3-a to III-2-3-d: 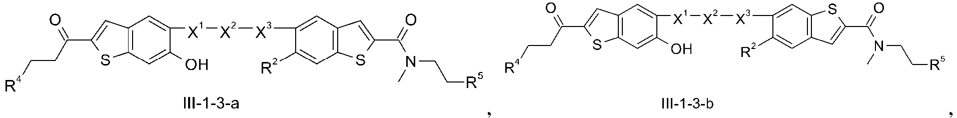 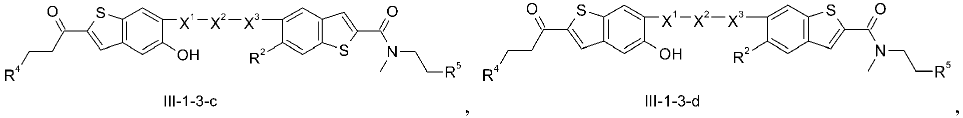 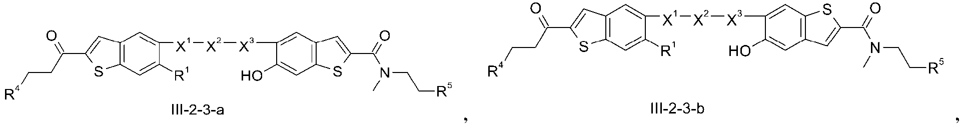 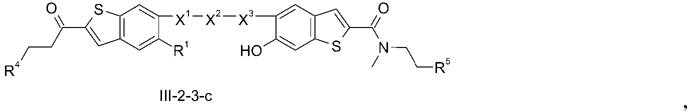 and

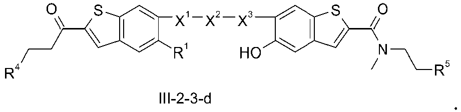

In some embodiments of the present disclosure, the compound represented by formula III-1 or formula III-2 is selected from the following structures: 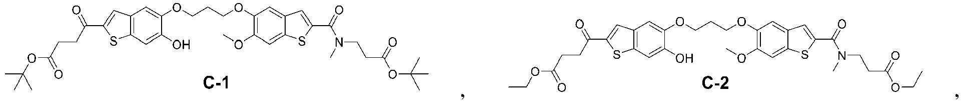 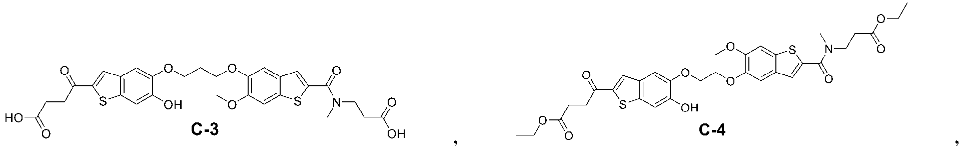 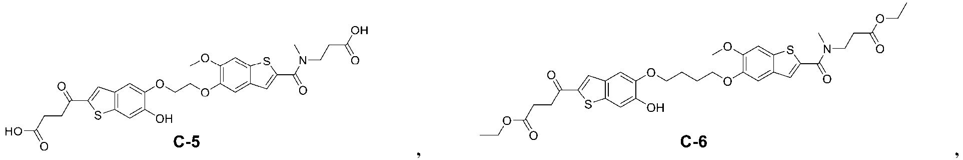 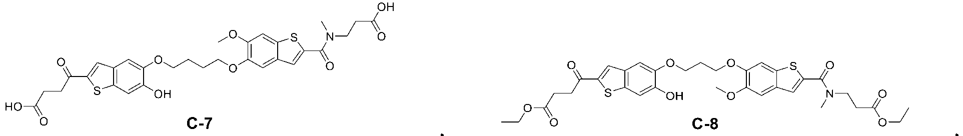 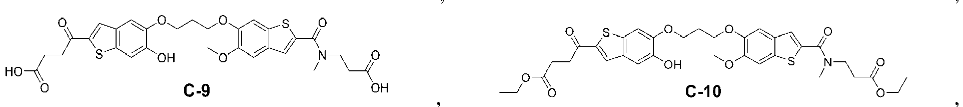 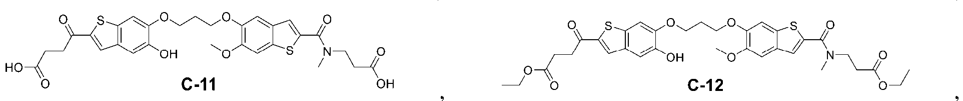 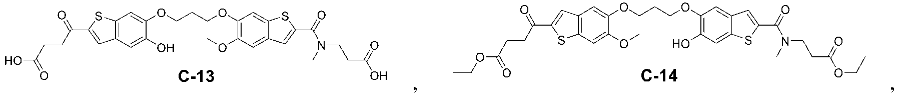 and

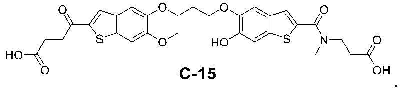

The present disclosure further provides a compound represented by formula IV-1 or IV-2 or a pharmaceutically acceptable salt, ester, stereoisomer, tautomer, polymorph, solvate, N-oxide, isotopically labeled compound, metabolite, or prodrug thereof: 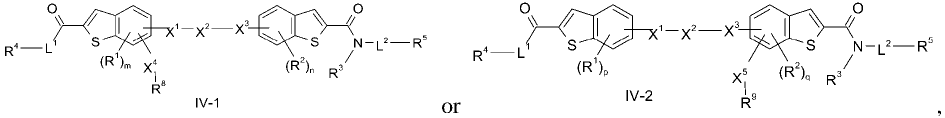
wherein R¹, R², R³, R⁴, R⁵, L¹, L², X¹, X², X³, X⁴, X⁵, m, n, p, and q are as defined above;
R⁸ and R⁹ are each independently selected from -C₁₋₆ alkylene-NR¹²-C(=O)-C₁₋₆ alkylene-NR¹⁰R¹¹, - C(=O)O-C₆₋₁₀ aryl, -C(=O)NR¹²-C₁₋₆ alkylene-NR¹⁰R¹¹, -C₁₋₆ alkylene-O-C₁₋₆ alkyl, and -C₁₋₆ alkylene-C(=O)-C₆₋₁₀ aryl, wherein the aryl is optionally substituted with amino or nitro;
R¹⁰ and R¹¹ are each independently selected from H, C₁₋₆ alkyl, Fmoc, and Boc;
R¹² is selected from H and C₁₋₆ alkyl.

In some embodiments of the present disclosure, the compound represented by formula IV-1 or IV-2 is selected from the following structures: 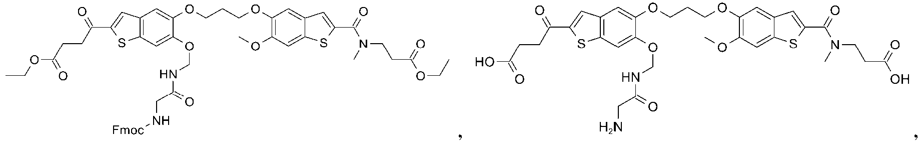 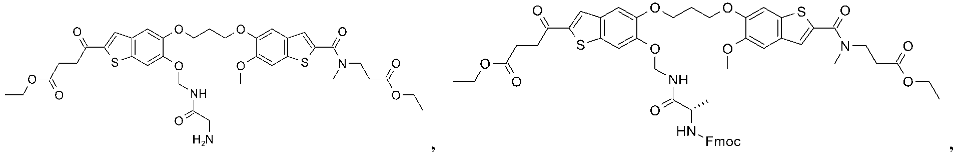 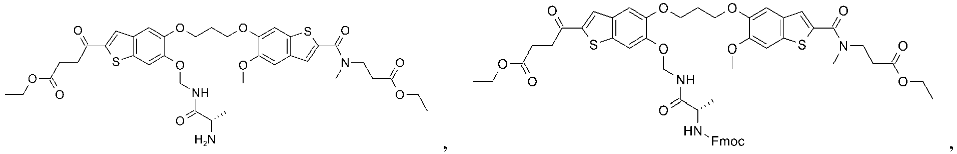 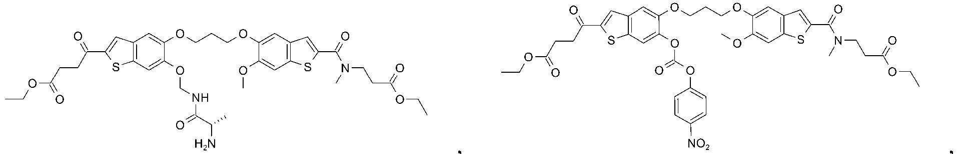 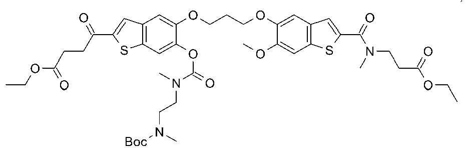 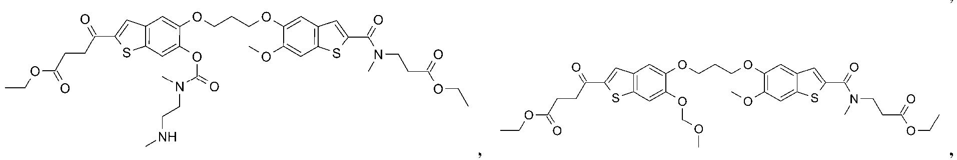 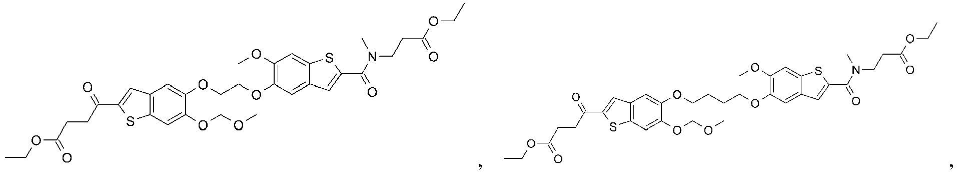 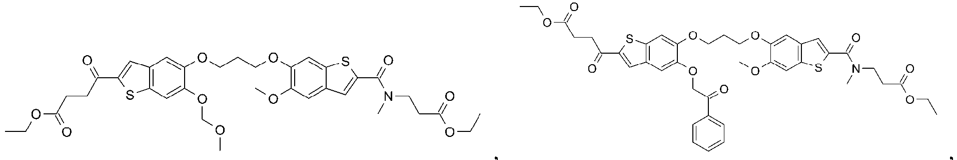 and

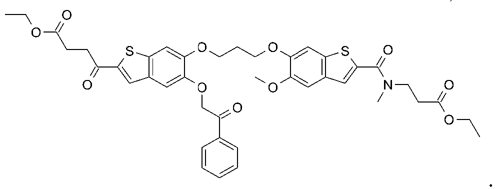

### Pharmaceutical Composition and Kit

Another objective of the present disclosure is to provide a pharmaceutical composition comprising a prophylactically or therapeutically effective amount of the immune-stimulating antibody conjugate of the present disclosure, the drug-linker compound of formula II or the compound of formula III-1, formula III-2, formula IV-1, or formula IV-2 or the pharmaceutically acceptable salt, ester, stereoisomer, tautomer, polymorph, solvate, N-oxide, isotopically labeled compound, metabolite, or prodrug thereof of the present disclosure, or the composition of the antibody-drug conjugate of the present disclosure, and one or more pharmaceutically acceptable carriers.

Another objective of the present disclosure is to provide a pharmaceutical composition comprising the immune-stimulating antibody conjugate according to any one of the above. In some embodiments, the pharmaceutical composition further comprises one or more pharmaceutically acceptable auxiliary materials. In some embodiments, the pharmaceutical composition has a DAR value of 1.0-10.0, e.g., 6.0-9.0, e.g., 1.0-2.0, 1.0-3.0, 1.0-3.5, 1.0-4.0, 1.0-4.5, 1.0-5.0, 1.0-5.5, 1.0-6.0, 1.0-6.5, 1.0-7.0, 1.0-7.5, 1.0-8.0, 1.0-8.5, 1.0-9.0, 1.0-9.5, 2.0-3.0, 2.0-3.5, 2.0-4.0, 2.0-4.5, 2.0-5.0, 2.0-5.5, 2.0-6.0, 2.0-6.5, 2.0-7.0, 2.0-7.5, 2.0-8.0, 2.0-8.5, 2.0-9.0, 2.0-9.5, 2.0-10.0, 3.0-3.5, 3.0-4.0, 3.0-4.5, 3.0-5.0, 3.0-5.5, 3.0-6.0, 3.0-6.5, 3.0-7.0, 3.0-7.5, 3.0-8.0, 3.0-8.5, 3.0-9.0, 3.0-9.5, 3.0-10.0, 3.5-4.0, 3.5-4.5, 3.5-5.0, 4.0-4.5, 4.5-5.0, 4.0-5.0, 4.0-5.5, 4.0-6.0, 4.0-6.5, 4.0-7.0, 4.0-7.5, 4.0-8.0, 4.0-8.5, 4.0-9.0, 4.0-9.5, 4.0-10.0, 5.0-5.5, 5.0-6.0, 5.0-6.5, 5.0-7.0, 5.0-7.5, 5.0-8.0, 5.0-8.5, 5.0-9.0, 5.0-9.5, 5.0-10.0, 6.0-6.5, 6.0-7.0, 6.0-7.5, 6.0-8.0, 6.0-8.5, 6.0-9.0, 6.0-9.5, 6.0-10.0, 7.0-7.5, 7.0-8.0, 7.0-8.5, 7.0-9.0, 7.0-9.5, 7.0-10.0, 8.0-8.5, 8.0-9.0, 8.0-9.5, or 8.0-10.0, e.g., 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0.

Another objective of the present disclosure is to provide a kit comprising the immune-stimulating antibody conjugate of the present disclosure, the drug-linker compound of formula II or the compound of formula III-1, formula III-2, formula IV-1, or formula IV-2 and the pharmaceutically acceptable salt, ester, stereoisomer, tautomer, polymorph, solvate, *N*-oxide, isotopically labeled compound, metabolite, or prodrug thereof of the present disclosure, the composition of the antibody-drug conjugate of the present disclosure, or the pharmaceutical composition of the present disclosure. Optionally, the kit further comprises a package insert.

The term "pharmaceutically acceptable carrier" in the present disclosure refers to a diluent, an adjuvant, an excipient, or a vehicle administered together with a therapeutic agent, which is suitable, within the scope of sound medical judgment, for contact with the tissues of humans and/or other animals without undue toxicity, irritation, allergic response, or other problems or complications relative to a reasonable benefit/risk ratio.

Pharmaceutically acceptable carriers that can be used in the pharmaceutical composition of the present disclosure include, but are not limited to, sterile liquids. The pharmaceutical composition may be, for example, in the form of a solid formulation, a semisolid formulation, a liquid formulation, a gaseous formulation, or the like.

The pharmaceutical composition of the present disclosure may act systemically and/or topically. For this purpose, they can be administered via a suitable route, e.g., by injection or transdermally, or orally or by inhalation.

A content or amount of the compound of the present disclosure in the pharmaceutical composition may be about 0.001 mg to about 1000 mg, suitably 0.01-800 mg, preferably 0.05-500 mg.

In some embodiments, the present disclosure provides a method for preparing the pharmaceutical composition of the present disclosure, comprising combining the compound or the pharmaceutically acceptable salt, ester, stereoisomer, tautomer, polymorph, solvate, N-oxide, isotopically labeled compound, metabolite, or prodrug thereof of the present disclosure with one or more pharmaceutically acceptable carriers.

### Treatment Method and Use

The present disclosure provides use of the immune-stimulating antibody conjugate according to any one of the above in the preparation of a medicament for treating and/or preventing cancer (e.g., HER2-positive cancer, such as HER2-positive gastric cancer, breast cancer, or non-small cell lung cancer).

In another aspect, the present disclosure provides use of the drug-linker compound or the compound or the pharmaceutically acceptable salt, ester, stereoisomer, tautomer, polymorph, solvate, N-oxide, isotopically labeled compound, metabolite, or prodrug thereof according to any one of the above in the preparation of an immune-stimulating antibody conjugate. In some embodiments, the immune-stimulating antibody conjugate comprises a small-molecule ligand capable of interacting with STING. In some embodiments, the immune-stimulating antibody conjugate is as described in any one of the above.

In another aspect, the compound or the pharmaceutically acceptable salt, ester, stereoisomer, tautomer, polymorph, solvate, N-oxide, isotopically labeled compound, metabolite, or prodrug thereof of the present disclosure, or the pharmaceutical composition of the present disclosure, has a strong agonistic effect on the target STING.

In another aspect, the present disclosure provides use of the immune-stimulating antibody conjugate, the drug-linker compound, or the compound or the pharmaceutically acceptable salt, ester, stereoisomer, tautomer, polymorph, solvate, N-oxide, isotopically labeled compound, metabolite, or prodrug thereof according to any one of the above, the composition of the immune-stimulating antibody conjugate, or the pharmaceutical composition of the present disclosure in the preparation of a medicament for treating and/or preventing cancer (e.g., HER2-positive cancer, such as HER2-positive gastric cancer, breast cancer, or non-small cell lung cancer).

In another aspect, the present disclosure provides the immune-stimulating antibody conjugate, the drug-linker compound, or the compound or the pharmaceutically acceptable salt, ester, stereoisomer, tautomer, polymorph, solvate, *N*-oxide, isotopically labeled compound, metabolite, or prodrug thereof according to any one of the above, the composition of the immune-stimulating antibody conjugate, or the pharmaceutical composition of the present disclosure for use in the treatment and/or prevention of cancer (e.g., HER2-positive cancer, such as HER2-positive gastric cancer, breast cancer, or non-small cell lung cancer).

In another aspect, the present disclosure provides a method for treating and/or preventing cancer (e.g., HER2-positive cancer, such as HER2-positive gastric cancer, breast cancer, or non-small cell lung cancer), comprising administering to an individual in need thereof a therapeutically and/or prophylactically effective amount of the immune-stimulating antibody conjugate, the drug-linker compound, or the compound or the pharmaceutically acceptable salt, ester, stereoisomer, tautomer, polymorph, solvate, N-oxide, isotopically labeled compound, metabolite, or prodrug thereof according to any one of the above, the composition of the immune-stimulating antibody conjugate, or the pharmaceutical composition of the present disclosure.

The term "effective amount" as used herein refers to an amount sufficient to achieve a desired prophylactic or therapeutic effect, e.g., an amount for palliating one or more symptoms associated with the disease to be treated.

The administration regimen may be adjusted to provide the optimum desired response. For example, a single bolus can be administered, several separate doses can be administered over time, or the dose can be proportionally reduced or increased as indicated by the urgent need for treatment. It is noted that dose values may vary with the type and severity of the condition to be palliated, and may include single or multiple doses. It will be further appreciated that for any particular individual, the specific administration regimen will be adjusted over time according to the individual's needs and the professional judgment of a person who administers the immune-stimulating antibody conjugate, the compound, or the pharmaceutical composition of the present disclosure or supervises the administration of the immune-stimulating antibody conjugate, the compound, or the pharmaceutical composition of the present disclosure.

The amount of the immune-stimulating antibody conjugate, the compound, or the pharmaceutical composition of the present disclosure administered will depend on the individual being treated, the severity of the disorder or condition, the rate of administration, the disposition of the compound, and the judgment of the prescribing physician. In some cases, dosage levels not higher than the lower limit of the aforementioned range may be sufficient, while in other cases, a larger dosage may still be employed without causing any adverse side effects, provided that the larger dosage is first divided into several smaller dosages for administration throughout the day.

Unless otherwise stated, the term "treat", "treated", "treating", or "treatment" as used herein means reversing, palliating, or ameliorating the progression of a disorder or condition to which such a term applies or one or more symptoms of such a disorder or condition.

The term "prevent", "prevented", "preventing", or "prevention" refers to inhibiting or delaying the onset of a disease and includes not only prevention prior to the development of the disease but also prevention of a recurrence of the disease after treatment.

The term "individual" as used herein includes humans or non-human animals. Exemplary human individuals include human individuals suffering from a disease (e.g., a disease described herein), which are referred to as patients, or normal individuals. In the present disclosure, "non-human animals" include all vertebrates, e.g., non-mammals (e.g., birds, amphibians, reptiles) and mammals, e.g., non-human primates, livestock, and/or domesticated animals (e.g., sheep, dogs, cats, cows, pigs, etc.).

### BRIEF DESCRIPTION OF THE DRAWING

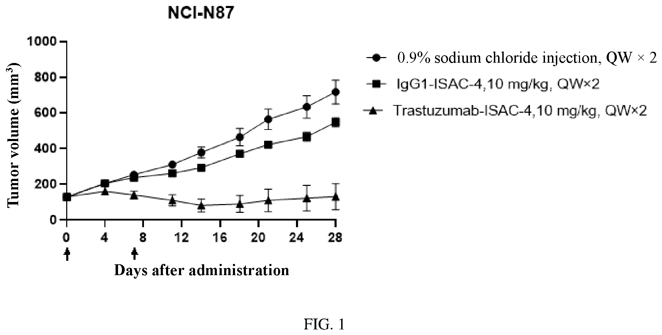

FIG. 1 shows curves of the tumor volume of each group in Experimental Example 4 changing over time.

### DETAILED DESCRIPTION

In order to render the objectives and technical solutions of the present disclosure clearer, the embodiments of the present disclosure will be described in detail below with reference to examples. Those skilled in the art will appreciate, however, that the following examples are intended only to illustrate the present disclosure, and should not be considered to limit the scope of the present disclosure. Examples without specified conditions were all conducted under conventional conditions or conditions recommended by the manufacturers. Reagents or instruments used without specified manufacturers were all commercially available conventional products.

The structures of compounds were determined by nuclear magnetic resonance (¹H NMR) or mass spectrometry (MS). ¹H NMR analysis was performed using a JEOL Eclipse 400 nuclear magnetic resonance instrument, with deuterated methanol (CD₃OD), deuterated chloroform (CDCl₃), or hexadeuterated dimethyl sulfoxide (DMSO-*d*₆) as a solvent and tetramethylsilane (TMS) as an internal standard. The chemical shifts (δ) are given in parts per million (ppm).

MS analysis was performed using an Agilent (ESI) mass spectrometer (manufacturer: Agilent; model: Agilent 6120B).

Preparation method for preparative high performance liquid chromatography:
Instrument model: Agilent 1260; column: Waters SunFire Prep C18 OBD (19 mm × 150 mm × 5.0 µm); column temperature: 25 °C; flow rate: 20.0 mL/min; detection wavelength: 214 nm; elution gradient: (0 min: 10% A, 90% B; 16.0 min: 90% A, 10% B); mobile phase A: acetonitrile; mobile phase B: 0.05% formic acid in water.

Aluminum plates (20 × 20 cm) produced by Merck were used for thin-layer chromatography (TLC) silica gel plates. GF 254 plates (1 mm) produced in Yantai were used for thin-layer chromatography separation and purification.

Thin-layer chromatography (TLC) or LC-MS was used for reaction monitoring; the developing solvent systems used included a dichloromethane and methanol system, a n-hexane and ethyl acetate system, and a petroleum ether and ethyl acetate system, and the volume ratio of the solvents was adjusted depending on the polarity of the compound or by adding triethylamine, etc.

A Biotage Initiator+ (400 W, RT to 300 °C) microwave reactor was used for microwave reactions.

200-300 mesh silica gel was generally used as the carrier in column chromatography. Eluent systems included a dichloromethane and methanol system and a petroleum ether and ethyl acetate system, and the volume ratio of the solvents was adjusted depending on the polarity of the compound, or it could be adjusted by adding a small amount of triethylamine.

In the examples, the reaction temperature was room temperature (20 °C to 35 °C), unless otherwise specified.

The reagents used in the present disclosure were purchased from Acros Organics, Aldrich Chemical Company, Topbiochem, etc.

In conventional synthesis methods, the examples, and the intermediate synthesis examples, the abbreviations have the following meanings.

| Abbreviation | Meaning |
|---|---|
| TLC | Thin-layer chromatography |
| LC-MS | Liquid chromatography-mass spectrometry |
| DMSO | Dimethyl sulfoxide |
| EDTA | Ethylenediaminetetraacetic acid |
| TCEP | Tris(2-carboxyethyl)phosphine |
| DMSO-*d*₆ | Hexadeuterated dimethyl sulfoxide |
| TMS | Tetramethylsilane |
| NMR | Nuclear magnetic resonance |
| MS | Mass spectrometry |
| s | Singlet |
| d | Doublet |
| t | Triplet |
| q | Quartet |
| dd | Double doublet |
| m | Multiplet |
| br | Broad |
| *J* | Coupling constant |
| Hz | Hertz |

The sequences involved in the present disclosure are described in the table below.

| SEQ ID NO | Description | Sequence |
|---|---|---|
| 1 | Heavy chain amino acid sequence of trastuzumab | EVQLVESGGGLVQPGGSLRLSCAASGFNIKDTYIHWVRQAPGK GLEWVARIYPTNGYTRYADSVKGRFTISADTSKNTAYLQMNSL RAEDTAVYYCSRWGGDGFYAMDYWGQGTLVTVSSASTKGPS VFPLAPSSKSTSGGTAALGCLVKDYFPEPVTVSWNSGALTSGV HTFPAVLQSSGLYSLSSVVTVPSSSLGTQTYICNVNHKPSNTKV DKKVEPKSCDKTHTCPPCPAPELLGGPSVFLFPPKPKDTLMISRT PEVTCVVVDVSHEDPEVKFNWYVDGVEVHNAKTKPREEQYNS TYRVVSVLTVLHQDWLNGKEYKCKVSNKALPAPIEKTISKAKG QPREPQVYTLPPSREEMTKNQVSLTCLVKGFYPSDIAVEWESN GQPENNYKTTPPVLDSDGSFFLYSKLTVDKSRWQQGNVFSCSV MHEALHNHYTQKSLSLSPGK |
| 2 | Light chain amino acid sequence of trastuzumab | DIQMTQSPSSLSASVGDRVTITCRASQDVNTAVAWYQQKPGKA PKLLIYSASFLYSGVPSRFSGSRSGTDFTLTISSLQPEDFATYYCQ QHYTTPPTFGQGTKVEIKRTVAAPSVFIFPPSDEQLKSGTASVVC LLNNFYPREAKVQWKVDNALQSGNSQESVTEQDSKDSTYSLSS TLTLSKADYEKHKVYACEVTHQGLSSPVTKSFNRGEC |
| 3 | Anti-Her2-CDR-H1 Chothia | GFNIKDT |
| 4 | Anti-Her2-CDR-H2 Chothia | YPTNGY |
| 5 | Anti-Her2-CDR-H3 Chothia/Kabat/Ab M | WGGDGFYAMDY |
| 6 | Anti-Her2-CDR-L1 Chothia/Kabat/Ab M | RASQDVNTAVA |
| 7 | Anti-Her2-CDR-L2 Chothia/Kabat/Ab M | SASFLYS |
| 8 | Anti-Her2-CDR-L3 Chothia/Kabat/Ab M/IMGT | QQHYTTPPT |
| 9 | Anti-Her2-CDR-H1 Kabat | DTYIH |
| 10 | Anti-Her2-CDR-H2 Kabat | RIYPTNGYTRYADSVKG |
| 11 | Anti-Her2-CDR-H1 IMGT | GFNIKDTY |
| 12 | Anti-Her2-CDR-H2 IMGT | IYPTNGYT |
| 13 | Anti-Her2-CDR-H3 IMGT | SRWGGDGFYAMDY |
| 14 | Anti-Her2-CDR-L1 IMGT | QDVNTA |
| 15 | Anti-Her2-CDR-L2 IMGT | SA |
| 16 | Anti-Her2-CDR-H1 AbM | GFNIKDTYIH |
| 17 | Anti-Her2-CDR-H2 AbM | RIYPTNGYTR |
| 18 | Anti-Her2-VH | |
| 19 | Anti-Her2-VL | |
| 20 | Anti-Her2-CH | ASTKGPSVFPLAPSSKSTSGGTAALGCLVKDYFPEPVTVSWNSG ALTSGVHTFPAVLQSSGLYSLSSVVTVPSSSLGTQTYICNVNHK PSNTKVDKKVEPKSCDKTHTCPPCPAPELLGGPSVFLFPPKPKD TLMISRTPEVTCVVVDVSHEDPEVKFNWYVDGVEVHNAKTKP REEQYNSTYRVVSVLTVLHQDWLNGKEYKCKVSNKALPAPIE KTISKAKGQPREPQVYTLPPSREEMTKNQVSLTCLVKGFYPSDI AVEWESNGQPENNYKTTPPVLDSDGSFFLYSKLTVDKSRWQQ GNVFSCSVMHEALHNHYTQKSLSLSPGK |
| 21 | Anti-Her2-CL | |

### I. Intermediate Preparation Examples

### Intermediate Preparation Example 1.1: Preparation of Ethyl 3-(6-methoxy-N-methyl-5-(3-(tosyloxy)propoxy)benzo[b]thiophene-2-carboxamido)propanoate

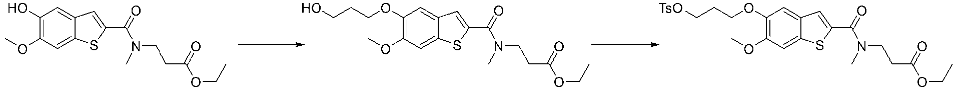

### Step 1: Preparation of ethyl 3-(5-(3-hydroxypropoxy)-6-methoxy-N-methylbenzo[b]thiophene-2-carboxamido)propanoate

Ethyl 3-(5-hydroxy-6-methoxy-*N*-methylbenzo[b]thiophene-2-carboxamido)propanoate (7.1 g, 21.0 mmol), 3-bromo-1-propanol (3.5 g, 25.2 mmol), potassium iodide (0.17 g, 1.05 mmol), and potassium carbonate (4.64 g, 33.6 mmol) were sequentially added to N,N-dimethylformamide (75 mL), and the mixture was slowly heated to 50 °C and stirred for 12 h. After the reaction system was cooled to room temperature, the reaction solution was poured into water (300 mL) and extracted three times with ethyl acetate (300 mL). The organic phases were combined, washed three times with saturated brine (100 mL), dried over anhydrous sodium sulfate, and concentrated to give the title compound (6.3 g, yield: 75.9%).

MS m/z (ESI): 396.2 [M+H]⁺.

### Step 2: Preparation of ethyl 3-(6-methoxy-N-methyl-5-(3-(tosyloxy)propoxy)benzo[b]thiophene-2-carboxamido)propanoate

Ethyl 3-(5-(3-hydroxypropoxy)-6-methoxy-*N*-methylbenzo[*b*]thiophene-2-carboxamido)propanoate (6.0 g, 15.2 mmol) and triethylamine (7.7 g, 76.0 mmol) were sequentially added to dichloromethane (100 mL), and the mixture was cooled in an ice-water bath. p-Toluenesulfonyl chloride (5.8 g, 30.4 mmol) was slowly added in batches to the reaction solution at 0 °C, and the mixture was slowly warmed to room temperature and stirred for 12 h. The reaction system was concentrated to remove the solvent, and the resulting residue was purified by silica gel column chromatography (eluent: ethyl acetate:petroleum ether = 1:1, volume ratio) to give the title compound (6.97 g, yield: 83.4%).

MS m/z (ESI): 550.1 [M+H]⁺.

### Intermediate Preparation Example 1.2: Preparation of Ethyl 3-(6-methoxy-N-methyl-5-(2-(tosyloxy)ethoxy)benzo[b]thiophene-2-carboxamido)propanoate

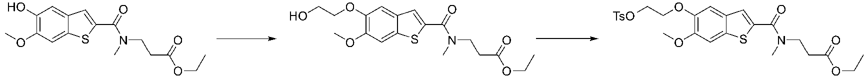

The title compound (136 mg, yield: 81.9%) was obtained by using the synthesis scheme in Intermediate Preparation Example 1.1 and replacing the starting material of the reaction 3-bromo-1-propanol with 2-bromoethanol.

MS m/z (ESI): 536.1 [M+H]⁺.

### Intermediate Preparation Example 1.3: Preparation of Ethyl 3-(6-methoxy-N-methyl-5-(4-(tosyloxy)butoxy)benzo[b]thiophene-2-carboxamido)propanoate

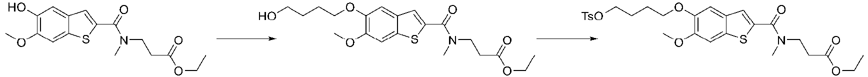

The title compound (25 mg, yield: 35.9%) was obtained by using the synthesis scheme in Intermediate Preparation Example 1.1 and replacing the starting material of the reaction 3-bromo-1-propanol with 4-bromo-1-butanol.

MS m/z (ESI): 564.2 [M+H]⁺.

### Intermediate Preparation Example 1.4: Preparation of Ethyl 3-(5-methoxy-N-methyl-6-(3-(tosyloxy)propoxy)benzo[b]thiophene-2-carboxamido)propanoate

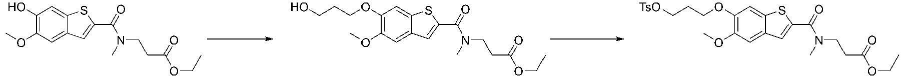

The title compound (25 mg, yield: 35.9%) was obtained by using the synthesis scheme in Intermediate Preparation Example 1.1 and replacing the starting material of the reaction ethyl 3-(5-hydroxy-6-methoxy-*N*-methylbenzo[*b*]thiophene-2-carboxamido)propanoate with ethyl 3-(6-hydroxy-5-methoxy-*N*-methylbenzo[*b*]thiophene-2-carboxamido)propanoate.

MS m/z (ESI): 550.1 [M+H]⁺.

### Intermediate Preparation Example 1.5: Preparation of Ethyl 4-(5-hydroxy-6-(methoxymethoxy)benzo[b]thiophen-2-yl)-4-oxobutanoate

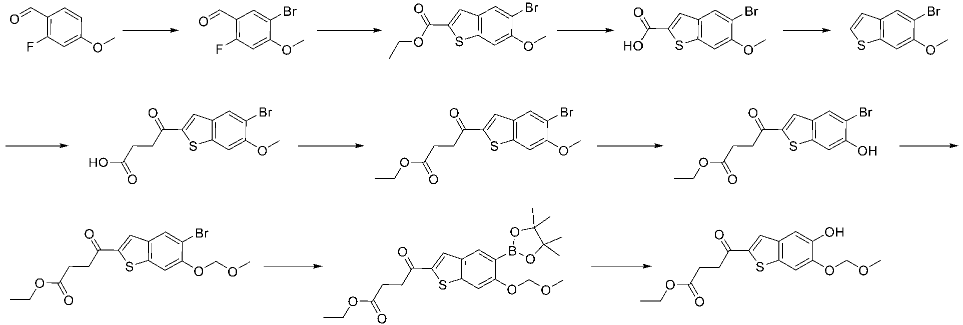

### Step 1: Preparation of 5-bromo-2-fluoro-4-methoxybenzaldehyde

2-Fluoro-4-methoxybenzaldehyde (20.0 g, 0.13 mol) was completely dissolved in methanol (150 mL), and the solution was cooled in an ice-water bath. Bromine (41.5 g, 0.26 mol) was slowly added dropwise to the reaction solution at 0 °C. After the addition, the mixture was slowly warmed to room temperature and stirred for 12 h. A saturated aqueous sodium bisulfite solution (300 mL) was slowly added to the reaction solution, and water (150 mL) was further added until a white solid precipitated. The mixture was filtered under vacuum, and the filter cake was washed with water (200 mL) and dried *in vacuo* at 50 °C to give the title compound (29.3 g, yield: 96.7%).

MS m/z (ESI): 234.9 [M+H]⁺.

### Step 2: Preparation of ethyl 5-bromo-6-methoxybenzo[b]thiophene-2-carboxylate

5-Bromo-2-fluoro-4-methoxybenzaldehyde (28.0 g, 0.12 mol), ethyl thioglycolate (15.9 g, 0.13 mol), and potassium carbonate (33.2 g, 0.24 mol) were sequentially added to *N,N*-dimethylacetamide (250 mL), and the mixture was heated to 100 °C and stirred for 12 h. After the reaction system was cooled to room temperature, the reaction solution was slowly poured into water (1000 mL), and a white solid precipitated. The mixture was filtered under vacuum, and the filter cake was washed with water (200 mL) and dried *in vacuo* at 50 °C to give the title compound (28.0 g, yield: 74.0%).

MS m/z (ESI): 315.0 [M+H]⁺.

### Step 3: Preparation of 5-bromo-6-methoxybenzo[b]thiophene-2-carboxylic acid

Ethyl 5-bromo-6-methoxybenzo[b]thiophene-2-carboxylate (28.0 g, 88.8 mmol) was completely dissolved in a mixed solvent of methanol (150 mL) and tetrahydrofuran (150 mL). In addition, sodium hydroxide (17.8 g, 0.44 mol) was completely dissolved in water (50 mL). The aqueous sodium hydroxide solution was slowly added dropwise to the reaction system at 0 °C, and the mixture was slowly warmed to room temperature and stirred for 2 h. Water (200 mL) was added to the reaction solution, and the mixed system was concentrated under reduced pressure at 40 °C to remove the organic solvent. The pH of the residue was adjusted to 4 with dilute hydrochloric acid (1 N, 300 mL), and filtration was performed under vacuum. The filter cake was washed with water (300 mL) and dried *in vacuo* at 50 °C. The resulting residue was purified by slurrying it with methyl tert-butyl ether (100 mL) to give the title compound (24.2 g, yield: 94.8%).

MS m/z (ESI): 286.9 [M+H]⁺.

### Step 4: Preparation of 5-bromo-6-methoxybenzo[b]thiophene

5-Bromo-6-methoxybenzo[b]thiophene-2-carboxylic acid (24.0 g, 83.6 mmol) and cuprous oxide (23.9 g, 167.2 mmol) were sequentially added to *N,N* -dimethylacetamide (250 mL), and the mixture was heated to 180 °C and stirred for 4 h. The reaction system was cooled to room temperature and then filtered under vacuum. Water (1000 mL) was added to the filtrate, and extraction was performed three times with ethyl acetate (500 mL). The organic phases were combined, washed three times with saturated brine (200 mL), dried over anhydrous sodium sulfate, and concentrated. The resulting residue was purified by silica gel column chromatography (eluent: ethyl acetate:petroleum ether = 1:20, volume ratio) to give the title compound (17.1 g, yield: 84.2%).

MS m/z (ESI): 242.9 [M+H]⁺.

### Step 5: Preparation of 4-(5-bromo-6-methoxybenzo[b]thiophen-2-yl)-4-oxobutanoic acid

Succinic anhydride (4.2 g, 42.0 mmol) was added to dichloromethane (200 mL), and the mixture was cooled in an ice-water bath. Aluminum trichloride powder (16.8 g, 126.0 mmol) was slowly added in batches to the reaction solution at 0 °C. In addition, 5-bromo-6-methoxybenzo[*b*]thiophene (10.2 g, 42.0 mmol) was completely dissolved in dichloromethane (100 mL), and the solution was slowly added dropwise to the reaction system at 0 °C. After the addition, the mixture was slowly warmed to room temperature and stirred for 1 h. Dilute hydrochloric acid (1 N, 300 mL) was slowly added to the reaction solution, and extraction was performed three times with dichloromethane (200 mL). The organic phases were combined, washed three times with saturated brine (150 mL), dried over anhydrous sodium sulfate, and concentrated to give the title compound (12.4 g, yield: 86.2%).

MS m/z (ESI): 343.0 [M+H]⁺.

### Step 6: Preparation of ethyl 4-(5-bromo-6-methoxybenzo[b]thiophen-2-yl)-4-oxobutanoate

4-(5-Bromo-6-methoxybenzo[*b*]thiophen-2-yl)-4-oxobutanoic acid (12.4 g, 36.1 mmol) was completely dissolved in absolute ethanol (150 mL), and the solution was cooled in an ice-water bath. Thionyl chloride (21.5 g, 180.7 mmol) was slowly added dropwise to the reaction solution at 0 °C. After the addition, the mixture was slowly warmed to room temperature and stirred for 4 h, and concentrated to remove the solvent. The residue was dissolved in ethyl acetate (300 mL), and the solution was washed three times with saturated brine (100 mL). The organic phase was dried over anhydrous sodium sulfate and concentrated. The resulting residue was purified by silica gel column chromatography (eluent: dichloromethane:petroleum ether = 2:1, volume ratio) to give the title compound (13.0 g, yield: 97.0%).

MS m/z (ESI): 371.0 [M+H]⁺.

### Step 7: Preparation of ethyl 4-(5-bromo-6-hydroxybenzo[b]thiophen-2-yl)-4-oxobutanoate

Ethyl 4-(5-bromo-6-methoxybenzo[b]thiophen-2-yl)-4-oxobutanoate (10.0 g, 26.9 mmol) and aluminum trichloride (35.9 g, 269.4 mmol) were sequentially added to dichloromethane (300 mL), and the mixture was heated to 40 °C and stirred for 12 h. After the reaction system was cooled to room temperature, dilute hydrochloric acid (1 N, 100 mL) was added to the reaction solution. The mixed system was concentrated under reduced pressure at 40 °C to remove the organic solvent, and ethanol (300 mL) and a solution of hydrogen chloride in 1,4-dioxane (4 N, 20 mL) were added to the residue. The mixture was heated to 60 °C and stirred for 2 h. The reaction system was cooled to room temperature and then concentrated to remove the solvent, and the resulting residue was purified by silica gel column chromatography (eluent: ethyl acetate:petroleum ether = 1:4, volume ratio) to give the title compound (7.2 g, yield: 75.0%).

MS m/z (ESI): 357.1 [M+H]⁺.

### Step 8: Preparation of ethyl 4-(5-bromo-6-(methoxymethoxy)benzo[b]thiophen-2-yl)-4-oxobutanoate

Ethyl 4-(5-bromo-6-hydroxybenzo[*b*]thiophen-2-yl)-4-oxobutanoate (7.0 g, 19.6 mmol) and *N,N-*diisopropylethylamine (7.6 g, 58.8 mmol) were sequentially added to tetrahydrofuran (150 mL), and the mixture was cooled in an ice-water bath. Bromomethyl methyl ether (4.9 g, 39.2 mmol) was slowly added dropwise to the reaction solution at 0 °C, and the mixture was slowly warmed to room temperature and stirred for 4 h. The reaction system was concentrated to remove the solvent. The resulting residue was dissolved in ethyl acetate (200 mL), and the solution was washed three times with saturated brine (100 mL). The organic phase was dried over anhydrous sodium sulfate and concentrated to give the title compound (7.5 g, yield: 95.4%).

MS m/z (ESI): 401.0 [M+H]⁺.

### Step 9: Preparation of ethyl 4-(6-(methoxymethoxy)-5-(4,4,5,5-tetramethyl-1,3,2-dioxaborolan-2-yl)benzo[b]thiophen-2-yl)-4-oxobutanoate

Ethyl 4-(5-bromo-6-(methoxymethoxy)benzo[b]thiophen-2-yl)-4-oxobutanoate (7.0 g, 17.4 mmol), bis(pinacolato)diboron (8.9 g, 34.9 mmol), potassium acetate (3.4 g, 34.9 mmol), and [1,1'-bis(diphenylphosphino)ferrocene]palladium(II) dichloride (0.63 g, 0.87 mmol) were sequentially added to 1,4-dioxane (100 mL). After nitrogen was bubbled through the reaction system for 5 min, the reaction system was heated to 100 °C and stirred for 4 h. The reaction system was cooled to room temperature and then concentrated to remove the solvent. The resulting residue was dissolved in ethyl acetate (200 mL), and the solution was washed three times with saturated brine (100 mL). The organic phase was dried over anhydrous sodium sulfate and concentrated to give the title compound (6.8 g, yield: 87.3%).

MS m/z (ESI): 449.1 [M+H]⁺.

### Step 10: Preparation of ethyl 4-(5-hydroxy-6-(methoxymethoxy)benzo[b]thiophen-2-yl)-4-oxobutanoate

Ethyl 4-(6-(methoxymethoxy)-5-(4,4,5,5-tetramethyl-1,3,2-dioxaborolan-2-yl)benzo[*b*]thiophen-2-yl)-4-oxobutanoate (6.5 g, 14.5 mmol) was completely dissolved in a mixed solution of acetone (280 mL) and water (70 mL). After potassium peroxymonosulfate (25.1 g, 72.5 mmol) was slowly added in batches at room temperature, the reaction system was stirred at room temperature for 12 h. The reaction system was concentrated to remove acetone as much as possible. The resulting residue was dissolved in ethyl acetate (150 mL), and the solution was washed three times with saturated brine (60 mL). The resulting residue was purified by silica gel column chromatography (eluent: ethyl acetate:petroleum ether = 1:2, volume ratio) to give the title compound (2.1 g, yield: 43.8%).

MS m/z (ESI): 339.1 [M+H]⁺.

### Intermediate Preparation Example 1.6: Preparation of Ethyl 4-(6-hydroxy-5-(2-oxo-2-phenylethoxy)benzo[b]thiophen-2-yl)-4-oxobutanoate

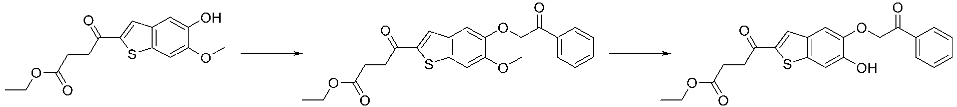

### Step 1: Preparation of ethyl 4-(6-methoxy-5-(2-oxo-2-phenylethoxy)benzo[b]thiophen-2-yl)-4-oxobutanoate

Ethyl 4-(5-hydroxy-6-methoxybenzo[b]thiophen-2-yl)-4-oxobutanoate (25 g, 80.27 mmol) was dissolved in *N,N*-dimethylformamide (100 mL), and 2-bromo-1-phenylethan-1-one (19.56 g, 96.32 mmol) and potassium carbonate (22.41 g, 160.53 mmol) were added. The mixture was stirred at room temperature for 6 h. The reaction solution was slowly poured into water (2500 mL), and a yellow solid precipitated. The mixture was filtered under vacuum, and the filter cake was washed three times with water (600 mL) and dried *in vacuo* at 50 °C to give the title compound (33.0 g, yield: 95.4%).

MS m/z (ESI): 427.1 [M+H]⁺.

### Step 2: Preparation of ethyl 4-(6-hydroxy-5-(2-oxo-2-phenylethoxy)benzo[b]thiophen-2-yl)-4-oxobutanoate

Ethyl 4-(6-methoxy-5-(2-oxo-2-phenylethoxy)benzo[*b*]thiophen-2-yl)-4-oxobutanoate (10 g, 23.21 mmol) and anhydrous aluminum chloride (12.51 g, 92.85 mmol) were added to 1,2-dichloroethane (80 mL), and the mixture was stirred at 80 °C for 2 h. Hydrochloric acid (2 N, 80 mL) was added to the reaction solution. After 10 min of stirring, the mixture was extracted three times with dichloromethane (80 mL). The organic phases were combined, dried over anhydrous sodium sulfate, and filtered. The filtrate was concentrated, and the resulting crude product was slurried with methyl tert-butyl ether to give the title compound (8.0 g, yield: 83.7%).

MS m/z (ESI): 413.1 [M+H]⁺.

### Intermediate Preparation Example 1.7: Preparation of Ethyl 3-(6-bromo-5-hydroxy-N-methylbenzo[b]thiophene-2-carboxamido)propanoate

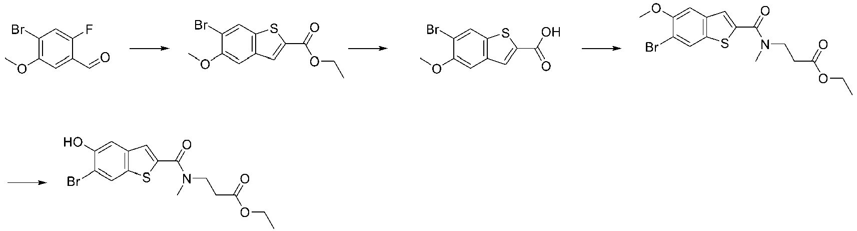

### Step 1: Preparation of ethyl 6-bromo-5-methoxybenzo[b]thiophene-2-carboxylate

4-Bromo-2-fluoro-5-methoxybenzaldehyde (5.0 g, 21.46 mmol), ethyl thioglycolate (3.1 g, 25.79 mmol), and potassium carbonate (8.9 g, 64.45 mmol) were sequentially added to *N,N-*dimethylacetamide (50 mL), and the mixture was heated to 60 °C and stirred for 15 h. After the reaction system was cooled to room temperature, the reaction solution was slowly poured into water (250 mL), and a white solid precipitated. The mixture was filtered under vacuum, and the filter cake was washed with water (200 mL) and dried *in vacuo* at 50 °C to give the title compound (6.0 g, yield: 88.7%).

MS m/z (ESI): 315.0 [M+H]⁺.

### Step 2: Preparation of 6-bromo-5-methoxybenzo[b]thiophene-2-carboxylic acid

Ethyl 6-bromo-5-methoxybenzo[*b*]thiophene-2-carboxylate (6.0 g, 19.04 mmol) was completely dissolved in a mixed solvent of methanol (25 mL) and tetrahydrofuran (25 mL). In addition, lithium hydroxide monohydrate (4.0 g, 95.24 mmol) was completely dissolved in water (10 mL). The aqueous lithium hydroxide solution was slowly added dropwise to the reaction system at 0 °C, and the mixture was slowly warmed to room temperature and stirred for 2 h. Water (50 mL) was added to the reaction solution, and the mixed system was concentrated under reduced pressure at 40 °C to remove the organic solvent. The pH of the residue was adjusted to 4 with dilute hydrochloric acid (1 N, 300 mL), and filtration was performed under vacuum. The filter cake was washed with water (100 mL) and dried *in vacuo* at 50 °C. The resulting residue was purified by slurrying it with methyl tert-butyl ether (20 mL) to give the title compound (2.7 g, yield: 49.4%).

MS m/z (ESI): 286.9 [M+H]⁺.

### Step 3: Preparation of ethyl 3-(6-bromo-5-methoxy-N-methylbenzo[b]thiophene-2-carboxamido)propanoate

6-Bromo-5-methoxybenzo[*b*]thiophene-2-carboxylic acid (1.5 g, 5.22 mmol) and ethyl 3-(methylamino)propanoate (1.0 g, 7.62 mmol) were dissolved in *N,N*-dimethylacetamide (20 mL), and then 1-(3-dimethylaminopropyl)-3-ethylcarbodiimide hydrochloride (1.53 g, 7.98 mol) and 4-dimethylaminopyridine (1.0 g, 8.18 mmol) were sequentially added. The mixture was stirred at 25 °C for 15 h. The reaction solution was poured into water (100 mL) and extracted three times with ethyl acetate (30 mL). The organic phases were combined, washed three times with saturated brine (10 mL), dried over anhydrous sodium sulfate, and concentrated. The resulting residue was purified by silica gel column chromatography (eluent: ethyl acetate:petroleum ether = 1:1, volume ratio) to give the title compound (1.5 g, yield: 71.8%).

MS m/z (ESI): 400.0 [M+H]⁺.

### Step 4: Preparation of ethyl 3-(6-bromo-5-hydroxy-N-methylbenzo[b]thiophene-2-carboxamido)propanoate

Ethyl 3-(6-bromo-5-methoxy-N-methylbenzo[*b*]thiophene-2-carboxamido)propanoate (860.0 mg, 2.15 mmol) was added to dichloromethane (30 mL), and a solution of boron tribromide in dichloromethane (1 M, 11 mL) was slowly added dropwise in batches to the reaction solution. The mixture was stirred at 25 °C for 12 h. Dilute hydrochloric acid (1 N, 10 mL) was added to the reaction solution, and the reaction solution was then poured into water (100 mL) and extracted three times with dichloromethane (30 mL). The organic phases were combined, washed three times with saturated brine (10 mL), dried over anhydrous sodium sulfate, and concentrated to give the title compound (800.0 mg, yield: 96.4%).

MS m/z (ESI): 386.0 [M+H]⁺.

### Intermediate Preparation Example 1.8: Preparation of 2,5-Dioxopyrrolidin-1-yl (6-(2,5-dioxo-2,5-dihydro-1H-pyrrol-1-yl)hexanoyl)glycylglycyl-L-phenylalaninate

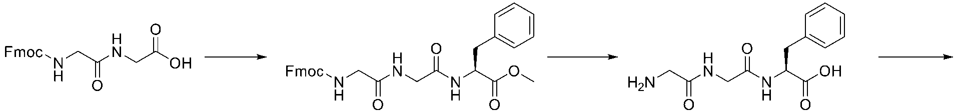

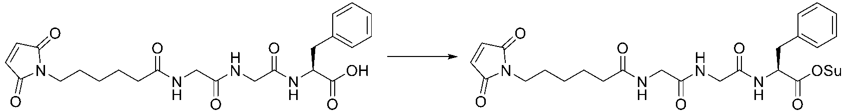

### Step 1: Preparation of methyl (((9H-fluoren-9-yl)methoxy)carbonyl)glycylglycyl-L-phenylalaninate

(((9*H*-Fluoren-9-yl)methoxy)carbonyl)glycylglycine (1.0 g, 2.82 mmol), methyl *L*-phenylalaninate (531.0 mg, 2.96 mmol), and *N*,*N*,*N*',*N*'-tetramethyl-O-(7-azabenzotriazol-1-yl)uronium hexafluorophosphate (1.18 g, 3.10 mmol) were added to *N,N*-dimethylformamide (10 mL), and *N,N-*diisopropylethylamine (728.9 mg, 5.64 mmol) was added at room temperature. The reaction system was stirred at room temperature for 2 h. The reaction solution was poured into water (150 mL) and extracted three times with ethyl acetate (100 mL). The organic phases were combined, washed three times with saturated brine (60 mL), dried over anhydrous sodium sulfate, and concentrated. The resulting residue was purified by silica gel column chromatography (eluent: 100% ethyl acetate) to give the title compound (1.28 g, yield: 88.0%).

MS m/z (ESI): 516.2 [M+H]⁺.

### Step 2: Preparation of glycylglycyl-L-phenylalanine

Methyl (((9*H*-fluoren-9-yl)methoxy)carbonyl)glycylglycyl-*L*-phenylalaninate (1.2 g, 2.33 mmol) was completely dissolved in a mixed solvent of tetrahydrofuran (15 mL) and methanol (15 mL). In addition, lithium hydroxide monohydrate (0.49 g, 11.64 mmol) was completely dissolved in water (2 mL). The aqueous lithium hydroxide solution was slowly added dropwise to the reaction system at 0 °C, and the reaction solution was slowly warmed to room temperature and stirred for 2 h. Water (25 mL) was added to the reaction solution, and the mixed system was concentrated under reduced pressure at 40 °C to remove the organic solvent. The pH of the residue was adjusted to 4-5 with dilute hydrochloric acid (1 N, 100 mL), and filtration was performed under vacuum. The resulting filtrate was concentrated under reduced pressure to give the title compound (0.44 g, yield: 68.2%).

MS m/z (ESI): 280.1 [M+H]⁺.

### Step 3: Preparation of (6-(2,5-dioxo-2,5-dihydro-1H-pyrrol-1-yl)hexanoyl)glycylglycyl-L-phenylalanine

Glycylglycyl-*L*-phenylalanine (1.5 g, 5.37 mmol) and *N,N*-diisopropylethylamine (1.39 g, 10.74 mmol) were dissolved in *N,N*-dimethylformamide (15 mL), and 2,5-dioxopyrrolidin-1-yl 6-(2,5-dioxo-2,5-dihydro-1*H*-pyrrol-1-yl)hexanoate (1.66 g, 5.37 mmol) was added in batches to the reaction solution at room temperature. The mixture was stirred at room temperature for 12 h. The reaction solution was concentrated to remove the solvent, and the resulting residue was purified by preparative high performance liquid chromatography to give the title compound (1.2 g, yield: 47.2%).

MS m/z (ESI): 473.2 [M+H]⁺.

### Step 4: Preparation of 2,5-dioxopyrrolidin-1-yl (6-(2,5-dioxo-2,5-dihydro-1H-pyrrol-1-yl)hexanoyl)glycylglycyl-L-phenylalaninate

(6-(2,5-Dioxo-2,5-dihydro-1*H*-pyrrol-1-yl)hexanoyl)glycylglycyl-*L*-phenylalanine (1.0 g, 2.12 mmol) and *N*-hydroxysuccinimide (0.29 g, 2.54 mmol) were dissolved in *N,N-*dimethylformamide (10 mL), and 1-(3-dimethylaminopropyl)-3-ethylcarbodiimide hydrochloride (0.61 g, 3.18 mmol) was added in batches to the reaction solution at room temperature. The mixture was stirred at room temperature for 12 h. The reaction solution was concentrated to remove the solvent, and the resulting residue was purified by preparative high performance liquid chromatography to give the title compound (0.95 g, yield: 78.7%).

MS m/z (ESI): 570.1 [M+H]⁺.

### Intermediate Preparation Example 1.9: Preparation of Benzyl 4-((2S,5S)-38-azido-5-isopropyl-4,7,11-trioxo-2-(3-ureidopropyl)-9,15,18,21,24,27,30,33,36-nonaoxa-3,6,12-triazaoctatriacontanamido)methyl(2-(methylamino)ethyl)carbamate

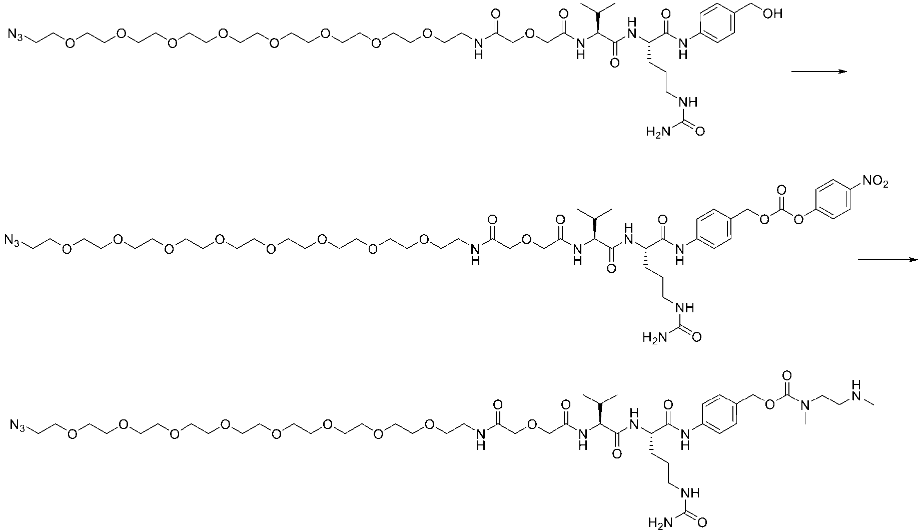

### Step 1: Preparation of 4-((2S,5S)-38-azido-5-isopropyl-4,7,11-trioxo-2-(3-ureidopropyl)-9,15,18,21,24,27,30,33,36-nonaoxa-3,6,12-triazaoctatriacontanamido)benzyl (4-nitrophenyl)carbonate

(*S*)-2-((*S*)-35-Azido-2-isopropyl-4,8-dioxo-6,12,15,18,21,24,27,30,33-nonaoxa-3,9-diazapentatriacontanamido)-*N*-(4-(hydroxymethyl)phenyl)-5-ureidopentanamide (1.2 g, 1.31 mmol) and *N,N*-diisopropylethylamine (0.34 g, 2.62 mmol) were dissolved in *N,N*-dimethylformamide (5 mL), and the solution was cooled in an ice-water bath. Bis(p-nitrophenyl) carbonate (0.6 g, 1.97 mmol) was added to the reaction solution at 0 °C, and the mixture was slowly warmed to room temperature and stirred for 3 h. The reaction solution was poured into water (50 mL) and extracted three times with ethyl acetate (60 mL). The organic phases were combined, washed three times with saturated brine (30 mL), dried over anhydrous sodium sulfate, and concentrated. The resulting residue was purified by silica gel column chromatography (eluent: ethyl acetate:petroleum ether = 1:1, volume ratio) to give the title compound (0.9 g, yield: 63.8%).

MS m/z (ESI): 1081.5 [M+H]⁺.

### Step 2: Preparation of benzyl 4-((2S,5S)-38-azido-5-isopropyl-4,7,11-trioxo-2-(3-ureidopropyl)-9,15,18,21,24,27,30,33,36-nonaoxa-3,6,12-triazaoctatriacontanamido)methyl(2-(methylamino)ethyl)carbamate

4-((2*S*,5*S*)-38-Azido-5-isopropyl-4,7,11-trioxo-2-(3-ureidopropyl)-9,15,18,21,24,27,30,33,36-nonaoxa-3,6,12-triazaoctatriacontanamido)benzyl (4-nitrophenyl)carbonate (350.0 mg, 0.32 mmol) and *N,N-*diisopropylethylamine (125.5 mg, 0.97 mmol) were dissolved in *N,N*-dimethylformamide (6 mL), and the solution was cooled in an ice-water bath. A solution of *N,N*'-dimethylethylenediamine (225.7 mg, 2.56 mmol) in *N,N*-dimethylformamide (1 mL) was slowly added dropwise to the reaction solution at 0 °C, and the mixture was slowly warmed to room temperature and stirred for 2 h. The reaction solution was purified by preparative high performance liquid chromatography to give the title compound (160.5 mg, yield: 48.7%).

MS m/z (ESI): 1030.6 [M+H]⁺.

### Intermediate Preparation Example 1.10: Preparation of 4-(6-((2-Aminoacetamido)methoxy)-5-(3-((2-((2-carboxyethyl)(methyl)carbamoyl)-6-methoxybenzo[b]thiophen-5-yl)oxy)propoxy)benzo[b]thiophen-2-yl)-4-oxobutanoic acid

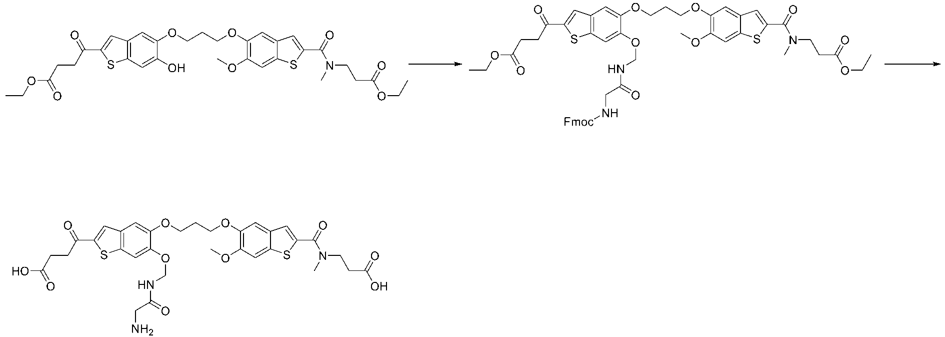

### Step 1: Preparation of ethyl 4-(6-((2-((((9H-fluoren-9-yl)methoxy)carbonyl)amino)acetamido)methoxy)-5-(3-((2-((3-ethoxy-3-oxopropyl)(methyl)carbamoyl)-6-methoxybenzo[b]thiophen-5-yl)oxy)propoxy)benzo[b]thiophen-2-yl)-4-oxobutanoate

Ethyl 4-(5-(3-((2-((3-ethoxy-3-oxopropyl)(methyl)carbamoyl)-6-methoxybenzo[b]thiophen-5-yl)oxy)propoxy)-6-hydroxybenzo[b]thiophen-2-yl)-4-oxobutanoate (464 mg, 0.69 mmol) was dissolved in trifluoroacetic acid (12 mL), and methyl (2-((((9*H*-fluoren-9-yl)methoxy)carbonyl)amino)acetamido)acetate (508 mg, 1.38 mmol) was slowly added in four batches to the reaction system at room temperature at intervals of 0.5 h. The reaction system was stirred at room temperature for 1 h. The reaction system was concentrated to remove the solvent, and the resulting residue was purified by silica gel column chromatography (eluent: dichloromethane:ethyl acetate = 1:1, volume ratio) to give the title compound (254.3 mg, yield: 37.6%).

MS m/z (ESI): 980.2 [M+H]⁺.

### Step 2: Preparation of 4-(6-((2-aminoacetamido)methoxy)-5-(3-((2-((2-carboxyethyl)(methyl)carbamoyl)-6-methoxybenzo[b]thiophen-5-yl)oxy)propoxy)benzo[b]thiophen-2-yl)-4-oxobutanoic acid

Ethyl 4-(6-((2-((((9*H*-fluoren-9-yl)methoxy)carbonyl)amino)acetamido)methoxy)-5-(3-((2-((3-ethoxy-3-oxopropyl)(methyl)carbamoyl)-6-methoxybenzo[*b*]thiophen-5-yl)oxy)propoxy)benzo[b]thiophen-2-yl)-4-oxobutanoate (245 mg, 0.25 mmol) was dissolved in a mixed solvent of methanol (2 mL), tetrahydrofuran (2 mL), and water (0.5 mL), and lithium hydroxide monohydrate (31.5 mg, 0.75 mmol) was added to the reaction solution at room temperature. The mixture was stirred at room temperature for 4 h. The reaction system was concentrated to remove the solvent, and water (5 mL) was added to the residue. The pH was adjusted to 5 with dilute hydrochloric acid (1 N, 2 mL), and filtration was performed under vacuum. The filter cake was washed with water (5 mL) and dried *in vacuo* at 50 °C to give the title compound (115 mg, yield: 65.5%).

MS m/z (ESI): 702.0 [M+H]⁺.

### Intermediate Preparation Example 1.11: Preparation of Ethyl 4-(6-((2-aminoacetamido)methoxy)-5-(3-((2-((3-ethoxy-3-oxopropyl)(methyl)carbamoyl)-6-methoxybenzo[b]thiophen-5-yl)oxy)propoxy)benzo[b]thiophen-2-yl)-4-oxobutanoate

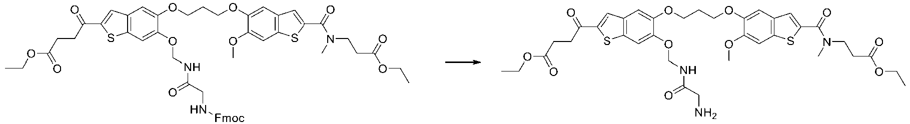

Ethyl 4-(6-((2-((((9*H*-fluoren-9-yl)methoxy)carbonyl)amino)acetamido)methoxy)-5-(3-((2-((3-ethoxy-3-oxopropyl)(methyl)carbamoyl)-6-methoxybenzo[b]thiophen-5-yl)oxy)propoxy)benzo[*b*]thiophen-2-yl)-4-oxobutanoate (140 mg, 142.84 µmol) and diethylamine (31.34 mg, 428.49 µmol) were dissolved in *N,N*-dimethylformamide (2 mL), and the solution was stirred at 20 °C for 3 h. The resulting reaction solution was purified by preparative high performance liquid chromatography to give the title compound (50 mg, yield: 46.2%).

MS m/z (ESI): 758.2 [M+H]⁺.

### Intermediate Preparation Example 1.12: Preparation of Ethyl (S)-4-(6-((2-aminopropanamido)methoxy)-5-(3-((2-((3-ethoxy-3-oxopropyl)(methyl)carbamoyl)-5-methoxybenzo[b]thiophen-6-yl)oxy)propoxy)benzo[b]thiophen-2-yl)-4-oxobutanoate

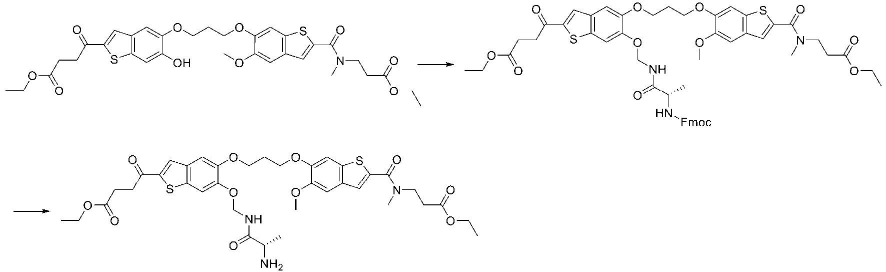

### Step 1: Preparation of ethyl (S)-4-(6-((2-((((9H-fluoren-9-yl)methoxy)carbonyl)amino)propanamido)methoxy)-5-(3-((2-((3-ethoxy-3-oxopropyl)(methyl)carbamoyl)-5-methoxybenzo[b]thiophen-6-yl)oxy)propoxy)benzo[b]thiophen-2-yl)-4-oxobutanoate

The title compound (300 mg, yield: 40.5%) was obtained by using the synthesis method of step 1 in Intermediate Preparation Example 1.10, replacing the starting material of the reaction of step 1 ethyl 4-(5-(3-((2-((3-ethoxy-3-oxopropyl)(methyl)carbamoyl)-6-methoxybenzo[*b*]thiophen-5-yl)oxy)propoxy)-6-hydroxybenzo[*b*]thiophen-2-yl)-4-oxobutanoate with ethyl 4-(5-(3-((2-((3-ethoxy-3-oxopropyl)(methyl)carbamoyl)-5-methoxybenzo[*b*]thiophen-6-yl)oxy)propoxy)-6-hydroxybenzo[*b*]thiophen-2-yl)-4-oxobutanoate, and replacing methyl (2-((((9*H*-fluoren-9-yl)methoxy)carbonyl)amino)acetamido)acetate with methyl (*S*)-(2-((((9*H*-fluoren-9-yl)methoxy)carbonyl)amino)propanamido)acetate.

MS m/z (ESI): 994.3 [M+H]⁺.

### Step 2: Preparation of ethyl (S)-4-(6-((2-aminopropanamido)methoxy)-5-(3-((2-((3-ethoxy-3-oxopropyl)(methyl)carbamoyl)-5-methoxybenzo[b]thiophen-6-yl)oxy)propoxy)benzo[b]thiophen-2-yl)-4-oxobutanoate

The title compound (100 mg, yield: 42.9%) was obtained by using the synthesis method in Intermediate Preparation Example 1.11 and replacing the starting material of the reaction ethyl 4-(6-((2-((((9*H*-fluoren-9-yl)methoxy)carbonyl)amino)acetamido)methoxy)-5-(3-((2-((3-ethoxy-3-oxopropyl)(methyl)carbamoyl)-6-methoxybenzo[*b*]thiophen-5-yl)oxy)propoxy)benzo[*b*]thiophen-2-yl)-4-oxobutanoate with ethyl (*S*)-4-(6-((2-((((9*H*-fluoren-9-yl)methoxy)carbonyl)amino)propanamido)methoxy)-5-(3-((2-((3-ethoxy-3-oxopropyl)(methyl)carbamoyl)-5-methoxybenzo[b]thiophen-6-yl)oxy)propoxy)benzo[*b*]thiophen-2-yl)-4-oxobutanoate.

MS m/z (ESI): 772.2 [M+H]⁺.

### Intermediate Preparation Example 1.13: Preparation of Ethyl (S)-4-(6-((2-aminopropanamido)methoxy)-5-(3-((2-((3-ethoxy-3-oxopropyl)(methyl)carbamoyl)-6-methoxybenzo[b]thiophen-5-yl)oxy)propoxy)benzo[b]thiophen-2-yl)-4-oxobutanoate

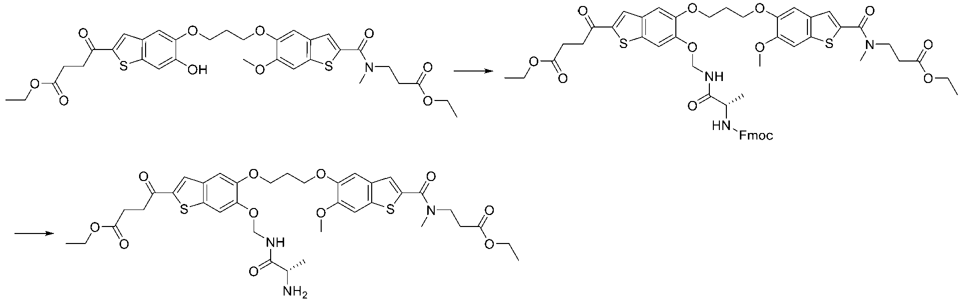

The title compound (224 mg, yield: 55.9%) was obtained by using the synthesis scheme in Intermediate Preparation Example 1.12 and replacing the starting material of the reaction of step 1 ethyl 4-(5-(3-((2-((3-ethoxy-3-oxopropyl)(methyl)carbamoyl)-5-methoxybenzo[*b*]thiophen-6-yl)oxy)propoxy)-6-hydroxybenzo[*b*]thiophen-2-yl)-4-oxobutanoate with ethyl 4-(5-(3-((2-((3-ethoxy-3-oxopropyl)(methyl)carbamoyl)-6-methoxybenzo[*b*]thiophen-5-yl)oxy)propoxy)-6-hydroxybenzo[*b*]thiophen-2-yl)-4-oxobutanoate.

MS m/z (ESI): 772.2 [M+H]⁺.

### Intermediate Preparation Example 1.14: Preparation of Ethyl 4-(5-(3-((2-((3-ethoxy-3-oxopropyl)(methyl)carbamoyl)-6-methoxybenzo[b]thiophen-5-yl)oxy)propoxy)-6-((methyl(2-(methylamino)ethyl)carbamoyl)oxy)benzo[b]thiophen-2-yl)-4-oxobutanoate

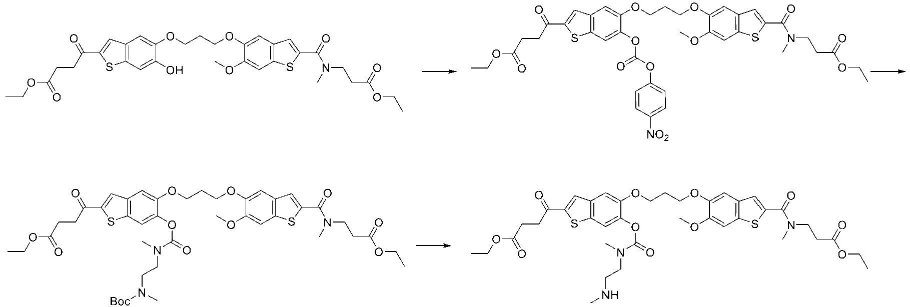

### Step 1: Preparation of ethyl 4-(5-(3-((2-((3-ethoxy-3-oxopropyl)(methyl)carbamoyl)-6-methoxybenzo[b]thiophen-5-yl)oxy)propoxy)-6-(((4-nitrophenoxy)carbonyl)oxy)benzo[b]thiophen-2-yl)-4-oxobutanoate

Ethyl 4-(5-(3-((2-((3-ethoxy-3-oxopropyl)(methyl)carbamoyl)-6-methoxybenzo[*b*]thiophen-5-yl)oxy)propoxy)-6-hydroxybenzo[b]thiophen-2-yl)-4-oxobutanoate (300 mg, 446.57 µmol) and *N,N-*diisopropylethylamine (116.6 mg, 902.20 µmol) were dissolved in *N,N*-dimethylformamide (3 mL), and the solution was cooled in an ice-water bath. Bis(4-nitrophenyl) carbonate (286 mg, 940.14 µmol) was added to the reaction solution at 0 °C, and the mixture was slowly warmed to room temperature and stirred for 3 h. The reaction solution was poured into water (10 mL) and extracted three times with ethyl acetate (6 mL). The organic phases were combined, washed three times with saturated brine (5 mL), dried over anhydrous sodium sulfate, and concentrated. The resulting residue was purified by silica gel column chromatography (eluent: ethyl acetate:petroleum ether = 1:1, volume ratio) to give the title compound (230 mg, yield: 61.5%).

MS m/z (ESI): 837.2 [M+H]⁺.

### Step 2: Preparation of ethyl 4-(6-(((2-((tert-butoxycarbonyl)(methyl)amino)ethyl)(methyl)carbamoyl)oxy)-5-(3-((2-((3-ethoxy-3-oxopropyl)(methyl)carbamoyl)-6-methoxybenzo[b]thiophen-5-yl)oxy)propoxy)benzo[b]thiophen-2-yl)-4-oxobutanoate

Ethyl 4-(5-(3-((2-((3-ethoxy-3-oxopropyl)(methyl)carbamoyl)-6-methoxybenzo[*b*]thiophen-5-yl)oxy)propoxy)-6-(((4-nitrophenoxy)carbonyl)oxy)benzo[b]thiophen-2-yl)-4-oxobutanoate (200.0 mg, 238.98 µmol) and *N,N*-diisopropylethylamine (98.0 mg, 758.28 µmol) were dissolved in *N,N-*dimethylformamide (4 mL), and the solution was cooled in an ice-water bath. A solution of tert-butyl methyl(2-(methylamino)ethyl)carbamate (58.5 mg, 310.72 µmol) in *N,N*-dimethylformamide (1 mL) was slowly added dropwise to the reaction solution at 0 °C, and the mixture was slowly warmed to room temperature and stirred for 2 h. The reaction solution was purified by preparative high performance liquid chromatography to give the title compound (182.0 mg, yield: 85.9%).

MS m/z (ESI): 886.3 [M+H]⁺.

### Step 3: Preparation of ethyl 4-(5-(3-((2-((3-ethoxy-3-oxopropyl)(methyl)carbamoyl)-6-methoxybenzo[b]thiophen-5-yl)oxy)propoxy)-6-((methyl(2-(methylamino)ethyl)carbamoyl)oxy)benzo[b]thiophen-2-yl)-4-oxobutanoate

Ethyl 4-(6-(((2-((tert-butoxycarbonyl)(methyl)amino)ethyl)(methyl)carbamoyl)oxy)-5-(3-((2-((3-ethoxy-3-oxopropyl)(methyl)carbamoyl)-6-methoxybenzo[*b*]thiophen-5-yl)oxy)propoxy)benzo[b]thiophen-2-yl)-4-oxobutanoate (90 mg, 101.58 µmol) was dissolved in dichloromethane (7 mL), and trifluoroacetic acid (0.6 mL) was added to the reaction solution at 0 °C. The mixture was slowly warmed to room temperature and stirred for 1 h. The reaction solution was diluted with dichloromethane (20 mL) and concentrated to remove the solvent to give the title compound (80.0 mg, yield: 87.5%).

MS m/z (ESI): 786.3 [M+H]⁺.

### II. Compound Examples

### Example 2.1: Preparation of Ethyl 4-(5-(3-((2-((3-ethoxy-3-oxopropyl)(methyl)carbamoyl)-6-methoxybenzo[b]thiophen-5-yl)oxy)propoxy)-6-hydroxybenzo[b]thiophen-2-yl)-4-oxobutanoate (C-2)

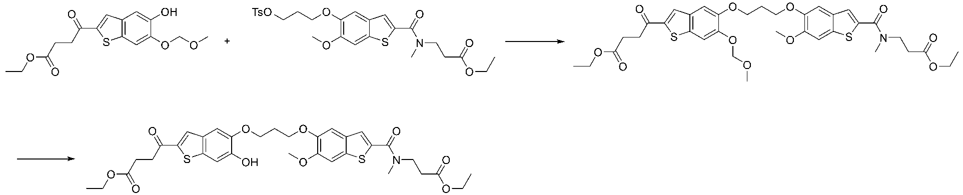

### Step 1: Preparation of ethyl 4-(5-(3-((2-((3-ethoxy-3-oxopropyl)(methyl)carbamoyl)-6-methoxybenzo[b]thiophen-5-yl)oxy)propoxy)-6-(methoxymethoxy)benzo[b]thiophen-2-yl)-4-oxobutanoate

Ethyl 4-(5-hydroxy-6-(methoxymethoxy)benzo[*b*]thiophen-2-yl)-4-oxobutanoate (2.0 g, 5.91 mmol), ethyl 3-(6-methoxy-*N*-methyl-5-(3-(tosyloxy)propoxy)benzo[*b*]thiophene-2-carboxamido)propanoate (3.25 g, 5.91 mmol), and potassium carbonate (1.63 g, 11.83 mmol) were sequentially added to *N,N-*dimethylformamide (50 mL), and the mixture was heated to 50 °C and stirred for 12 h. After the reaction system was cooled to room temperature, the reaction solution was poured into water (300 mL) and extracted three times with ethyl acetate (300 mL). The organic phases were combined, washed three times with saturated brine (100 mL), dried over anhydrous sodium sulfate, and concentrated. The resulting residue was purified by silica gel column chromatography (eluent: ethyl acetate:petroleum ether = 1:1, volume ratio) to give the title compound (3.2 g, yield: 75.6%).

MS m/z (ESI): 716.2 [M+H]⁺.

### Step 2: Preparation of ethyl 4-(5-(3-((2-((3-ethoxy-3-oxopropyl)(methyl)carbamoyl)-6-methoxybenzo[b]thiophen-5-yl)oxy)propoxy)-6-hydroxybenzo[b]thiophen-2-yl)-4-oxobutanoate

Ethyl 4-(5-(3-((2-((3-ethoxy-3-oxopropyl)(methyl)carbamoyl)-6-methoxybenzo[*b*]thiophen-5-yl)oxy)propoxy)-6-(methoxymethoxy)benzo[*b*]thiophen-2-yl)-4-oxobutanoate (3.0 g, 4.19 mmol) was completely dissolved in dichloromethane (50 mL). After trifluoroacetic acid (5 mL) was added at room temperature, the reaction system was stirred at room temperature for 2 h. The reaction system was concentrated to remove the solvent to give the title compound (2.68 g, yield: 95.3%).

MS m/z (ESI): 672.2 [M+H]⁺.

### Example 2.2: Preparation of 4-(5-(3-((2-((2-Carboxyethyl)(methyl)carbamoyl)-6-methoxybenzo[b]thiophen-5-yl)oxy)propoxy)-6-hydroxybenzo[b]thiophen-2-yl)-4-oxobutanoic acid (C-3)

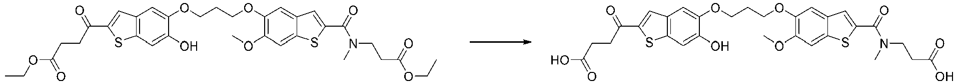

Ethyl 4-(5-(3-((2-((3-ethoxy-3-oxopropyl)(methyl)carbamoyl)-6-methoxybenzo[*b*]thiophen-5-yl)oxy)propoxy)-6-hydroxybenzo[*b*]thiophen-2-yl)-4-oxobutanoate (2.0 g, 2.98 mmol) was completely dissolved in a mixed solvent of tetrahydrofuran (40 mL) and water (10 mL). In addition, lithium hydroxide monohydrate (0.29 g, 11.9 mmol) was completely dissolved in water (2 mL). The aqueous lithium hydroxide solution was slowly added dropwise to the reaction system at 0 °C, and the mixture was slowly warmed to room temperature and stirred for 12 h. Water (50 mL) was added to the reaction solution, and the mixed system was concentrated under reduced pressure at 40 °C to remove the organic solvent. The pH of the residue was adjusted to 4 with dilute hydrochloric acid (1 N, 100 mL), and filtration was performed under vacuum. The filter cake was washed with water (100 mL) and dried *in vacuo* at 50 °C to give the title compound (1.5 g, yield: 81.3%).

MS m/z (ESI): 616.1 [M+H]⁺.

¹H NMR (400 MHz, DMSO-*d*₆): δ 12.21 (br, 2H), 9.72 (s, 1H), 8.07 (s, 1H), 7.56 (s, 1H), 7.49 (s, 1H), 7.44 (s, 1H), 7.40 (s, 1H), 7.27 (s, 1H), 4.21-4.14 (m, 4H), 3.77 (s, 3H), 3.65-3.58 (m, 2H), 3.20-3.15 (m, 5H), 2.56-2.48 (m, 4H), 2.25-2.21 (m, 2H).

### Example 2.3: Preparation of tert-Butyl 4-(5-(3-((2-((3-(tert-butoxy)-3-oxopropyl)(methyl)carbamoyl)-6-methoxybenzo[b]thiophen-5-yl)oxy)propoxy)-6-hydroxybenzo[b]thiophen-2-yl)-4-oxobutanoate (C-1)

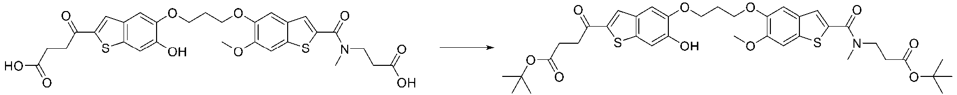

4-(5-(3-((2-((2-Carboxyethyl)(methyl)carbamoyl)-6-methoxybenzo[*b*]thiophen-5-yl)oxy)propoxy)-6-hydroxybenzo[*b*]thiophen-2-yl)-4-oxobutanoic acid (0.5 g, 0.81 mmol) and *O*-tert-butyl-*N,N'-*diisopropylisourea (0.65 g, 3.24 mmol) were dissolved in tetrahydrofuran (5 mL), and the solution was heated to 50 °C and stirred for 12 h. The reaction system was concentrated to remove the solvent, and the resulting residue was purified by silica gel column chromatography (eluent: ethyl acetate:petroleum ether = 1:1, volume ratio) to give the title compound (267 mg, yield: 45.3%).

MS m/z (ESI): 728.2 [M+H]⁺.

¹H NMR (400 MHz, DMSO-*d*₆): δ 9.77 (s, 1H), 8.13 (s, 1H), 7.62 (s, 1H), 7.56 (s, 1H), 7.50 (s, 1H), 7.47 (s, 1H), 7.33 (s, 1H), 4.27-4.22 (m, 4H), 3.84 (s, 3H), 3.70-3.62 (m, 2H), 3.34 (s, 3H), 3.23-3.20 (m, 2H), 2.58-2.50 (m, 4H), 2.30-2.27 (m, 2H), 1.39-1.37 (m, 18H).

### Example 2.4: Preparation of Ethyl 4-(5-(2-((2-((3-ethoxy-3-oxopropyl)(methyl)carbamoyl)-6-methoxybenzo[b]thiophen-5-yl)oxy)ethoxy)-6-hydroxybenzo[b]thiophen-2-yl)-4-oxobutanoate (C-4)

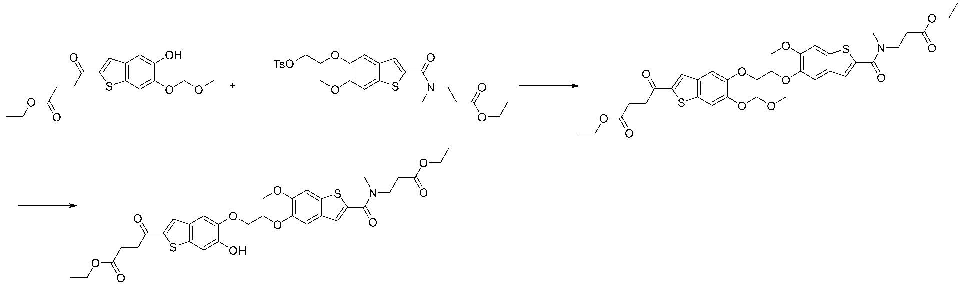

The title compound (20.0 mg, yield: 84.5%) was obtained by using the synthesis scheme in Example 2.1 and replacing the starting material of the reaction of step 1 ethyl 3-(6-methoxy-*N*-methyl-5-(3-(tosyloxy)propoxy)benzo[*b*]thiophene-2-carboxamido)propanoate with ethyl 3-(6-methoxy-*N*-methyl-5-(2-(tosyloxy)ethoxy)benzo[*b*]thiophene-2-carboxamido)propanoate.

MS m/z (ESI): 658.2 [M+H]⁺.

### Example 2.5: Preparation of 4-(5-(2-((2-((2-Carboxyethyl)(methyl)carbamoyl)-6-methoxybenzo[b]thiophen-5-yl)oxy)ethoxy)-6-hydroxybenzo[b]thiophen-2-yl)-4-oxobutanoic acid (C-5)

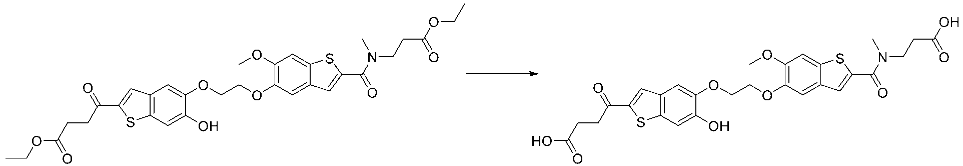

The title compound (10.0 mg, yield: 54.7%) was obtained by using the synthesis scheme in Example 2.2 and replacing the starting material of the reaction ethyl 4-(5-(3-((2-((3-ethoxy-3-oxopropyl)(methyl)carbamoyl)-6-methoxybenzo[*b*]thiophen-5-yl)oxy)propoxy)-6-hydroxybenzo[*b*]thiophen-2-yl)-4-oxobutanoate with ethyl 4-(5-(2-((2-((3-ethoxy-3-oxopropyl)(methyl)carbamoyl)-6-methoxybenzo[*b*]thiophen-5-yl)oxy)ethoxy)-6-hydroxybenzo[*b*]thiophen-2-yl)-4-oxobutanoate.

MS m/z (ESI): 602.1 [M+H]⁺.

¹H NMR (400 MHz, DMSO-*d*₆): δ 8.15 (s, 1H), 7.65 (s, 1H), 7.59-7.57 (m, 3H), 7.36 (s, 1H), 4.43 (s, 4H), 3.85 (s, 3H), 3.71 (s, 2H), 3.27-3.12 (m, 5H), 2.60-2.57 (m, 4H).

### Example 2.6: Preparation of Ethyl 4-(5-(4-((2-((3-ethoxy-3-oxopropyl)(methyl)carbamoyl)-6-methoxybenzo[b]thiophen-5-yl)oxy)butoxy)-6-hydroxybenzo[b]thiophen-2-yl)-4-oxobutanoate (C-6)

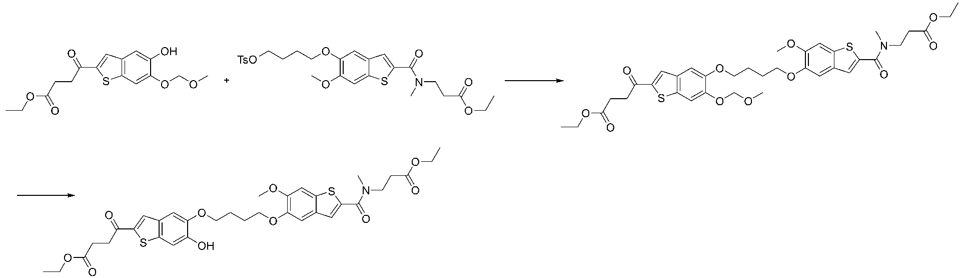

The title compound (32 mg, yield: 94.6%) was obtained by using the synthesis scheme in Example 2.1 and replacing the starting material of the reaction of step 1 ethyl 3-(6-methoxy-*N-*methyl-5-(3-(tosyloxy)propoxy)benzo[*b*]thiophene-2-carboxamido)propanoate with ethyl 3-(6-methoxy-*N*-methyl-5-(4-(tosyloxy)butoxy)benzo[*b*]thiophene-2-carboxamido)propanoate.

MS m/z (ESI): 686.2 [M+H]⁺.

### Example 2.7: Preparation of 4-(5-(4-((2-((2-Carboxyethyl)(methyl)carbamoyl)-6-methoxybenzo[b]thiophen-5-yl)oxy)butoxy)-6-hydroxybenzo[b]thiophen-2-yl)-4-oxobutanoic acid (C-7)

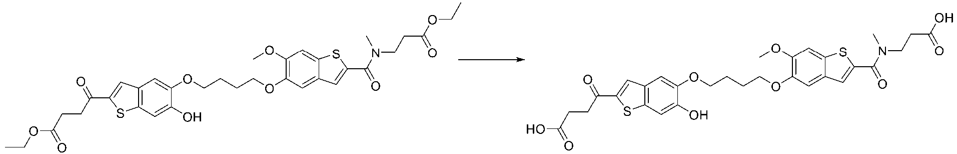

The title compound (16 mg, yield: 52.8%) was obtained by using the synthesis scheme in Example 2.2 and replacing the starting material of the reaction ethyl 4-(5-(3-((2-((3-ethoxy-3-oxopropyl)(methyl)carbamoyl)-6-methoxybenzo[b]thiophen-5-yl)oxy)propoxy)-6-hydroxybenzo[*b*]thiophen-2-yl)-4-oxobutanoate with ethyl 4-(5-(4-((2-((3-ethoxy-3-oxopropyl)(methyl)carbamoyl)-6-methoxybenzo[b]thiophen-5-yl)oxy)butoxy)-6-hydroxybenzo[*b*]thiophen-2-yl)-4-oxobutanoate.

MS m/z (ESI): 630.1 [M+H]⁺.

¹H NMR (400 MHz, DMSO-*d*₆): δ 12.25 (s, 2H), 9.85 (s, 1H), 8.13 (s, 1H), 7.64 (s, 1H), 7.56 (s, 1H), 7.47 (s, 1H), 7.45 (s, 1H), 7.33 (s, 1H), 4.20-4.35 (m, 4H), 3.84 (s, 3H), 3.71 (s, 2H), 3.09-3.28 (m, 5H), 2.56-2.63 (m, 4H), 1.98 (s, 4H).

### Example 2.8: Preparation of Ethyl 4-(5-(3-((2-((3-ethoxy-3-oxopropyl)(methyl)carbamoyl)-5-methoxybenzo[b]thiophen-6-yl)oxy)propoxy)-6-hydroxybenzo[b]thiophen-2-yl)-4-oxobutanoate (C-8)

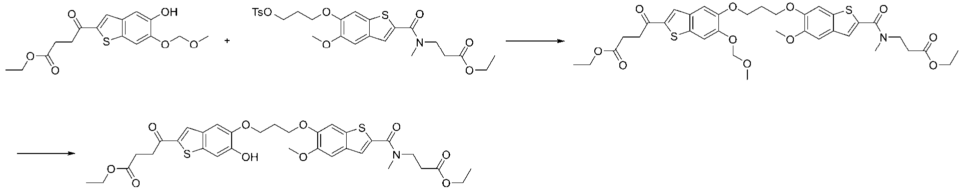

The title compound (40 mg, yield: 96.8%) was obtained by using the synthesis scheme in Example 2.1 and replacing the starting material of the reaction of step 1 ethyl 3-(6-methoxy-*N*-methyl-5-(3-(tosyloxy)propoxy)benzo[*b*]thiophene-2-carboxamido)propanoate with ethyl 3-(5-methoxy-*N*-methyl-6-(3-(tosyloxy)propoxy)benzo[*b*]thiophene-2-carboxamido)propanoate.

MS m/z (ESI): 672.2 [M+H]⁺.

### Example 2.9: Preparation of 4-(5-(3-((2-((2-Carboxyethyl)(methyl)carbamoyl)-5-methoxybenzo[b]thiophen-6-yl)oxy)propoxy)-6-hydroxybenzo[b]thiophen-2-yl)-4-oxobutanoic acid (C-9)

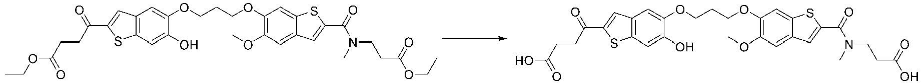

The title compound (15 mg, yield: 38.8%) was obtained by using the synthesis scheme in Example 2.2 and replacing the starting material of the reaction ethyl 4-(5-(3-((2-((3-ethoxy-3-oxopropyl)(methyl)carbamoyl)-6-methoxybenzo[*b*]thiophen-5-yl)oxy)propoxy)-6-hydroxybenzo[*b*]thiophen-2-yl)-4-oxobutanoate with ethyl 4-(5-(3-((2-((3-ethoxy-3-oxopropyl)(methyl)carbamoyl)-5-methoxybenzo[*b*]thiophen-6-yl)oxy)propoxy)-6-hydroxybenzo[*b*]thiophen-2-yl)-4-oxobutanoate.

MS m/z (ESI): 616.1 [M+H]⁺.

¹H NMR (400 MHz, DMSO-*d*₆): δ 9.85 (s, 1H), 8.12 (s, 1H), 7.65 (s, 1H), 7.61 (s, 1H), 7.50 (s, 1H), 7.43 (s, 1H), 7.34 (s, 1H), 4.21-4.31 (m, 4H), 3.83 (s, 3H), 3.70 (s, 2H), 3.21-3.24 (m, 5H), 2.56-2.60 (m, 4H), 2.27-2.33 (m, 2H).

### Example 2.10: Preparation of Ethyl 4-(6-(3-((2-((3-ethoxy-3-oxopropyl)(methyl)carbamoyl)-6-methoxybenzo[b]thiophen-5-yl)oxy)propoxy)-5-hydroxybenzo[b]thiophen-2-yl)-4-oxobutanoate (C-10)

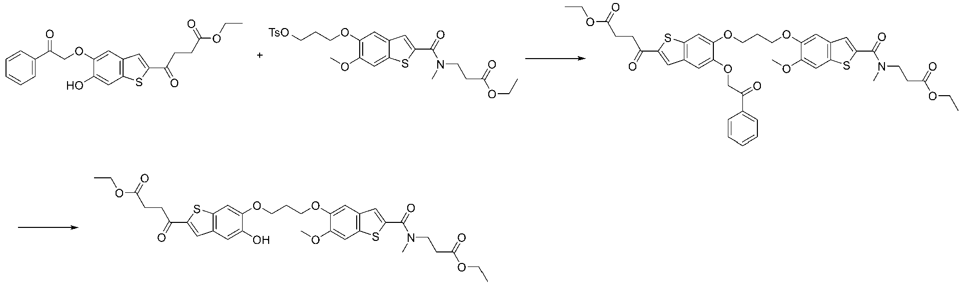

### Step 1: Preparation of ethyl 4-(6-(3-((2-((3-ethoxy-3-oxopropyl)(methyl)carbamoyl)-6-methoxybenzo[b]thiophen-5-yl)oxy)propoxy)-5-(2-oxo-2-phenylethoxy)benzo[b]thiophen-2-yl)-4-oxobutanoate

Ethyl 4-(6-hydroxy-5-(2-oxo-2-phenylethoxy)benzo[*b*]thiophen-2-yl)-4-oxobutanoate (97.0 mg, 235.17 µmol), ethyl 3-(6-methoxy-*N*-methyl-5-(3-(tosyloxy)propoxy)benzo[*b*]thiophene-2-carboxamido)propanoate (129.2 mg, 235.06 µmol), potassium carbonate (65.0 mg, 471.01 µmol), and potassium iodide (39.4 mg, 237.35 µmol) were sequentially added to *N*,*N*-dimethylformamide (1 mL), and the mixture was heated to 50 °C and stirred for 12 h. After the reaction system was cooled to room temperature, the reaction solution was poured into water (10 mL) and extracted three times with ethyl acetate (10 mL). The organic phases were combined, washed three times with saturated brine (10 mL), dried over anhydrous sodium sulfate, and concentrated. The resulting residue was purified by silica gel column chromatography (eluent: ethyl acetate:petroleum ether = 3:1, volume ratio) to give the title compound (36 mg, yield: 19.4%).

MS m/z (ESI): 790.2 [M+H]⁺.

### Step 2: Preparation of ethyl 4-(6-(3-((2-((3-ethoxy-3-oxopropyl)(methyl)carbamoyl)-6-methoxybenzo[b]thiophen-5-yl)oxy)propoxy)-5-hydroxybenzo[b]thiophen-2-yl)-4-oxobutanoate

Ethyl 4-(6-(3-((2-((3-ethoxy-3-oxopropyl)(methyl)carbamoyl)-6-methoxybenzo[*b*]thiophen-5-yl)oxy)propoxy)-5-(2-oxo-2-phenylethoxy)benzo[b]thiophen-2-yl)-4-oxobutanoate (36 mg, 45.57 µmol) was completely dissolved in tetrahydrofuran (3 mL). After acetic acid (1 mL) was added at room temperature, zinc powder (59 mg, 907.69 µmol) was added to the reaction solution, and the reaction system was stirred at 60 °C for 6 h. After the reaction solution was filtered, the filtrate was collected and concentrated to remove the solvent to give the title compound (21.0 mg, yield: 68.6%).

MS m/z (ESI): 672.2 [M+H]⁺.

### Example 2.11: Preparation of 4-(6-(3-((2-((2-Carboxyethyl)(methyl)carbamoyl)-6-methoxybenzo[b]thiophen-5-yl)oxy)propoxy)-5-hydroxybenzo[b]thiophen-2-yl)-4-oxobutanoic acid (C-11)

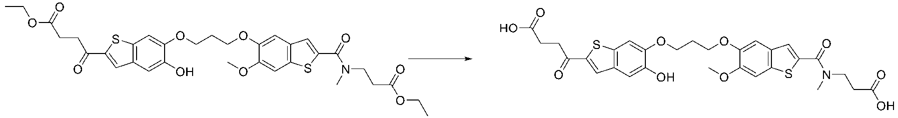

Ethyl 4-(6-(3-((2-((3-ethoxy-3-oxopropyl)(methyl)carbamoyl)-6-methoxybenzo[*b*]thiophen-5-yl)oxy)propoxy)-5-hydroxybenzo[*b*]thiophen-2-yl)-4-oxobutanoate (21.0 mg, 31.26 µmol) was completely dissolved in a mixed solvent of tetrahydrofuran (2 mL) and water (0.3 mL). In addition, lithium hydroxide monohydrate (5.3 gm, 126.31 µmol) was completely dissolved in water (0.2 mL). The aqueous lithium hydroxide solution was slowly added dropwise to the reaction system at 0 °C, and the mixture was slowly warmed to room temperature and stirred for 12 h. Water (10 mL) was added to the reaction solution, and the mixed system was concentrated under reduced pressure at 40 °C to remove the organic solvent. The pH of the residue was adjusted to 4 with dilute hydrochloric acid (1 N, 10 mL), and filtration was performed under vacuum. The filter cake was washed with water (10 mL) and dried *in vacuo* at 50 °C to give the title compound (10.0 mg, yield: 52.0%).

MS m/z (ESI): 616.1 [M+H]⁺.

¹H NMR (400 MHz, DMSO-*d*₆): δ 12.22 (s, 2H), 9.32 (s, 1H), 8.16 (s, 1H), 7.63 (s, 1H), 7.59 (s, 1H), 7.56 (s, 1H), 7.47 (s, 1H), 7.34 (s, 1H), 4.25 (t, *J =* 5.2 Hz, 4H), 3.84 (s, 3H), 3.70 (s, 2H), 3.16-3.27 (m, 5H), 2.56-2.62 (m, 4H), 2.23-2.33 (m, 2H).

### Example 2.12: Preparation of Ethyl 4-(6-(3-((2-((3-ethoxy-3-oxopropyl)(methyl)carbamoyl)-5-methoxybenzo[b]thiophen-6-yl)oxy)propoxy)-5-hydroxybenzo[b]thiophen-2-yl)-4-oxobutanoate (C-12)

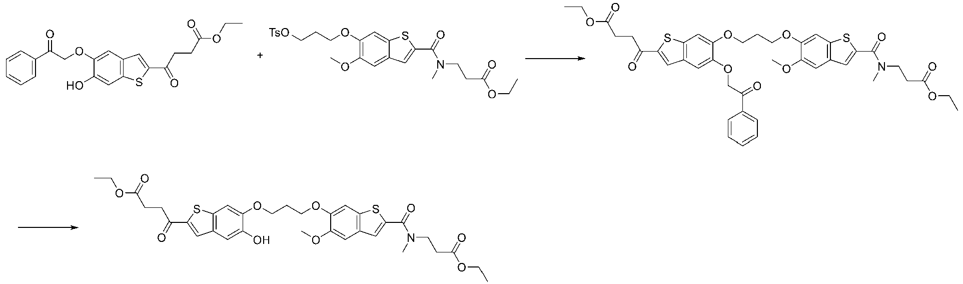

The title compound (18 mg, yield: 47.5%) was obtained by using the synthesis scheme in Example 2.10 and replacing the starting material of the reaction of step 1 ethyl 3-(6-methoxy-*N*-methyl-5-(3-(tosyloxy)propoxy)benzo[*b*]thiophene-2-carboxamido)propanoate with ethyl 3-(5-methoxy-*N*-methyl-6-(3-(tosyloxy)propoxy)benzo[*b*]thiophene-2-carboxamido)propanoate.

MS m/z (ESI): 672.2 [M+H]⁺.

### Example 2.13: Preparation of 4-(6-(3-((2-((2-Carboxyethyl)(methyl)carbamoyl)-5-methoxybenzo[b]thiophen-6-yl)oxy)propoxy)-5-hydroxybenzo[b]thiophen-2-yl)-4-oxobutanoic acid (C-13)

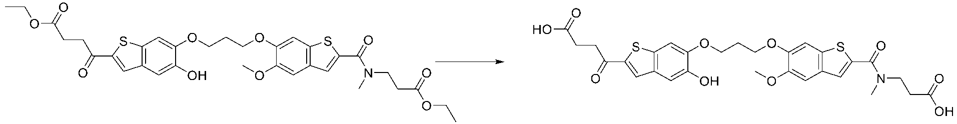

The title compound (16 mg, yield: 52.9%) was obtained by using the synthesis scheme in Example 2.11 and replacing the starting material of the reaction ethyl 4-(6-(3-((2-((3-ethoxy-3-oxopropyl)(methyl)carbamoyl)-6-methoxybenzo[*b*]thiophen-5-yl)oxy)propoxy)-5-hydroxybenzo[*b*]thiophen-2-yl)-4-oxobutanoate with ethyl 4-(6-(3-((2-((3-ethoxy-3-oxopropyl)(methyl)carbamoyl)-5-methoxybenzo[b]thiophen-6-yl)oxy)propoxy)-5-hydroxybenzo[*b*]thiophen-2-yl)-4-oxobutanoate.

MS m/z (ESI): 616.1 [M+H]⁺.

¹H NMR (400 MHz, DMSO-*d*₆): δ 12.20 (s, 2H), 9.31 (s, 1H), 8.16 (s, 1H), 7.65 (s, 1H), 7.61 (s, 1H), 7.59 (s, 1H), 7.42 (s, 1H), 7.34 (s, 1H), 4.22-4.31 (m, 4H), 3.82 (s, 3H), 3.70 (s, 2H), 3.16-3.28 (m, 5H), 2.56-2.61 (m, 4H), 2.29 (dd, *J =* 16.0, 10.4 Hz, 2H).

### Example 2.14: Preparation of Ethyl 4-(5-(3-((2-((3-ethoxy-3-oxopropyl)(methyl)carbamoyl)-6-hydroxybenzo[b]thiophen-5-yl)oxy)propoxy)-6-methoxybenzo[b]thiophen-2-yl)-4-oxobutanoate (C-14)

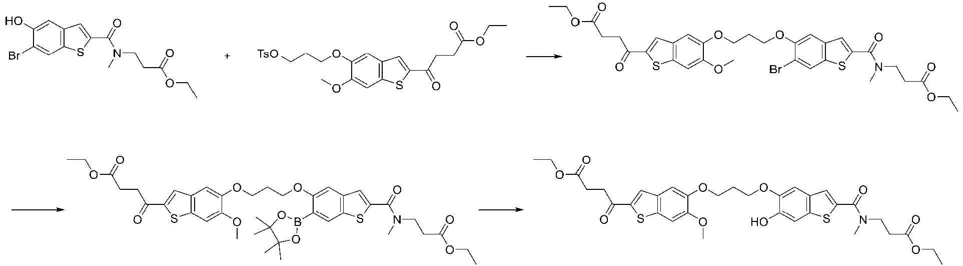

### Step 1: Preparation of ethyl 4-(5-(3-((6-bromo-2-((3-ethoxy-3-oxopropyl)(methyl)carbamoyl)benzo[b]thiophen-5-yl)oxy)propoxy)-6-methoxybenzo[b]thiophen-2-yl)-4-oxobutanoate

Ethyl 3-(6-bromo-5-hydroxy-*N*-methylbenzo[*b*]thiophene-2-carboxamido)propanoate (750.0 mg, 1.94 mmol), ethyl 4-(6-methoxy-5-(3-(tosyloxy)propoxy)benzo[*b*]thiophen-2-yl)-4-oxobutanoate (1.0 g, 1.92 mmol), and potassium carbonate (536.0 mg, 3.88 mmol) were sequentially added to *N,N-*dimethylformamide (10 mL), and the mixture was heated to 50 °C and stirred for 12 h. After the reaction system was cooled to room temperature, the reaction solution was poured into water (50 mL) and extracted three times with ethyl acetate (30 mL). The organic phases were combined, washed three times with saturated brine (10 mL), dried over anhydrous sodium sulfate, and concentrated. The resulting residue was purified by silica gel column chromatography (eluent: ethyl acetate:petroleum ether = 2:1, volume ratio) to give the title compound (1.1 g, yield: 77.1%).

MS m/z (ESI): 734.1 [M+H]⁺.

### Step 2: Preparation of ethyl 4-(5-(3-((2-((3-ethoxy-3-oxopropyl)(methyl)carbamoyl)-6-(4,4,5,5-tetramethyl-1,3,2-dioxaborolan-2-yl)benzo[b]thiophen-5-yl)oxy)propoxy)-6-methoxybenzo[b]thiophen-2-yl)-4-oxobutanoate

Ethyl 4-(5-(3-((6-bromo-2-((3-ethoxy-3-oxopropyl)(methyl)carbamoyl)benzo[*b*]thiophen-5-yl)oxy)propoxy)-6-methoxybenzo[*b*]thiophen-2-yl)-4-oxobutanoate (590 mg, 803.08 µmol), bis(pinacolato)diboron (306 mg, 1.20 mmol), potassium acetate (160 mg, 1.63 mmol), and [1,1'-bis(diphenylphosphino)ferrocene]palladium(II) dichloride (120 mg, 164 µmol) were sequentially added to 1,4-dioxane (6 mL). After nitrogen was bubbled through the reaction system for 5 min, the reaction system was heated to 100 °C and stirred for 4 h. The reaction system was cooled to room temperature and then concentrated to remove the solvent. The resulting residue was dissolved in ethyl acetate (20 mL), and the solution was washed three times with saturated brine (10 mL). The organic phase was dried over anhydrous sodium sulfate and concentrated. The resulting residue was purified by silica gel column chromatography (eluent: ethyl acetate:petroleum ether = 1:1, volume ratio) to give the title compound (60 mg, yield: 9.6%).

MS m/z (ESI): 782.3 [M+H]⁺.

### Step 3: Preparation of ethyl 4-(5-(3-((2-((3-ethoxy-3-oxopropyl)(methyl)carbamoyl)-6-hydroxybenzo[b]thiophen-5-yl)oxy)propoxy)-6-methoxybenzo[b]thiophen-2-yl)-4-oxobutanoate

Ethyl 4-(5-(3-((2-((3-ethoxy-3-oxopropyl)(methyl)carbamoyl)-6-(4,4,5,5-tetramethyl-1,3,2-dioxaborolan-2-yl)benzo[*b*]thiophen-5-yl)oxy)propoxy)-6-methoxybenzo[*b*]thiophen-2-yl)-4-oxobutanoate (35 mg, 44.77 µmol) was completely dissolved in a mixed solution of acetone (2 mL) and water (0.5 mL). After potassium peroxymonosulfate (40 mg, 115.51 µmol) was slowly added in batches at room temperature, the reaction system was stirred at room temperature for 4 h. The reaction system was concentrated to remove acetone as much as possible. The resulting residue was dissolved in ethyl acetate (5 mL), and the solution was washed three times with saturated brine (6 mL). The resulting residue was purified by silica gel column chromatography (eluent: ethyl acetate:petroleum ether = 2:1, volume ratio) to give the title compound (12 mg, yield: 40.0%).

MS m/z (ESI): 672.2 [M+H]⁺.

### Example 2.15: Preparation of 4-(5-(3-((2-((2-Carboxyethyl)(methyl)carbamoyl)-6-hydroxybenzo[b]thiophen-5-yl)oxy)propoxy)-6-methoxybenzo[b]thiophen-2-yl)-4-oxobutanoic acid (C-15)

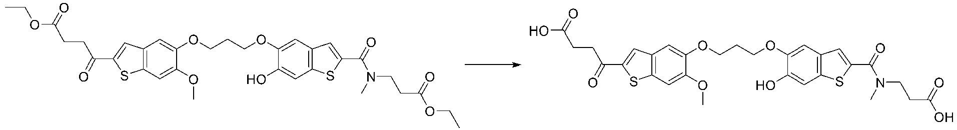

Ethyl 4-(5-(3-((2-((3-ethoxy-3-oxopropyl)(methyl)carbamoyl)-6-hydroxybenzo[*b*]thiophen-5-yl)oxy)propoxy)-6-methoxybenzo[*b*]thiophen-2-yl)-4-oxobutanoate (12.0 mg, 17.86 µmol) was completely dissolved in a mixed solvent of tetrahydrofuran (2 mL) and water (0.3 mL). In addition, lithium hydroxide monohydrate (3.3 mg, 78.64 µmol) was completely dissolved in water (0.2 mL). The aqueous lithium hydroxide solution was slowly added dropwise to the reaction system at 0 °C, and the mixture was slowly warmed to room temperature and stirred for 12 h. Water (5 mL) was added to the reaction solution, and the mixed system was concentrated under reduced pressure at 40 °C to remove the organic solvent. The pH of the residue was adjusted to 4 with dilute hydrochloric acid (1 N, 10 mL), and filtration was performed under vacuum. The filter cake was washed with water (10 mL) and dried *in vacuo* at 50 °C to give the title compound (2.0 mg, yield: 18.2%).

MS m/z (ESI): 616.1 [M+H]⁺.

### III. Preparation of Drug-Linker Compounds

### Example 3.1: Preparation of 4-(5-(3-((2-((2-Carboxyethyl)(methyl)carbamoyl)-6-methoxybenzo[b]thiophen-5-yl)oxy)propoxy)-6-(((2-((((4-((2S,5S)-5-isopropyl-38-(4-((6-(2-(methylsulfonyl)pyrimidin-5-yl)hex-5-ynamido)methyl)-1H-1,2,3-triazol-1-yl)-4,7,11-trioxo-2-(3-ureidopropyl)-9,15,18,21,24,27,30,33,36-nonaoxa-3,6,12-triazaoctatriacontanamido)benzyl)oxy)carbonyl)(methyl)amino)ethyl)(methyl)carbamoyl)oxy)benzo[b]t hiophen-2-yl)-4-oxobutanoic acid (DL-1)

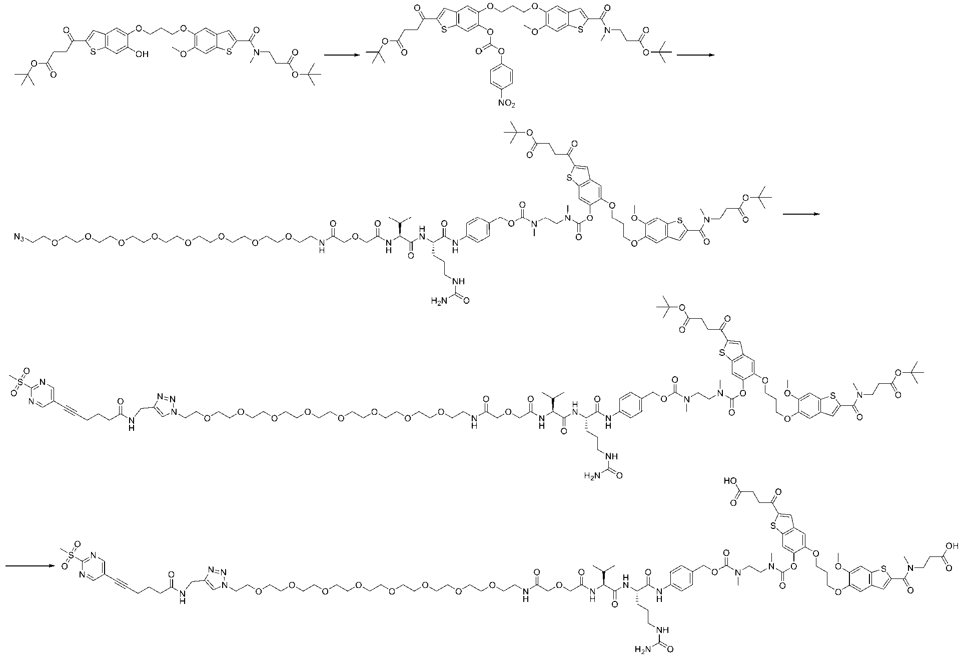

### Step 1: Preparation of tert-butyl 4-(5-(3-((2-((3-(tert-butoxy)-3-oxopropyl)(methyl)carbamoyl)-6-methoxybenzo[b]thiophen-5-yl)oxy)propoxy)-6-(((4-nitrophenoxy)carbonyl)oxy)benzo[b]thiophen-2-yl)-4-oxobutanoate

tert-Butyl 4-(5-(3-((2-((3-(tert-butoxy)-3-oxopropyl)(methyl)carbamoyl)-6-methoxybenzo[b]thiophen-5-yl)oxy)propoxy)-6-hydroxybenzo[b]thiophen-2-yl)-4-oxobutanoate (86 mg, 0.12 mmol) and *N,N-*diisopropylethylamine (31 mg, 0.24 mmol) were dissolved in *N*,*N*-dimethylformamide (1 mL), and bis(p-nitrophenyl) carbonate (73 mg, 0.24 mmol) was slowly added to the reaction solution at 0 °C. The mixture was slowly warmed to room temperature and stirred for 3 h. The reaction solution was poured into water (20 mL) and extracted three times with ethyl acetate (30 mL). The organic phases were combined, washed three times with saturated brine (15 mL), dried over anhydrous sodium sulfate, and concentrated. The resulting residue was purified by silica gel column chromatography (eluent: ethyl acetate:petroleum ether = 1:1, volume ratio) to give the title compound (81 mg, yield: 75.8%).

MS m/z (ESI): 893.3 [M+H]⁺.

### Step 2: Preparation of tert-butyl 4-(6-(((2-((((4-((2S,5S)-38-azido-5-isopropyl-4,7,11-trioxo-2-(3-ureidopropyl)-9,15,18,21,24,27,30,33,36-nonaoxa-3,6,12-triazaoctatriacontanamido)benzyl)oxy)carbonyl)(methyl)amino)ethyl)(methyl)carbamoyl)oxy)-5-(3-((2-((3-(tert-butoxy)-3-oxopropyl)(methyl)carbamoyl)-6-methoxybenzo[b]thiophen-5-yl)oxy)propoxy)benzo[b]thiophen-2-yl)-4-oxobutanoate

tert-Butyl 4-(5-(3-((2-((3-(tert-butoxy)-3-oxopropyl)(methyl)carbamoyl)-6-methoxybenzo[b]thiophen-5-yl)oxy)propoxy)-6-(((4-nitrophenoxy)carbonyl)oxy)benzo[*b*]thiophen-2-yl)-4-oxobutanoate (80 mg, 0.09 mmol) and *N*,*N*-diisopropylethylamine (35 mg, 0.27 mmol) were dissolved in *N,N-*dimethylformamide (1 mL), and benzyl 4-((2S,5S)-38-azido-5-isopropyl-4,7,11-trioxo-2-(3-ureidopropyl)-9,15,18,21,24,27,30,33,36-nonaoxa-3,6,12-triazaoctatriacontanamido)methyl(2-(methylamino)ethyl)carbamate (93 mg, 0.09 mmol) was slowly added to the reaction solution at 0 °C. The reaction solution was stirred at 20 °C for 2 h. The reaction solution was purified by preparative high performance liquid chromatography to give the title compound (109 mg, yield: 67.7%).

MS m/z (ESI): 1783.8 [M+H]⁺.

### Step 3: Preparation of tert-butyl 4-(5-(3-((2-((3-(tert-butoxy)-3-oxopropyl)(methyl)carbamoyl)-6-methoxybenzo[b]thiophen-5-yl)oxy)propoxy)-6-(((2-(((4-((2S,5S)-5-isopropyl-38-(4-((6-(2-(methylsulfonyl)pyrimidin-5-yl))hex-5-ynamido)methyl)-1H-1,2,3-triazol-1-yl)-4,7,11-trioxo-2-(3-ureidopropyl)-9,15,18,21,24,27,30,33,36-nonaoxa-3,6,12-triazaoctatriacontanamido)benzyl)oxy)carbonyl)(methyl)amino)ethyl)(methyl)carbamoyl)oxy)benzo[b]t hiophen-2-yl)-4-oxobutanoate

tert-Butyl 4-(6-(((2-((((4-((2*S*,5*S*)-38-azido-5-isopropyl-4,7,11-trioxo-2-(3-ureidopropyl)-9,15,18,21,24,27,30,33,36-nonaoxa-3,6,12-triazaoctatriacontanamido)benzyl)oxy)carbonyl)(methyl)amino)ethyl)(methyl)carbamoyl)oxy)-5-(3-((2-((3-(tert-butoxy)-3-oxopropyl)(methyl)carbamoyl)-6-methoxybenzo[*b*]thiophen-5-yl)oxy)propoxy)benzo[*b*]thiophen-2-yl)-4-oxobutanoate (109 mg, 0.06 mmol), sodium ascorbate (24 mg, 0.12 mmol), and anhydrous copper sulfate (29 mg, 0.18 mmol) were added to a mixed solvent of tetrahydrofuran (2 mL) and water (0.5 mL), and 6-(2-(methylsulfonyl)pyrimidin-5-yl)-*N*-(prop-2-yn-1-yl)hex-5-ynamide (21 mg, 0.07 mmol) was added to the reaction solution at 0 °C. The reaction solution was stirred at 0 °C for 3 h. The reaction solution was concentrated to remove the solvent, and the resulting residue was purified by preparative high performance liquid chromatography to give the title compound (58 mg, yield: 46.2%).

MS m/z (ESI): 2089.9 [M+H]⁺.

### Step 4: Preparation of 4-(5-(3-((2-((2-carboxyethyl)(methyl)carbamoyl)-6-methoxybenzo[b]thiophen-5-yl)oxy)propoxy)-6-(((2-((((4-((2S,5S)-5-isopropyl-38-(4-((6-(2-(methylsulfonyl)pyrimidin-5-yl)hex-5-ynamido)methyl)-1H-1,2,3-triazol-1-yl)-4,7,11-trioxo-2-(3-ureidopropyl)-9,15,18,21,24,27,30,33,36-nonaoxa-3,6,12-triazaoctatriacontanamido)benzyl)oxy)carbonyl)(methyl)amino)ethyl)(methyl)carbamoyl)oxy)benzo[b]t hiophen-2-yl)-4-oxobutanoic acid

tert-Butyl 4-(5-(3-((2-((3-(tert-butoxy)-3-oxopropyl)(methyl)carbamoyl)-6-methoxybenzo[b]thiophen-5-yl)oxy)propoxy)-6-(((2-(((4-((2*S*,5*S*)-5-isopropyl-38-(4-((6-(2-(methylsulfonyl)pyrimidin-5-yl))hex-5-ynamido)methyl)-1*H*-1,2,3-triazol-1-yl)-4,7,11-trioxo-2-(3-ureidopropyl)-9,15,18,21,24,27,30,33,36-nonaoxa-3,6,12-triazaoctatriacontanamido)benzyl)oxy)carbonyl)(methyl)amino)ethyl)(methyl)carbamoyl)oxy)benzo[*b*]t hiophen-2-yl)-4-oxobutanoate (51 mg, 0.024 mmol) was dissolved in dichloromethane (1 mL), and trifluoroacetic acid (0.1 mL) was added to the reaction solution at 0 °C. The mixture was slowly warmed to room temperature and stirred for 1 h. The reaction solution was diluted with dichloromethane (20 mL) and concentrated to remove the solvent, and the resulting residue was purified by preparative high performance liquid chromatography to give the title compound (9.5 mg, yield: 19.7%).

MS m/z (ESI): 1976.7 [M+H]⁺.

### Example 3.2: Preparation of 4-(5-(3-((2-((2-Carboxyethyl)(methyl)carbamoyl)-6-methoxybenzo[b]thiophen-5-yl)oxy)propoxy)-6-((methyl(2-(methyl(((4-((S)-2-((S)-3-methyl-2-(6-(2-(methylsulfonyl)pyrimidin-5-yl)hex-5-ynamido)butanamido)propanamido)benzyl)oxy)carbonyl)amino)ethyl)carbamoyl)oxy)benzo[b]thiophe n-2-yl)-4-oxobutanoic acid (DL-2)

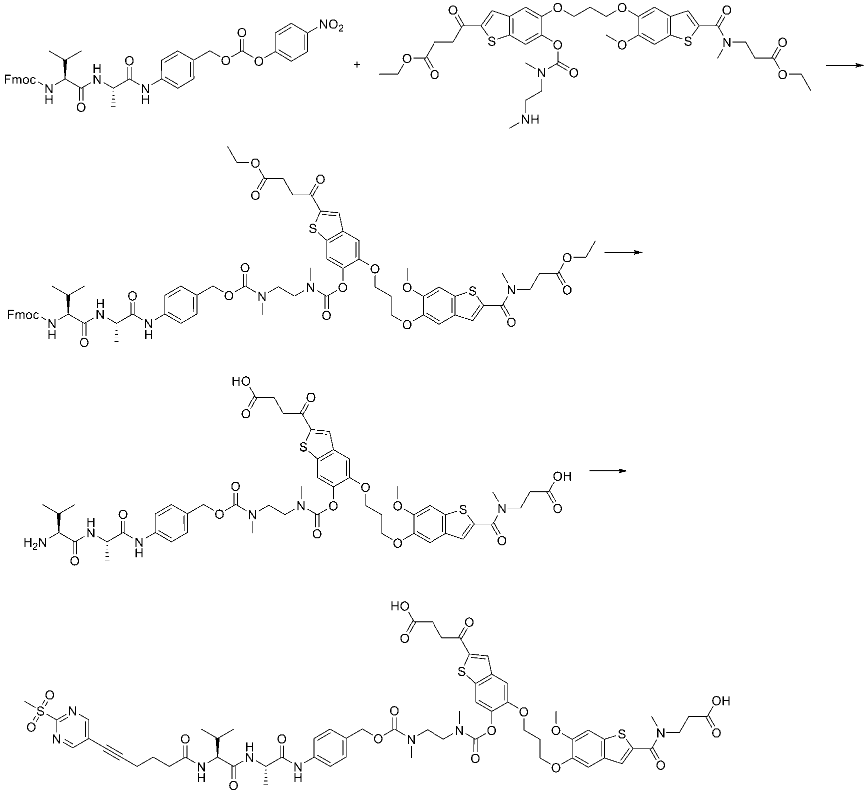

### Step 1: Preparation of ethyl 4-(6-(((2-((((4-((S)-2-((S)-2-((((9H-fluoren-9-yl)methoxy)carbonyl)amino)-3-methylbutanamido)propanamido)benzyl)oxy)carbonyl)(methyl)amino)ethyl)(methyl)carbamoyl)oxy)-5-(3-((2-((3-ethoxy-3-oxopropyl)(methyl)carbamoyl)-6-methoxybenzo[b]thiophen-5-yl)oxy)propoxy)benzo[b]thiophen-2-yl)-4-oxobutanoate

(9*H*-Fluoren-9-yl)methyl ((*S*)-3-methyl-1-(((*S*)-1-((4-((((4-nitrophenoxy)carbonyl)oxy)methyl)phenyl)amino)-1-oxopropan-2-yl)amino)-1-oxobutan-2-yl)carbamate (27.5 mg, 40.40 µmol) and *N*,*N*-diisopropylethylamine (11 mg, 85.11 µmol) were dissolved in *N*,*N*-dimethylformamide (1 mL), and ethyl 4-(5-(3-((2-((3-ethoxy-3-oxopropyl)(methyl)carbamoyl)-6-methoxybenzo[*b*]thiophen-5-yl)oxy)propoxy)-6-((methyl(2-(methylamino)ethyl)carbamoyl)oxy)benzo[*b*]thiophen-2-yl)-4-oxobutanoate (31.75 mg, 40.40 µmol) was slowly added to the reaction solution at 0 °C. The reaction solution was stirred at 20 °C for 2 h. The reaction solution was purified by preparative high performance liquid chromatography to give the title compound (12 mg, yield: 22.4%).

MS m/z (ESI): 1327.5 [M+H]⁺.

### Step 2: Preparation of 4-(6-(((2-((((4-((S)-2-((S)-2-amino-3-methylbutanamido)propanamido)benzyl)oxy)carbonyl)(methyl)amino)ethyl)(methyl)carbamoyl)oxy)-5-(3-((2-((2-carboxyethyl)(methyl)carbamoyl)-6-methoxybenzo[b]thiophen-5-yl)oxy)propoxy)benzo[b]thiophen-2-yl)-4-oxobutanoic acid

Ethyl 4-(6-(((2-((((4-((S)-2-((S)-2-((((9H-fluoren-9-yl)methoxy)carbonyl)amino)-3-methylbutanamido)propanamido)benzyl)oxy)carbonyl)(methyl)amino)ethyl)(methyl)carbamoyl)oxy)-5-(3-((2-((3-ethoxy-3-oxopropyl)(methyl)carbamoyl)-6-methoxybenzo[b]thiophen-5-yl)oxy)propoxy)benzo[*b*]thiophen-2-yl)-4-oxobutanoate (12 mg, 9.04 µmol) was dissolved in a mixed solvent of methanol (1 mL), tetrahydrofuran (1 mL), and water (0.05 mL), and lithium hydroxide monohydrate (3.0 mg, 71.42 µmol) was added to the reaction solution at room temperature. The mixture was stirred at room temperature for 4 h. The reaction system was concentrated to remove the solvent, and water (5 mL) was added to the residue. The pH was adjusted to 5 with dilute hydrochloric acid (1 N, 2 mL), and filtration was performed under vacuum. The filter cake was washed with water (5 mL) and dried to give the title compound (9 mg, yield: 94.9%).

MS m/z (ESI): 1049.4 [M+H]⁺.

### Step 3: Preparation of 4-(5-(3-((2-((2-carboxyethyl)(methyl)carbamoyl)-6-methoxybenzo[b]thiophen-5-yl)oxy)propoxy)-6-((methyl(2-(methyl(((4-((S)-2-((S)-3-methyl-2-(6-(2-(methylsulfonyl)pyrimidin-5-yl)hex-5-ynamido)butanamido)propanamido)benzyl)oxy)carbonyl)amino)ethyl)carbamoyl)oxy)benzo[b]thiophe n-2-yl)-4-oxobutanoic acid

4-(6-(((2-((((4-((*S*)-2-((*S*)-2-Amino-3-methylbutanamido)propanamido)benzyl)oxy)carbonyl)(methyl)amino)ethyl)(methyl)carbamoyl)oxy)-5-(3-((2-((2-carboxyethyl)(methyl)carbamoyl)-6-methoxybenzo[*b*]thiophen-5-yl)oxy)propoxy)benzo[*b*]thiophen-2-yl)-4-oxobutanoic acid (9 mg, 8.58 µmol) and *N,N-*diisopropylethylamine (5.8 mg, 44.88 µmol) were dissolved in *N*,*N*-dimethylformamide (1 mL), and 2,5-dioxopyrrolidin-1-yl 6-(2-methylsulfonylpyrimidin-5-yl)hex-5-ynoate (5.3 mg, 14.51 µmol) was added to the reaction solution at room temperature. The mixture was stirred at room temperature for 4 h. The reaction system was concentrated to remove the solvent, and the resulting residue was purified by preparative high performance liquid chromatography to give the title compound (3 mg, yield: 26.9%).

MS m/z (ESI): 1299.4 [M+H]⁺.

### Example 3.3: Preparation of 4-(5-(3-((2-((2-Carboxyethyl)(methyl)carbamoyl)-6-methoxybenzo[b]thiophen-5-yl)oxy)propoxy)-6-((methyl(2-(methyl(((4-((S)-2-((S)-3-methyl-2-(6-(2-(methylsulfonyl)pyrimidin-5-yl)hex-5-ynamido)butanamido)-5-ureidopentanamido)benzyl)oxy)carbonyl)amino)ethyl)carbamoyl)oxy)benzo[b]thiophen-2-yl)-4-oxobutanoic acid (DL-3)

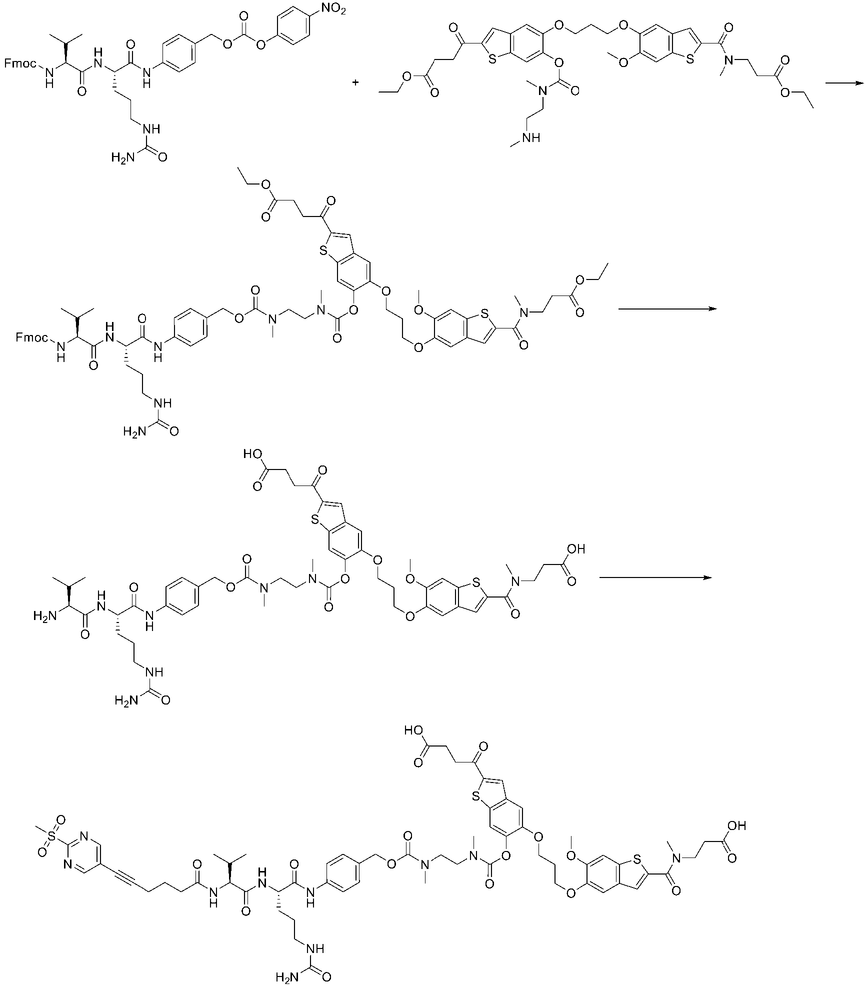

The title compound (2 mg, yield: 12.2%) was obtained by using the synthesis scheme in Example 3.2 and replacing the starting material of the reaction of step 1 (9*H*-fluoren-9-yl)methyl ((*S*)-3-methyl-1-(((*S*)-1-((4-((((4-nitrophenoxy)carbonyl)oxy)methyl)phenyl)amino)-1-oxopropan-2-yl)amino)-1-oxobutan-2-yl)carbamate with (9*H*-fluoren-9-yl)methyl ((*S*)-3-methyl-1-(((*S*)-1-((4-((((4-nitrophenoxy)carbonyl)oxy)methyl)phenyl)amino)-1-oxo-5-ureidopentan-2-yl)amino)-1-oxobutan-2-yl)carbamate.

MS m/z (ESI): 1385.5 [M+H]⁺.

### Example 3.4: Preparation of (S)-4-(6-((7-Benzyl-20-(2,5-dioxo-2,5-dihydro-1H-pyrrol-1-yl)-3,6,9,12,15-pentaoxo-2,5,8,11,14-pentaazaicosyl)oxy)-5-(3-((2-((2-carboxyethyl)(methyl)carbamoyl)-6-methoxybenzo[b]thiophen-5-yl)oxy)propoxy)benzo[b]thiophen-2-yl)-4-oxobutanoic acid (DL-4)

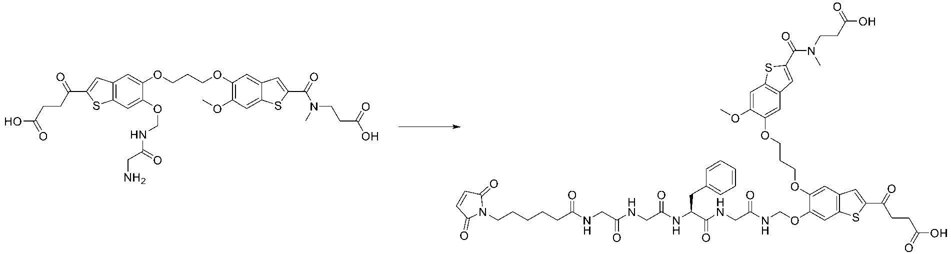

4-(6-((2-Aminoacetamido)methoxy)-5-(3-((2-((2-carboxyethyl)(methyl)carbamoyl)-6-methoxybenzo[b]thiophen-5-yl)oxy)propoxy)benzo[b]thiophen-2-yl)-4-oxobutanoic acid (100 mg, 0.14 mmol) and *N*,*N*-diisopropylethylamine (37 mg, 0.28 mmol) were dissolved in *N*,*N*-dimethylformamide (2 mL), and 2,5-dioxopyrrolidin-1-yl (6-(2,5-dioxo-2,5-dihydro-1*H*-pyrrol-1-yl)hexanoyl)glycylglycyl-L-phenylalaninate (187 mg, 0.34 mmol) was added to the reaction solution at room temperature. The mixture was stirred at room temperature for 4 h. The reaction system was concentrated to remove the solvent, and the resulting residue was purified by preparative high performance liquid chromatography to give the title compound (36 mg, yield: 11.3%).

MS m/z (ESI): 1156.4 [M+H]⁺.

### Example 3.5: Preparation of (S)-4-(6-((7-Benzyl-20-(2-(methylsulfonyl)pyrimidin-5-yl)-3,6,9,12,15-pentaoxo-2,5,8,11,14-pentaazaicos-19-yn-1-yl)oxy)-5-(3-((2-((2-carboxyethyl)(methyl)carbamoyl)-6-methoxybenzo[b]thiophen-5-yl)oxy)propoxy)benzo[b]thiophen-2-yl)-4-oxobutanoic acid (DL-5)

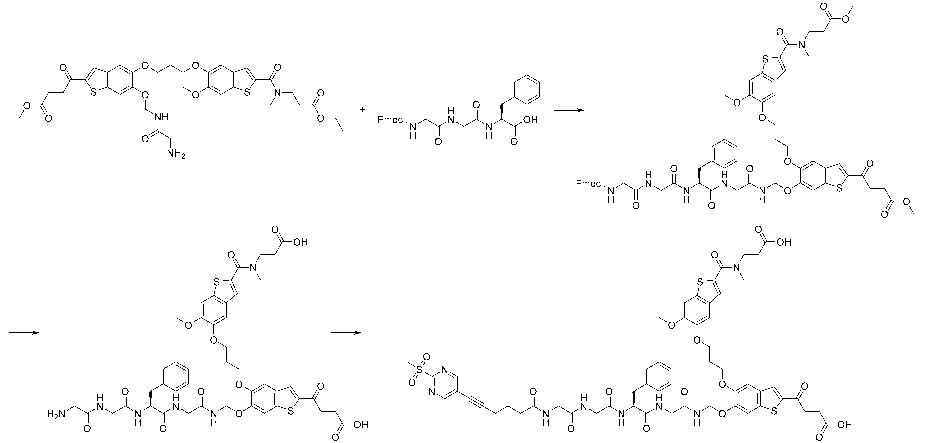

### Step 1: Preparation of ethyl (S)-4-(6-((11-benzyl-1-(9H-fluoren-9-yl)-3,6,9,12,15-pentaoxo-2-oxa-4,7,10,13,16-pentaazaheptadecan-17-yl)oxy)-5-(3-((2-((3-ethoxy-3-oxopropyl)(methyl)carbamoyl)-6-methoxybenzo[b]thiophen-5-yl)oxy)propoxy)benzo[b]thiophen-2-yl)-4-oxobutanoate

Ethyl 4-(6-((2-aminoacetamido)methoxy)-5-(3-((2-((3-ethoxy-3-oxopropyl)(methyl)carbamoyl)-6-methoxybenzo[b]thiophen-5-yl)oxy)propoxy)benzo[*b*]thiophen-2-yl)-4-oxobutanoate (50.0 mg, 65.97 µmol), (((9*H*-fluoren-9-yl)methoxy)carbonyl)glycylglycyl-L-phenylalanine (45.0 mg, 89.73 µmol), and *N,N,N',N*'-tetramethyl-O-(7-azabenzotriazol-1-yl)uronium hexafluorophosphate (53.0 mg, 139.39 µmol) were added to *N*,*N*-dimethylformamide (3 mL), and *N*,*N*-diisopropylethylamine (42.6 mg, 329.62 µmol) was added at room temperature. The reaction system was stirred at 25 °C for 2 h. The reaction solution was poured into water (15 mL) and extracted three times with ethyl acetate (10 mL). The organic phases were combined, washed three times with saturated brine (6 mL), dried over anhydrous sodium sulfate, and concentrated. The resulting residue was purified by silica gel column chromatography (eluent: 100% ethyl acetate) to give the title compound (50.0 mg, yield: 68.4%).

MS m/z (ESI): 1241.4 [M+H]⁺.

### Step 2: Preparation of (S)-4-(6-((13-amino-7-benzyl-3,6,9,12-tetraoxo-2,5,8,11-tetraazatridecyl)oxy)-5-(3-((2-((2-carboxyethyl)(methyl)carbamoyl)-6-methoxybenzo[b]thiophen-5-yl)oxy)propoxy)benzo[b]thiophen-2-yl)-4-oxobutanoic acid

Ethyl (*S*)-4-(6-((11-benzyl-1-(9*H*-fluoren-9-yl)-3,6,9,12,15-pentaoxo-2-oxa-4,7,10,13,16-pentaazaheptadecan-17-yl)oxy)-5-(3-((2-((3-ethoxy-3-oxopropyl)(methyl)carbamoyl)-6-methoxybenzo[b]thiophen-5-yl)oxy)propoxy)benzo[b]thiophen-2-yl)-4-oxobutanoate (50 mg, 40.28 µmol) was dissolved in a mixed solvent of methanol (1 mL), tetrahydrofuran (1 mL), and water (0.1 mL), and lithium hydroxide monohydrate (13.5 mg, 321.43 µmol) was added to the reaction solution at room temperature. The mixture was stirred at room temperature for 4 h. The reaction system was concentrated to remove the solvent, and the resulting residue was purified by preparative high performance liquid chromatography (30 mg, yield: 77.3%).

MS m/z (ESI): 963.3 [M+H]⁺.

### Step 3: Preparation of (S)-4-(6-((7-benzyl-20-(2-(methylsulfonyl)pyrimidin-5-yl)-3,6,9,12,15-pentaoxo-2,5,8,11,14-pentaazaicos-19-yn-1-yl)oxy)-5-(3-((2-((2-carboxyethyl)(methyl)carbamoyl)-6-methoxybenzo[b]thiophen-5-yl)oxy)propoxy)benzo[b]thiophen-2-yl)-4-oxobutanoic acid

(*S*)-4-(6-((13-Amino-7-benzyl-3,6,9,12-tetraoxo-2,5,8,11-tetraazatridecyl)oxy)-5-(3-((2-((2-carboxyethyl)(methyl)carbamoyl)-6-methoxybenzo[*b*]thiophen-5-yl)oxy)propoxy)benzo[b]thiophen-2-yl)-4-oxobutanoic acid (10 mg, 10.38 µmol) and *N,N-*diisopropylethylamine (2.7 mg, 20.89 µmol) were dissolved in *N,N-*dimethylformamide (1 mL), and 2,5-dioxopyrrolidin-1-yl 6-(2-methylsulfonylpyrimidin-5-yl)hex-5-ynoate (5.3 mg, 14.51 µmol) was added to the reaction solution at room temperature. The mixture was stirred at room temperature for 4 h. The reaction system was concentrated to remove the solvent, and the resulting residue was purified by preparative high performance liquid chromatography to give the title compound (4 mg, yield: 31.7%).

MS m/z (ESI): 1213.3 [M+H]⁺.

### Example 3.6: Preparation of 4-(5-(3-((2-((2-Carboxyethyl)(methyl)carbamoyl)-6-methoxybenzo[b]thiophen-5-yl)oxy)propoxy)-6-(((4S,7S,10S)-4,7,10-trimethyl-17-(2-(methylsulfonyl)pyrimidin-5-yl)-3,6,9,12-tetraoxo-2,5,8,11-tetraazaheptadec-16-yn-1-yl)oxy)benzo[b]thiophen-2-yl)-4-oxobutanoic acid (DL-6)

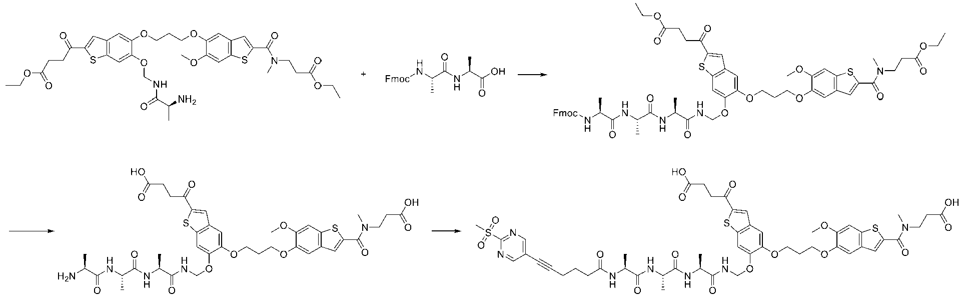

The title compound (8 mg, yield: 29.4%) was obtained by using the synthesis scheme in Example 3.5, replacing the starting material of the reaction of step 1 ethyl 4-(6-((2-aminoacetamido)methoxy)-5-(3-((2-((3-ethoxy-3-oxopropyl)(methyl)carbamoyl)-6-methoxybenzo[*b*]thiophen-5-yl)oxy)propoxy)benzo[b]thiophen-2-yl)-4-oxobutanoate with ethyl (*S*)-4-(6-((2-aminopropanamido)methoxy)-5-(3-((2-((3-ethoxy-3-oxopropyl)(methyl)carbamoyl)-6-methoxybenzo[*b*]thiophen-5-yl)oxy)propoxy)benzo[b]thiophen-2-yl)-4-oxobutanoate, and replacing (((9*H*-fluoren-9-yl)methoxy)carbonyl)glycylglycyl-*L*-phenylalanine with (((9*H*-fluoren-9-yl)methoxy)carbonyl)-*L*-alanyl-*L*-alanine.

MS m/z (ESI): 1108.3 [M+H]⁺.

### Example 3.7: Preparation of 4-(5-(3-((2-((2-Carboxyethyl)(methyl)carbamoyl)-5-methoxybenzo[b]thiophen-6-yl)oxy)propoxy)-6-(((4S,7S,10S)-4,7,10-trimethyl-17-(2-(methylsulfonyl)pyrimidin-5-yl)-3,6,9,12-tetraoxo-2,5,8,11-tetraazaheptadec-16-yn-1-yl)oxy)benzo[b]thiophen-2-yl)-4-oxobutanoic acid (DL-9)

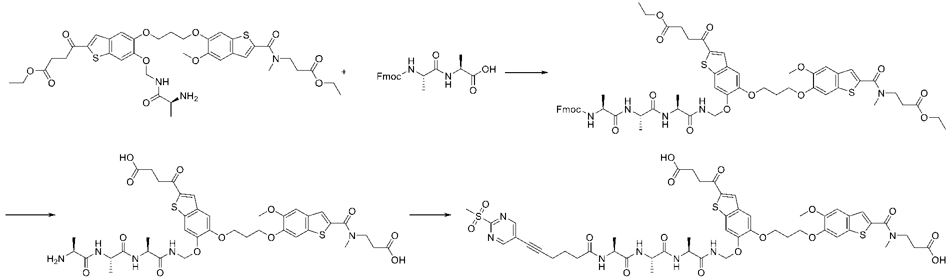

The title compound (5.8 mg, yield: 64.2%) was obtained by using the synthesis scheme in Example 3.5, replacing the starting material of the reaction of step 1 ethyl 4-(6-((2-aminoacetamido)methoxy)-5-(3-((2-((3-ethoxy-3-oxopropyl)(methyl)carbamoyl)-6-methoxybenzo[*b*]thiophen-5-yl)oxy)propoxy)benzo[*b*]thiophen-2-yl)-4-oxobutanoate with ethyl (*S*)-4-(6-((2-aminopropanamido)methoxy)-5-(3-((2-((3-ethoxy-3-oxopropyl)(methyl)carbamoyl)-5-methoxybenzo[b]thiophen-6-yl)oxy)propoxy)benzo[*b*]thiophen-2-yl)-4-oxobutanoate, and replacing (((9*H*-fluoren-9-yl)methoxy)carbonyl)glycylglycyl-*L*-phenylalanine with (((9H-fluoren-9-yl)methoxy)carbonyl)-*L*-alanyl-*L*-alanine.

MS m/z (ESI): 1108.3 [M+H]⁺.

### Example 3.8: Preparation of 4-(5-(3-((2-((2-Carboxyethyl)(methyl)carbamoyl)-5-methoxybenzo[b]thiophen-6-yl)oxy)propoxy)-6-(((4S,7R,10S)-4,7,10-trimethyl-17-(2-(methylsulfonyl)pyrimidin-5-yl)-3,6,9,12-tetraoxo-2,5,8,11-tetraazaheptadec-16-yn-1-yl)oxy)benzo[b]thiophen-2-yl)-4-oxobutanoic acid (DL-10)

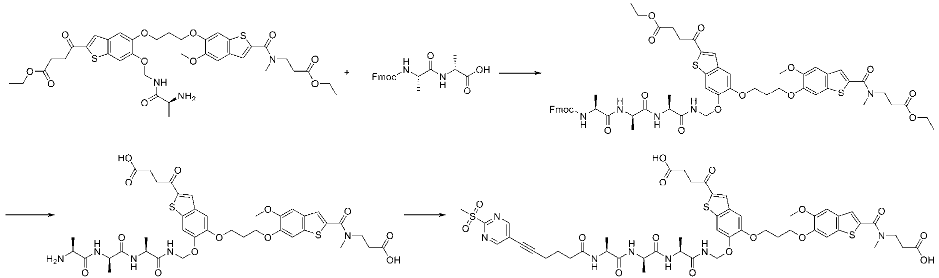

The title compound (14.0 mg, yield: 29.2%) was obtained by using the synthesis scheme in Example 3.5, replacing the starting material of the reaction of step 1 ethyl 4-(6-((2-aminoacetamido)methoxy)-5-(3-((2-((3-ethoxy-3-oxopropyl)(methyl)carbamoyl)-6-methoxybenzo[*b*]thiophen-5-yl)oxy)propoxy)benzo[*b*]thiophen-2-yl)-4-oxobutanoate with ethyl (*S*)-4-(6-((2-aminopropanamido)methoxy)-5-(3-((2-((3-ethoxy-3-oxopropyl)(methyl)carbamoyl)-5-methoxybenzo[*b*]thiophen-6-yl)oxy)propoxy)benzo[b]thiophen-2-yl)-4-oxobutanoate, and replacing (((9H-fluoren-9-yl)methoxy)carbonyl)glycylglycyl-*L*-phenylalanine with (((9*H*-fluoren-9-yl)methoxy)carbonyl)-*L*-alanyl-*D*-alanine.

MS m/z (ESI): 1108.3 [M+H]⁺.

### IV. Preparation of Immune-Stimulating Antibody Conjugates (ISACs)

### Preparation method A:

The pH of a trastuzumab stock solution was adjusted to 7.6-7.7 with a 1 M disodium hydrogen phosphate solution, and the stock solution was brought to volume with phosphate-buffered saline (PBS) (pH = 7.6-7.7) to obtain a 10 mg/mL trastuzumab buffer solution. 0.1 M EDTA and TCEP (6 eq, 10 mM) were added to the trastuzumab buffer solution. The mixture was shaken well and left to react at room temperature for 2 h. A solution of a drug-linker compound in DMSO (10 eq, 10 mM) was added to the reaction system at room temperature. The mixture was shaken well and left to react at room temperature for 2 h, then purified using a NAP-10 gel column, and washed with a histidine hydrochloride buffer solution (pH = 6.0-6.1). The filtrate was collected to give a trastuzumab-ISAC in the histidine hydrochloride buffer, and the product was then stored at 4 °C.

### Preparation method B:

The pH of an IgG1 monoclonal antibody stock solution was adjusted to 7.6-7.7 with a 1 M disodium hydrogen phosphate solution, and the stock solution was brought to volume with phosphate-buffered saline (PBS) (pH = 7.6-7.7) to obtain a 10 mg/mL IgG1 monoclonal antibody buffer solution. 0.1 M EDTA and TCEP (6 eq, 10 mM) were added to the IgG1 monoclonal antibody buffer solution. The mixture was shaken well and left to react at room temperature for 2 h. A solution of a drug-linker compound in DMSO (10 eq, 10 mM) was added to the reaction system at room temperature. The mixture was shaken well and left to react at room temperature for 2 h, then purified using a NAP-10 gel column, and washed with a histidine hydrochloride buffer solution (pH = 6.0-6.1). The filtrate was collected to give an IgG1-ISAC in the histidine hydrochloride buffer, and the product was then stored at 4 °C.

### V. Drug/Antibody Ratios of Immune-Stimulating Antibody Conjugates (ISACs): Determination of DAR Values

The molecular weight of the ISACs was determined by LC-MS, and their drug/antibody ratios (DAR values) were calculated.

### Chromatography conditions:

liquid chromatography column: Thermo MAbPac RP 3.0 × 100 mm;
mobile phase A: 0.1% FA/H₂O; mobile phase B: 0.1% FA/ACN;
flow rate: 0.25 mL/min; sample chamber temperature: 8 °C; column temperature: 60 °C; injection volume: 2 µL;

| Time (min) | 2 | 20 | 22 | 25 | 26 | 30 |
|---|---|---|---|---|---|---|
| Mobile phase A (volume%) | 75 | 60 | 5 | 5 | 75 | 75 |
| Mobile phase B (volume%) | 25 | 40 | 95 | 95 | 25 | 25 |

### Mass spectrometry conditions:

Mass spectrometer model: AB Sciex Triple TOF 5600+;
GS1 35; GS2 35; CUR 30; TEM 350; ISVF 5500; DP 250; CE 10; accumulation time 0.5 s;
m/z 600-4000; time bins to sum 40.

The mean DAR values of the immune-stimulating antibody conjugates (ISACs) were calculated by CE-SDS. The results are shown in Table 1:

**Table 1. The mean DAR values of immune-stimulating antibody conjugates (ISACs)**

| Immune-stimulating antibody conjugate (ISAC) | Preparation method for immune-stimulating antibody conjugate (ISAC) | Drug-linker (DL) compound | Mean DAR value |
|---|---|---|---|
| Trastuzumab-ISAC-2 | Preparation method A | DL-2 | 7.6 |
| Trastuzumab-ISAC-3 | Preparation method A | DL-3 | 7.9 |
| Trastuzumab-ISAC-4 | Preparation method A | DL-4 | 7.1 |
| Trastuzumab-ISAC-10 | Preparation method A | DL-10 | 8.0 |
| IgG1-ISAC-4 | Preparation method B | DL-4 | 7.8 |

### Biological Assays

### Experimental Example 1. Agonistic Effects of Compounds on STING-Mediated Interferon (IFN) Signal Reporter Gene in THP1-Blue^{™} ISG Cells

In this experiment, the activity of the secreted embryonic alkaline phosphatase (SEAP) reporter gene, which is regulated by interferon (IFN) regulatory factors, in THP1-Blue^{™} ISG cells (InvivoGen) was measured to assess the agonistic effects of the test compounds on the STING-mediated IFN signaling pathway at the cellular level.

THP1-Blue^{™} ISG cells (InvivoGen) in the logarithmic growth phase were centrifuged and resuspended in a cell culture medium to a density of 2 × 10⁶ cells/mL, and the cell suspension was seeded into a 96-well cell culture plate (Corning) at 50 µL/well. Stock solutions of the compounds were serially diluted with the cell culture medium to obtain 2× working concentration dilutions with concentrations of 60 µM, 7.5 µM,0.94 µM, 0.12 µM, 0.015 µM, 0.0018 µM, 0.00023 µM, 0.00003 µM, and 0 µM. The 2× working concentration dilutions of the compounds were added to the 96-well plate at 50 µL/well (the final concentration of DMSO was 1%). The culture plate was incubated in a cell incubator for 16 h. After the cell incubation, the cell culture supernatants (10 µL) were transferred to a 96-well plate, and a QUANTI-Blue (InvivoGen) solution was added at 90 µL/well. The plate was incubated at 37 °C for 3 h, and the absorbance at 620 nm (OD_{620 nm}) was measured using a microplate reader (BMG LABTECH).

EC₅₀ was calculated by log(agonist) vs. response -- Variable slope fitting in Graphpad Prism software. Emax was the OD_{620 nm} value where the maximum activation effect of the present test drug was achieved.

### Experimental results:

The activation activity of the compounds on THP1-Blue^{™} ISG cells was determined according to the method described above. The activity of the compounds was expressed in terms of EC₅₀ and Emax. The results are shown in Table 2:

**Table 2. The agonistic effects of the test compounds on the STING-mediated interferon (IFN) signaling pathway**

| Compound No. | EC₅₀ (µM) | Eₘₐₓ (OD₆₂₀ₙₘ) |
|---|---|---|
| C-3 | 0.068 | 1.67 |
| C-7 | 0.626 | 1.22 |
| C-9 | 0.079 | 1.25 |
| C-11 | 0.348 | 1.51 |

The results show that compound C-3, compound C-7, compound C-9, and compound C-11 exhibited very strong agonistic effects on the STING-mediated interferon (IFN) signaling pathway in THP1-Blue^{™} ISG cells.

### Experimental Example 2. Agonistic Effects of Compounds on Expression of Cytokine mIFN-β of STING Pathway in Murine Raw264.7 Cells

In this experiment, the expression of the cytokine mIFN-β of the STING signaling pathway in Raw264.7 cells (Nanjing Cobioer) was measured by ELISA to assess the agonistic effects of the test compounds on the expression of the cytokine mIFN-β of the STING pathway at the cellular level.

Raw264.7 cells (Nanjing Cobioer) in the logarithmic growth phase were centrifuged and resuspended in a cell culture medium to a density of 8 × 10⁶ cells/mL, and the cell suspension was seeded into 24-well cell culture plates (Corning) at 250 µL/well. The test compounds were diluted with the cell culture medium to obtain 2× working concentration dilutions with concentrations of 200 µM, 66.67 µM, 22.22 µM, 7.41 µM, 2.47 µM, 0.82 µM, 0.27 µM, 0.09 µM, and 0 µM. The dilutions of the test compounds (250 µL) were added to the 24-well plates (the final concentration of DMSO was 1%). After the dilutions were well mixed with cells, the plates were incubated in a cell incubator for 6 h. After the cell incubation, the plates were centrifuged at 300 g for 5 min, and the supernatants were collected. 100 µL of the cell culture solution was collected using a pipette and incubated with a primary antibody and an HRP-labeled secondary antibody according to the procedures of a mouse IFN-β ELISA kit (PBL-42400-2), and color development was performed. The absorbance at a wavelength of 450 nm (OD_{450 nm}) was measured.

EC₅₀ was calculated by log(agonist) vs. response -- Variable slope fitting in Graphpad Prism software. Emax was the amount of secreted mIFN-β where the maximum activation effect of the present test drug was achieved.

### Experimental results:

The activation activity of the compounds on the cytokine mIFN-β of the STING pathway in murine Raw264.7 cells was determined according to the method described above. The activity of the compounds was expressed in terms of EC₅₀ and Emax. The results are shown in Table 3:

**Table 3. The activation activity of the test compounds on the expression of the cytokine mIFN-β of the STING pathway in murine Raw264.7 cells**

| Compound No. | EC₅₀ (µM) | Eₘₐₓ (pg/mL) |
|---|---|---|
| C-3 | 0.51 | 2230 |
| C-9 | 2.23 | 1706 |
| C-11 | 7.84 | 1523 |

The results show that compound C-3, compound C-9, and compound C-11 exhibited very strong agonistic effects on the expression of the cytokine mIFN-β of the STING pathway in murine Raw264.7 cells.

### Experimental Example 3: Determination of Abilities of Immune-Stimulating Antibody Conjugates (ISACs) to Stimulate Secretion of TNF-α by Human Peripheral Blood Mononuclear Cells (PBMCs) in Presence of Tumor Cells

### Experimental procedures:

NCI-N87 tumor cells in the logarithmic growth phase (ATCC), which express HER2, were collected, washed twice with PBS, and resuspended in an RPMI1640 + 10% heat-inactivated FBS complete culture medium, and the cell density was adjusted to 2 × 10⁶ cells/mL. The NCI-N87 tumor cells were seeded in a 96-well plate (Corning) at 1 × 10⁵ cells/well. PBMCs (Oribiotech) were thawed and resuspended in an RPMI1640 + 10% heat-inactivated FBS complete culture medium, and the density was adjusted to 1 × 10⁷ cells/mL. The suspension was added at 5 × 10⁵ PBMCs/well to the 96-well plate in which the tumor cells had been seeded. Samples of trastuzumab and immune-stimulating antibody conjugates (ISACs) diluted in 100 µL of an RPMI1640 + 10% heat-inactivated FBS complete culture medium were added, with final concentrations of 50 nM, 25 nM, 12.5 nM, 6.25 nM, 3.13 nM, 1.56 nM, 0.78 nM, 0.39 nM, and 0.20 nM. 100 µL of an RPMI1640 + 10% heat-inactivated FBS complete culture medium was added to a blank well. After the plate was incubated in an incubator at 37 °C with 5% CO₂ for 24 h, 100 µL of the supernatant was taken from each well for analysis and incubated with a primary antibody and an HRP-labeled secondary antibody according to the procedures of a human TNF-α ELISA kit (R&D system), and color development was performed. The absorbance at a wavelength of 450 nm was measured. The concentration of TNF-α was calculated from a standard curve using Graphpad Prism software.

EC₅₀ was calculated by log(agonist) vs. response -- Variable slope fitting in Graphpad Prism software. Emax was the amount of secreted TNF-α where the maximum activation effect of the present test drug was achieved.

### Experimental results:

The abilities of trastuzumab and the immune-stimulating antibody conjugates (ISACs) to stimulate the secretion of TNF-α by human peripheral blood mononuclear cells (PBMCs) in the presence of NCI-N87 tumor cells were determined according to the method described above. The results are shown in Table 4:

**Table 4. The results of the stimulation of TNF-α secretion by the test samples in an in vitro NCI-N87 tumor cell and PBMC co-incubation system**

| Antibody or immune-stimulating antibody conjugate (ISAC) | EC₅₀ (nM) | Emax (TNF-α, pg/mL) |
|---|---|---|
| Trastuzumab | >50 | 460 |
| Trastuzumab-ISAC-2 | 1.94 | 1952 |
| Trastuzumab-ISAC-3 | 1.45 | 2179 |
| Trastuzumab-ISAC-4 | 0.36 | 2693 |

The results show that the immune-stimulating antibody conjugates trastuzumab-ISAC-2, trastuzumab-ISAC-3, and trastuzumab-ISAC-4 effectively stimulated the secretion of TNF-α by PBMCs in the *in vitro* NCI-N87 tumor cell and PBMC co-incubation system.

### Experimental Example 4. In Vivo Efficacy Study of Immune-Stimulating Antibody Conjugates (ISACs) in NCI-N87 Cell Subcutaneous Tumor-Bearing Mouse Model

### Experimental procedures:

Each BALB/c Nude mouse (GemPharmatech) was subcutaneously inoculated with 5 × 10⁶ NCI-N87 cells (suspended in 0.1 mL of PBS) in the right axilla. When the mean tumor volume reached 100-150 mm³, mice with irregular and excessively small or excessively large tumor volumes were excluded. The remaining mice were randomly grouped according to tumor volume and animal body weight, and the groups were intraperitoneally (i.p.) given a 0.9% sodium chloride injection (vehicle control), IgG1-ISAC-4 (10 mg/kg) (isotype control), and the immune-stimulating antibody conjugate trastuzumab-ISAC-4 (10 mg/kg), respectively, once weekly for a total of two doses. The efficacy of the test drugs in the tumor-bearing mouse model and the tolerance of the animals to the test drugs were observed. In the experiment, the mouse body weight and tumor volume were measured twice weekly, and the measurements were recorded.

Statistical analysis of the measurements was performed. The calculation formula for tumor volume (V) was V = 1/2 × a × b², where a and b represent length and width, respectively. The anti-tumor efficacy was evaluated using the tumor growth inhibition rate TGI (%). The calculation formula was TGI (%) _{(tumor volume)} = [1 - (T_{Vt} - T_{V0})/(C_{Vt} - C_{V0})] × 100%, where T_{V0} represents the mean tumor volume of the test compound group at the time of grouping and administration, T_{Vt} represents the mean tumor volume of the test compound group on day t post-dose, C_{V0} represents the mean tumor volume of the vehicle group at the time of grouping and administration, and C_{Vt} represents the mean tumor volume of the vehicle group on day t post-dose. When a tumor regressed, TGI (%)_{(tumor volume)} = 100% - (T_{Vt} - T_{V0})/T_{V0} × 100%. The case that the volume of a tumor shrinks compared to its initial volume, i.e., Vₜ < V₀, is defined as partial regression (PR) of the tumor, and the case that a tumor completely disappears is defined as complete regression (CR) of the tumor.

### Experimental results:

FIG. 1 shows curves of the tumor volume of each group in Experimental Example 4 changing over time, exhibiting the *in vivo* efficacy of each test drug in the xenograft tumor model of human gastric cancer constructed by subcutaneously inoculating BALB/c Nude mice with NCI-N87 cells. Twenty-eight days after administration, the immune-stimulating antibody conjugate trastuzumab-ISAC-4 significantly inhibited tumor growth, with a tumor volume TGI of 99.6% (P < 0.001) compared to the vehicle control group, while the isotype control IgG1-ISAC-4 exhibited a significantly weaker effect (FIG. 1, Table 5). The results show that in the xenograft tumor model of human gastric cancer constructed by subcutaneously inoculating BALB/c Nude mice with NCI-N87 cells, intraperitoneally (i.p.) injecting the immune-stimulating antibody conjugate trastuzumab-ISAC-4 once weekly for a total of two doses (QW × 2) exhibited a very significant anti-tumor effect, and the effect was significantly superior to those of the vehicle control group and the isotype control group.

**Table 5. The efficacy of immune-stimulating antibody conjugates in an NCI-N87 cell subcutaneous tumor-bearing mouse model**

| Drug | Tumor growth inhibition rate (TGI) | | | | Tumor regression |
|---|---|---|---|---|---|
| | Day 7 | Day 14 | Day 21 | Day 28 | |
| 0.9% sodium chloride injection | - | - | - | - | 0/6 |
| IgG1-ISAC-4 10 mg/kg | 13.81% | 34.31% | 32.55% | 28.91% | 0/6 |
| Trastuzumab-ISAC-4 10 mg/kg | 91.25% | 136.26% | 114.75% | 99.60% | 2/6(PR), 2/6(CR) |

PR stands for partial regression, and CR stands for complete regression.

The above examples do not limit the solutions of the present application in any way. Various modifications of the present disclosure, in addition to those described herein, will be apparent to those skilled in the art on the basis of the aforementioned description. Such modifications are also intended to fall within the scope of the appended claims. References (including all patents, patent applications, journal articles, books, and any other publications) cited in the present application are all incorporated herein by reference in their entirety.

## Claims

1. An immune-stimulating antibody conjugate of formula (I): 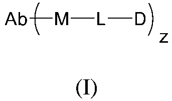 wherein
Ab is an antibody targeting a target antigen or an antigen-binding fragment thereof;
M is a linker part linking the antibody or the antigen-binding fragment thereof;
L is a linker linking M and D;
D is selected from structures of formula (D-I-1) and formula (D-I-2): 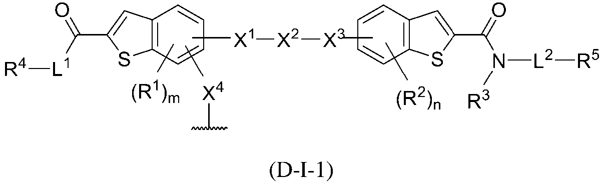 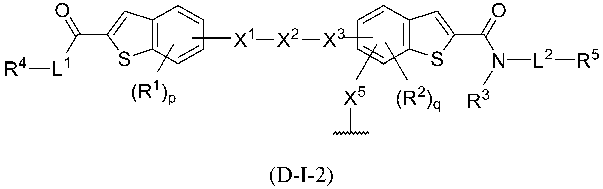
wherein 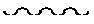 represents a point of attachment to L;
X¹ and X³ are identical or different and are each independently selected from a covalent bond, -O-, -S-, and -NR^{a}-;
X² is selected from C₁₋₆ alkylene, C₂₋₆ alkenylene, C₂₋₆ alkynylene, C₃₋₆ cycloalkyl, and 3-6 membered heterocyclyl, wherein the C₁₋₆ alkylene, C₂₋₆ alkenylene, C₂₋₆ alkynylene, C₃₋₆ cycloalkyl, and 3-6 membered heterocyclyl are each optionally substituted with one or more substituents independently selected from H, halogen, cyano, C₁₋₆ alkyl, -OR^{a}, and -NR^{a}R^{b};
X⁴ and X⁵ are each independently selected from -O- and -NR^{a}-;
L¹ is selected from a covalent bond and -(C(R⁶)₂)ⱼ-;
L² is selected from a covalent bond and -(C(R⁷)₂)ₖ-;
R¹ and R² are identical or different and are each independently selected from H, halogen, cyano, -OR^{a}, - NR^{a}R^{b}, -C(O)-OR^{a}, -C(O)-NR^{a}R^{b}, -NR^{a}-C(O)-R^{a}, C₁₋₆ alkyl, C₃₋₆ cycloalkyl, and 3-6 membered heterocyclyl, wherein the C₁₋₆ alkyl, C₃₋₆ cycloalkyl, and 3-6 membered heterocyclyl are each optionally substituted with one or more substituents independently selected from H, halogen, C₁₋₆ alkyl, -OR^{a}, and - NR^{a}R^{b};
R³ is selected from H and C₁₋₆ alkyl;
R⁴ is selected from H, -OR^{a}, -NR^{a}R^{b}, -C(O)-OR^{a}, -O-C(O)-R^{a}, -C(O)-NR^{a}R^{b}, -NR^{a}-C(O)-R^{a}, -O-C(O)-NR^{a}R^{b}, -NR^{a}-C(O)-OR^{a}, -NR^{a}-C(O)-NR^{a}R^{b}, C₁₋₆ alkyl, C₃₋₆ cycloalkyl, 3-6 membered heterocyclyl, C₆₋₁₀ aryl, and 5-10 membered heteroaryl, wherein the C₁₋₆ alkyl, C₃₋₆ cycloalkyl, 3-6 membered heterocyclyl, C₆₋₁₀ aryl, and 5-10 membered heteroaryl are each optionally substituted with one or more substituents independently selected from H, halogen, C₁₋₆ alkyl, -OR^{a}, -NR^{a}R^{b}, and -C(O)-OR^{a};
R⁵ is selected from H, C₁₋₆ alkyl, C₃₋₁₀ cycloalkyl, 3-10 membered heterocyclyl, -OR^{a}, and -C(O)-OR^{a}, wherein the C₁₋₆ alkyl, C₃₋₁₀ cycloalkyl, and 3-10 membered heterocyclyl are each optionally substituted with one or more substituents independently selected from H, halogen, C₁₋₆ alkyl, -OR^{a}, -NR^{a}R^{b}, and - C(O)-OR^{a};
each R⁶ is independently selected from H, -OR^{a}, -NR^{a}R^{b}, C₁₋₆ alkyl, C₂₋₆ alkenyl, C₂₋₆ alkynyl, C₃₋₆ cycloalkyl, and 3-6 membered heterocyclyl, wherein the C₁₋₆ alkyl, C₂₋₆ alkenyl, C₂₋₆ alkynyl, C₃₋₆ cycloalkyl, and 3-6 membered heterocyclyl are each optionally substituted with one or more substituents independently selected from H, halogen, C₁₋₆ alkyl, -OR^{a}, -NR^{a}R^{b}, and -C(O)-OR^{a}; or
two R⁶ groups on different carbon atoms, together with the carbon atoms therebetween, form C₃₋₆ cycloalkyl or 3-6 membered heterocyclyl; or
two R⁶ groups on the same carbon atom, together with the carbon atom to which they are both attached, form C₃₋₆ cycloalkyl or 3-6 membered heterocyclyl;
each R⁷ is independently selected from H, -OR^{a}, -NR^{a}R^{b}, C₁₋₆ alkyl, C₂₋₆ alkenyl, C₂₋₆ alkynyl, C₃₋₆ cycloalkyl, and 3-6 membered heterocyclyl, wherein the C₁₋₆ alkyl, C₂₋₆ alkenyl, C₂₋₆ alkynyl, C₃₋₆ cycloalkyl, and 3-6 membered heterocyclyl are each optionally substituted with one or more substituents independently selected from H, halogen, C₁₋₆ alkyl, -OR^{a}, -NR^{a}R^{b}, and -C(O)-OR^{a}; or
two R⁷ groups on the same carbon atom, together with the carbon atom to which they are both attached, form C₃₋₆ cycloalkyl or 3-6 membered heterocyclyl; or any one R⁷ and R³, together with the atoms therebetween, form 3-10 membered heterocyclyl;
R^{a} and R^{b} are each independently selected from H, C₁₋₆ alkyl, C₃₋₆ cycloalkyl, and 3-6 membered heterocyclyl, wherein the C₁₋₆ alkyl, C₃₋₆ cycloalkyl, and 3-6 membered heterocyclyl are each optionally substituted with one or more substituents independently selected from H, halogen, C₁₋₆ alkyl, hydroxy, amino, and carboxyl;
j and k are each independently selected from 1, 2, and 3;
m and q are each independently selected from 0, 1, and 2;
n and p are each independently selected from 0, 1, 2, and 3;
z is selected from 1-10.

2. The immune-stimulating antibody conjugate according to claim 1, wherein M is selected from the following structures: 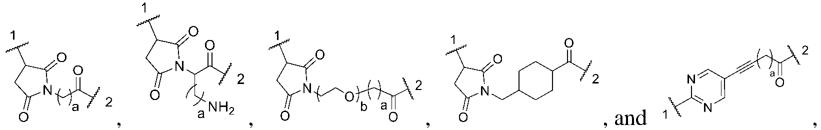
wherein position 1 of M is linked to Ab, and position 2 is linked to L; each a is independently selected from integers of 1-6, and b is selected from integers of 1-10;
preferably, M is selected from the following structures: 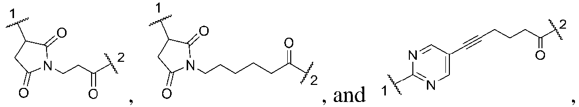
wherein position 1 of M is linked to Ab, and position 2 is linked to L.

3. The immune-stimulating antibody conjugate according to any one of claims 1-2, wherein L is selected from a non-cleavable linker and a cleavable linker; the cleavable linker is cleaved by an enzyme present in a pathological environment, and the enzyme is selected from a protease, a phosphatase, a pyrophosphatase, *β*-glucuronidase, *β*-galactosidase, and a sulfatase;
preferably, L is -L^{a}-L^{b}-L^{c}-, wherein:
L^{a} is a covalent bond or is selected from 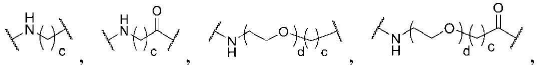 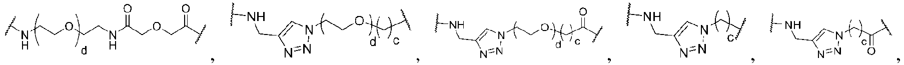 and 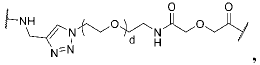
wherein each c is independently selected from integers of 1-6, and each d is independently selected from integers of 1-10;
L^{b} is a covalent bond or is selected from an amino acid fragment and a peptide fragment formed from two or more amino acids, wherein the amino acids are selected from Val, Cit, Glu, Lys, Arg, Phe, Leu, Gly, Ala, and Asn;
L^{c} is a covalent bond or -NH-CH₂-, or is selected from the following structures: 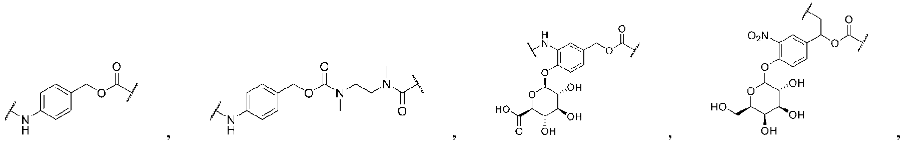 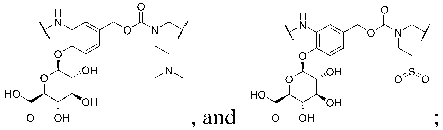
preferably, L^{a} is a covalent bond or 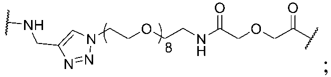 preferably, L^{a} is a covalent bond;
preferably, L^{b} is a covalent bond or is selected from the following structures: 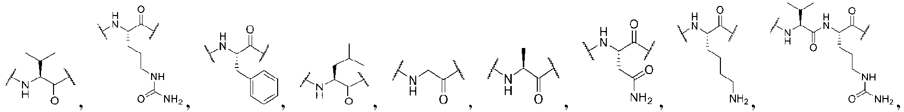 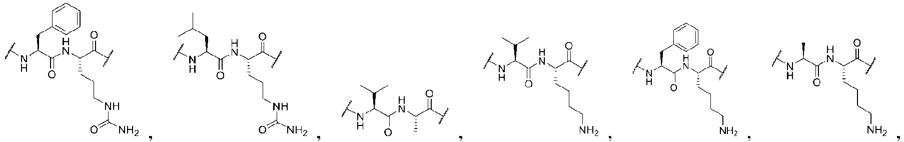 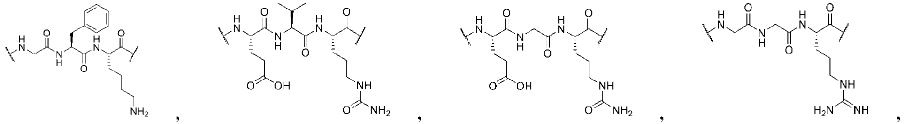 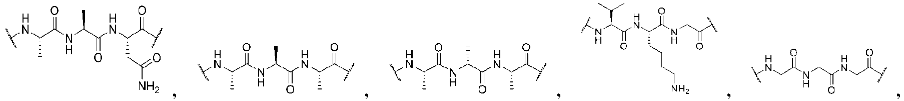 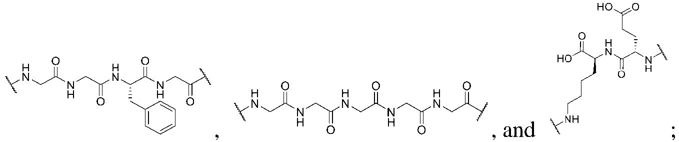
preferably, L^{b} is selected from 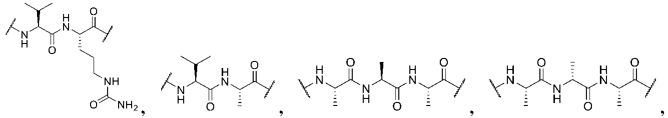 and

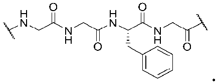

4. The immune-stimulating antibody conjugate according to any one of claims 1-3, wherein L is selected from the following structures: 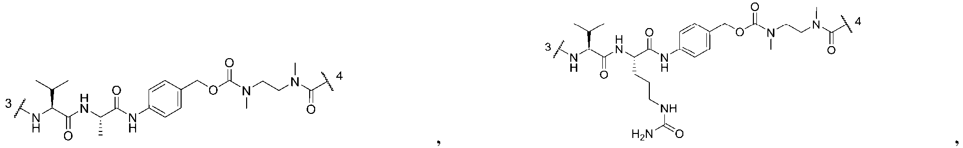 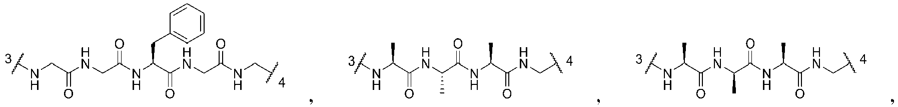 and 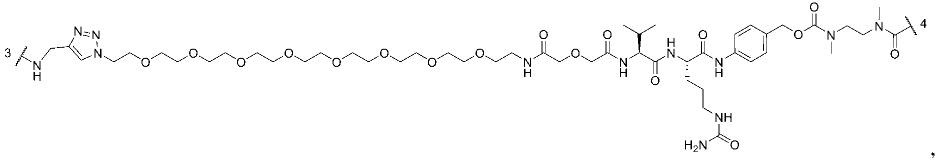 wherein position 3 of L is linked to M, and position 4 is linked to D.

5. The immune-stimulating antibody conjugate according to any one of claims 1-4, wherein D is selected from structures of formula (D-II-1) and formula (D-II-2): 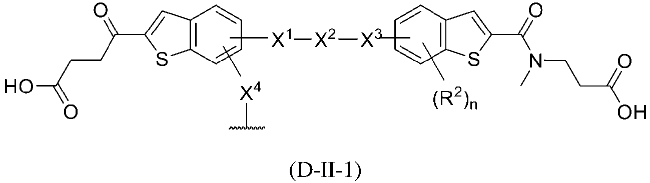 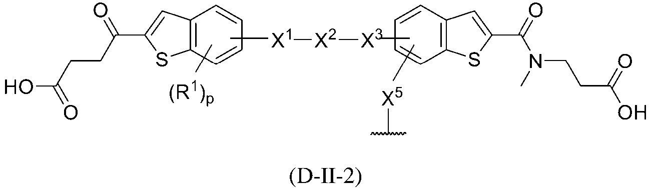
wherein 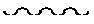 represents a point of attachment to L, and the groups R¹, R², X¹, X², X³, X⁴, and X⁵, n, and p are as defined in any one of claims 1-4;
preferably, D is selected from structures of formula (D-III-1), formula (D-III-2), formula (D-III-3), and formula (D-III-4): 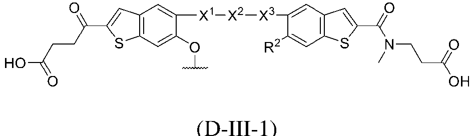 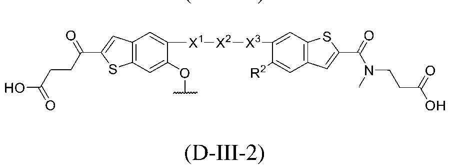 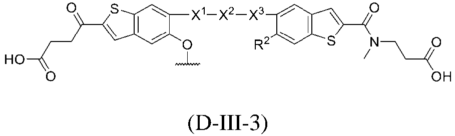 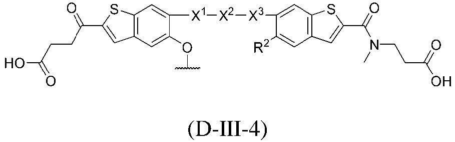
wherein 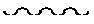 represents a point of attachment to L, and the groups R², X¹, X², and X³ are as defined in any one of claims 1-4.

6. The immune-stimulating antibody conjugate according to any one of claims 1-5, wherein D is selected from the following structures: 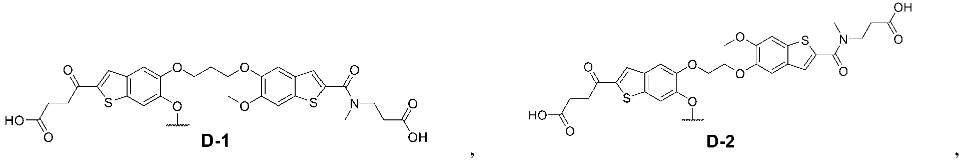 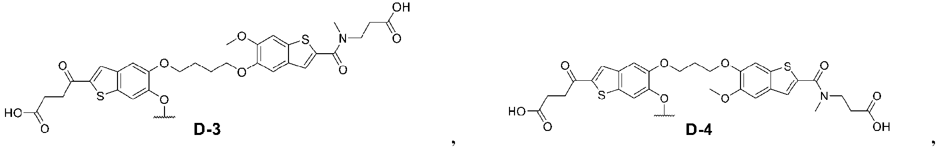 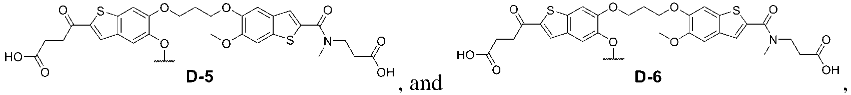
wherein 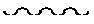 represents a point of attachment to L;
preferably, D is

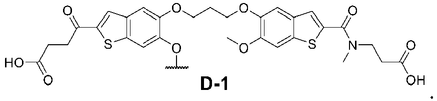

7. The immune-stimulating antibody conjugate according to any one of claims 1-6, wherein Ab is an antibody or an antigen-binding fragment thereof; the antibody is selected from a monoclonal antibody, a polyclonal antibody, a linear antibody, a bispecific antibody, a multispecific antibody, a chimeric antibody, a murine antibody, a humanized antibody, a fully human antibody, and a fusion protein comprising an antigen-binding moiety of an antibody; the antigen-binding fragment is selected from Fab, Fab', F(ab')2, Fv, disulfide-linked Fv, and scFv;
preferably, the antibody or the antigen-binding fragment thereof is an anti-Her-2 antibody;
preferably, the antibody or the antigen-binding fragment thereof comprises:
(1) the following heavy chain variable region (VH) and/or light chain variable region (VL):
a heavy chain variable region (VH) comprising the following 3 CDRs: a CDR-H1 whose sequence is SEQ ID NO: 3 or a variant thereof, a CDR-H2 whose sequence is SEQ ID NO: 4 or a variant thereof, and a CDR-H3 whose sequence is SEQ ID NO: 5 or a variant thereof; and/or a light chain variable region (VL) comprising the following 3 CDRs: a CDR-L1 whose sequence is SEQ ID NO: 6 or a variant thereof, a CDR-L2 whose sequence is SEQ ID NO: 7 or a variant thereof, and a CDR-L3 whose sequence is SEQ ID NO: 8 or a variant thereof;
(2) the following heavy chain variable region (VH) and/or light chain variable region (VL):
a heavy chain variable region (VH) comprising the following 3 CDRs: a CDR-H1 whose sequence is SEQ ID NO: 9 or a variant thereof, a CDR-H2 whose sequence is SEQ ID NO: 10 or a variant thereof, and a CDR-H3 whose sequence is SEQ ID NO: 5 or a variant thereof; and/or a light chain variable region (VL) comprising the following 3 CDRs: a CDR-L1 whose sequence is SEQ ID NO: 6 or a variant thereof, a CDR-L2 whose sequence is SEQ ID NO: 7 or a variant thereof, and a CDR-L3 whose sequence is SEQ ID NO: 8 or a variant thereof;
(3) the following heavy chain variable region (VH) and/or light chain variable region (VL):
a heavy chain variable region (VH) comprising the following 3 CDRs: a CDR-H1 whose sequence is SEQ ID NO: 11 or a variant thereof, a CDR-H2 whose sequence is SEQ ID NO: 12 or a variant thereof, and a CDR-H3 whose sequence is SEQ ID NO: 13 or a variant thereof; and/or a light chain variable region (VL) comprising the following 3 CDRs: a CDR-L1 whose sequence is SEQ ID NO: 14 or a variant thereof, a CDR-L2 whose sequence is SEQ ID NO: 15 or a variant thereof, and a CDR-L3 whose sequence is SEQ ID NO: 8 or a variant thereof; or
(4) the following heavy chain variable region (VH) and/or light chain variable region (VL):
a heavy chain variable region (VH) comprising the following 3 CDRs: a CDR-H1 whose sequence is SEQ ID NO: 16 or a variant thereof, a CDR-H2 whose sequence is SEQ ID NO: 17 or a variant thereof, and a CDR-H3 whose sequence is SEQ ID NO: 5 or a variant thereof; and/or a light chain variable region (VL) comprising the following 3 CDRs: a CDR-L1 whose sequence is SEQ ID NO: 6 or a variant thereof, a CDR-L2 whose sequence is SEQ ID NO: 7 or a variant thereof, and a CDR-L3 whose sequence is SEQ ID NO: 8 or a variant thereof;
preferably, the antibody or the antigen-binding fragment thereof comprises: a VH set forth in SEQ ID NO: 18 or a variant thereof, and/or a VL set forth in SEQ ID NO: 19 or a variant thereof;
preferably, the antibody or the antigen-binding fragment thereof comprises: a heavy chain comprising a VH set forth in SEQ ID NO: 18 or a variant thereof and a heavy chain constant region (CH) set forth in SEQ ID NO: 20 or a variant thereof, and/or a light chain comprising a VL set forth in SEQ ID NO: 19 or a variant thereof and a (light chain constant region) CL set forth in SEQ ID NO: 21 or a variant thereof;
preferably, the anti-Her-2 antibody is trastuzumab;
preferably, the amino acid sequence of a heavy chain of trastuzumab is set forth in SEQ ID NO: 1, and the amino acid sequence of a light chain of trastuzumab is set forth in SEQ ID NO: 2.

8. The immune-stimulating antibody conjugate according to any one of claims 1-7, wherein the immune-stimulating antibody conjugate is selected from the following structures: 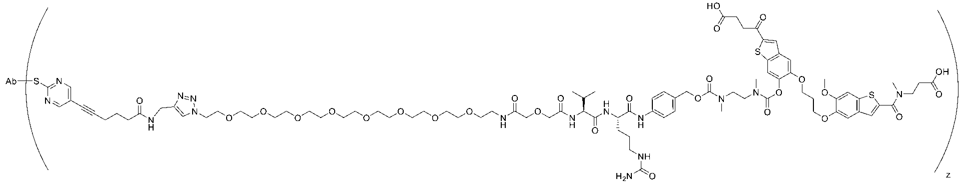 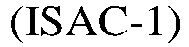 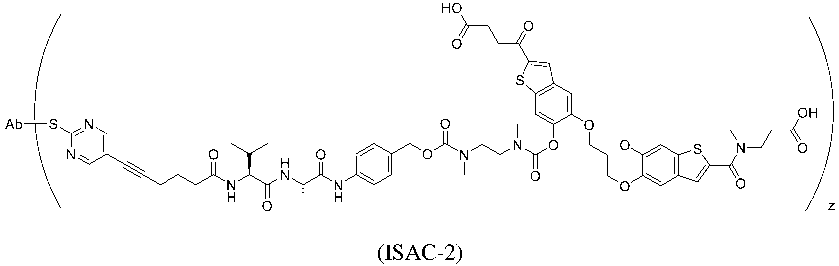 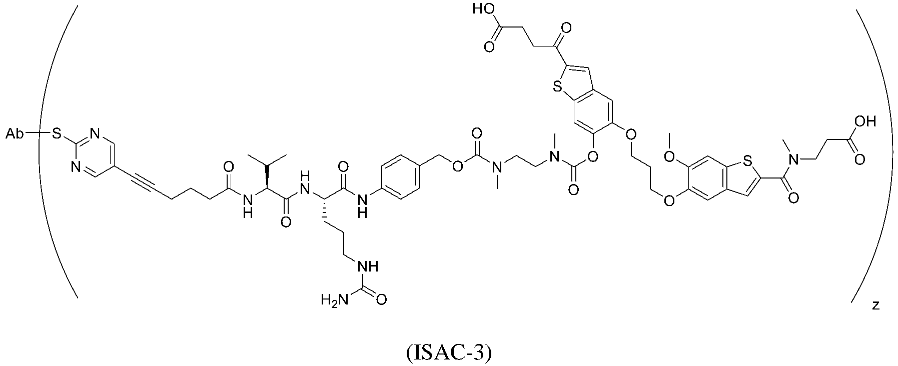 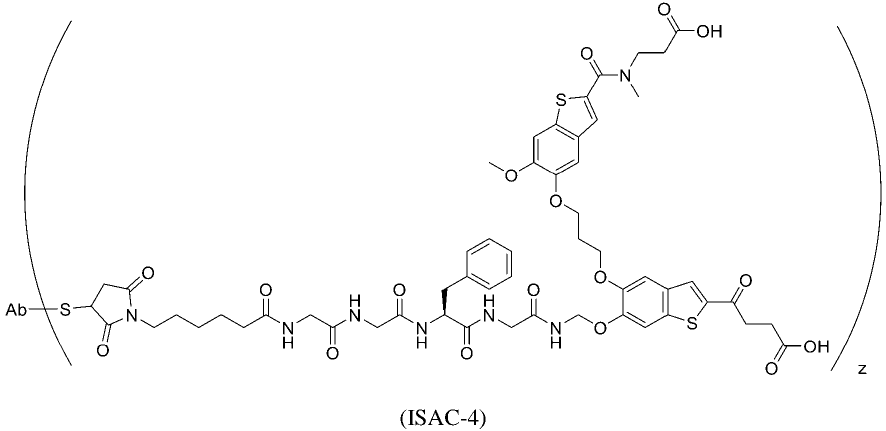 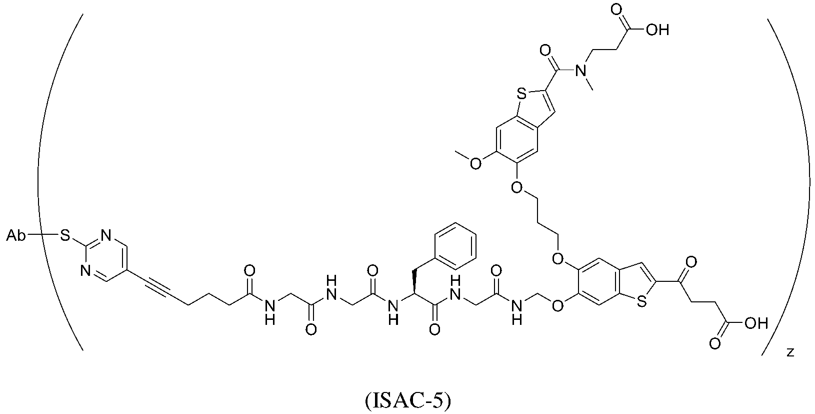 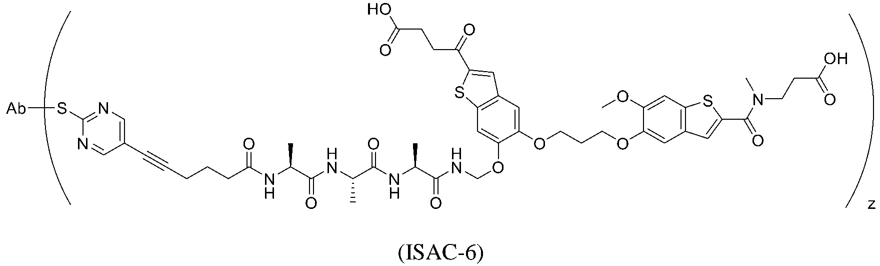 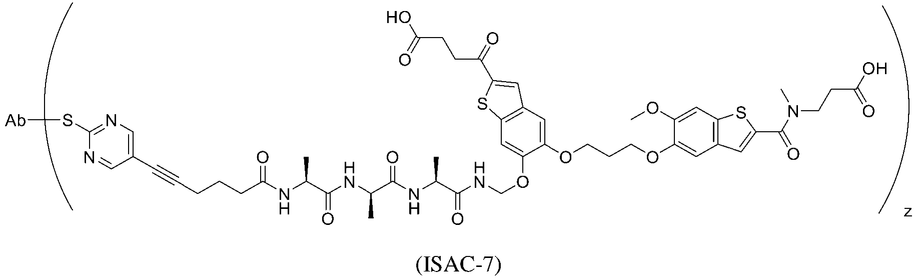 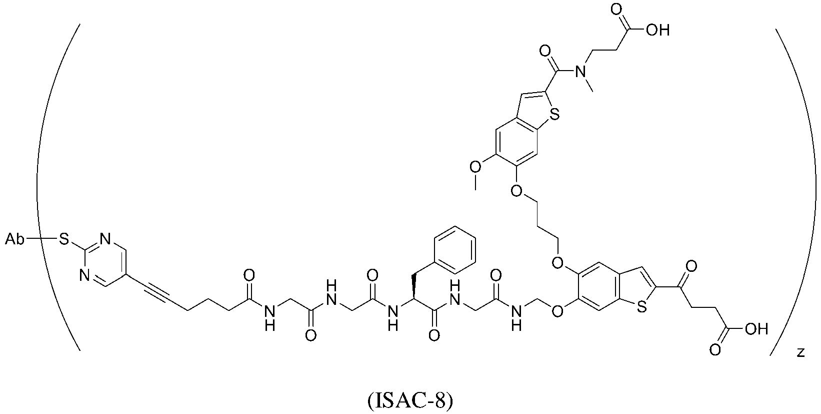 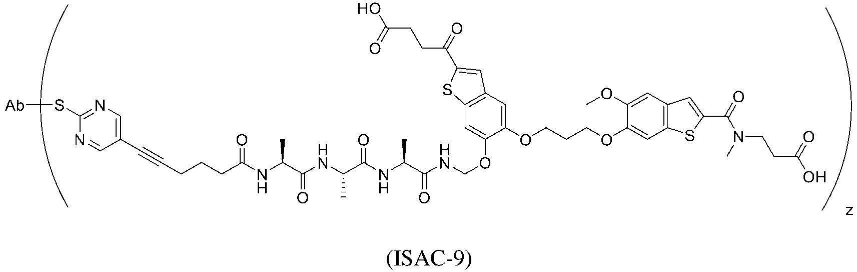 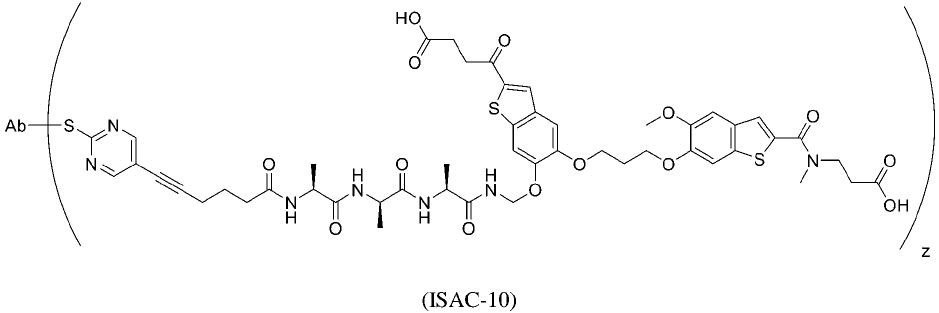
each z is selected from 1 to 10;
preferably, Ab is trastuzumab.

9. An immune-stimulating antibody conjugate, wherein the immune-stimulating antibody conjugate is selected from the following structures: 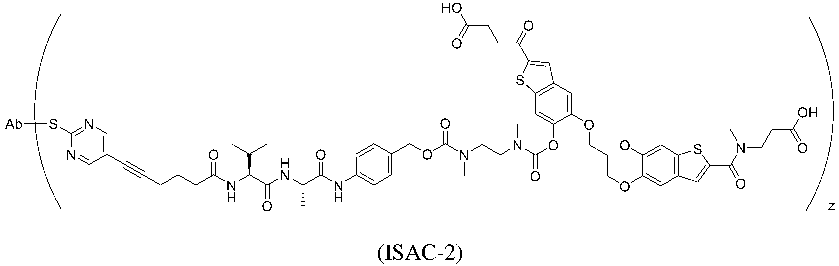 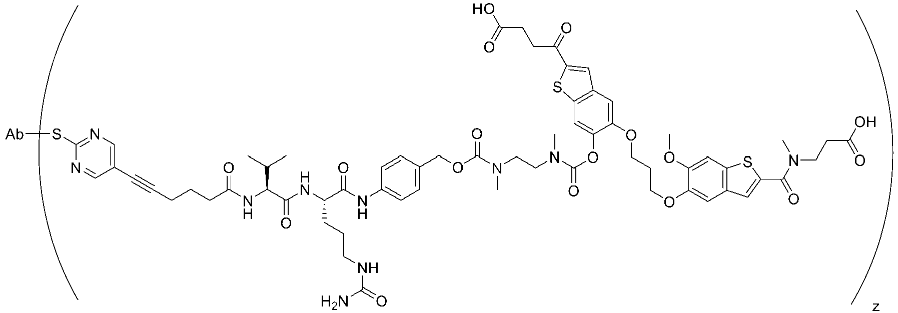 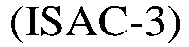 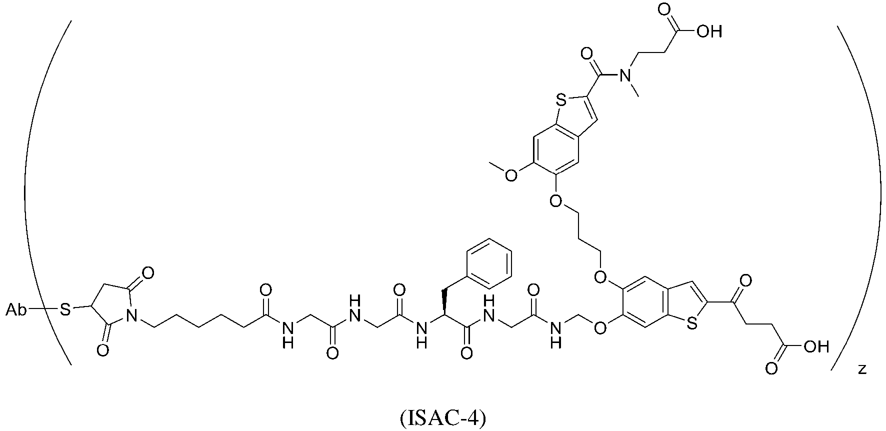
each z is selected from 1 to 10;
Ab is selected from an anti-Her-2 antibody;
preferably, Ab comprises a VH set forth in SEQ ID NO: 18 and/or a VL set forth in SEQ ID NO: 19;
preferably, Ab comprises a heavy chain comprising a VH set forth in SEQ ID NO: 18 and a CH set forth in SEQ ID NO: 20, and/or a light chain comprising a VL set forth in SEQ ID NO: 19 and a CL set forth in SEQ ID NO: 21;
preferably, Ab is trastuzumab.

10. A drug-linker compound of formula (II) or a pharmaceutically acceptable salt, ester, stereoisomer, tautomer, polymorph, solvate, N-oxide, isotopically labeled compound, metabolite, or prodrug thereof:
M¹-L-D (II)
wherein
M¹ is selected from 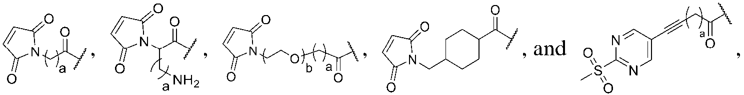
wherein each a is independently selected from integers of 1-6, and b is selected from integers of 1-10;
L and D are as defined in any one of claims 1-9.

11. The drug-linker compound according to claim 10, wherein the drug-linker compound is selected from the following structures:

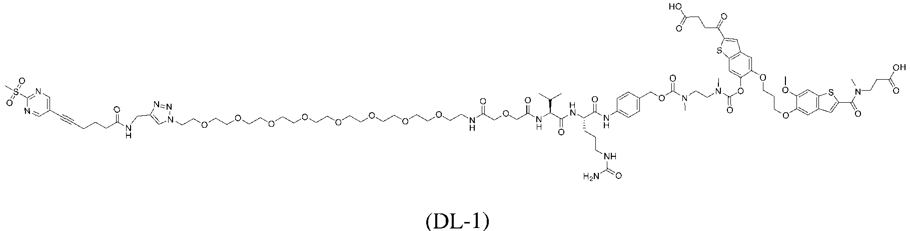

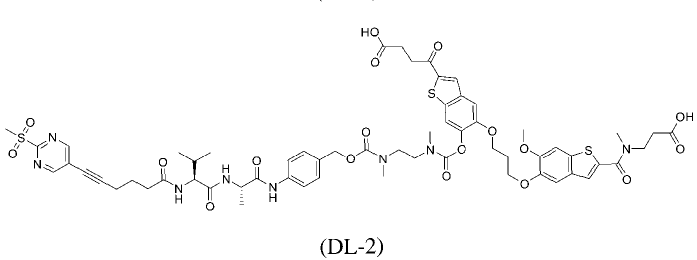

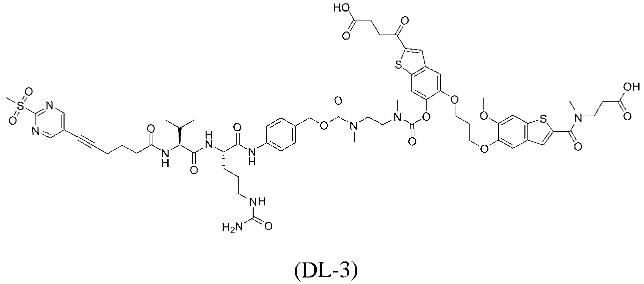

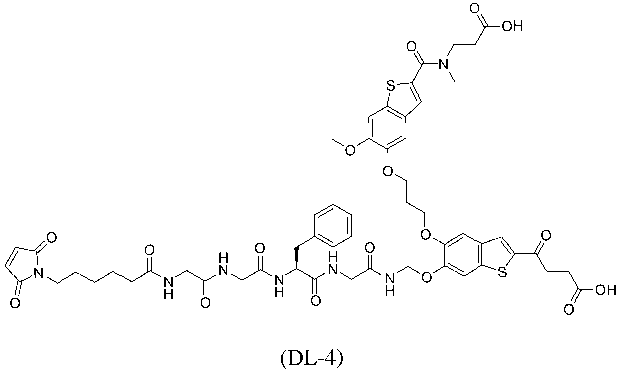

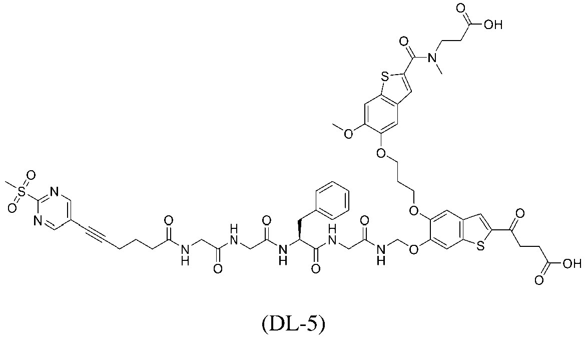

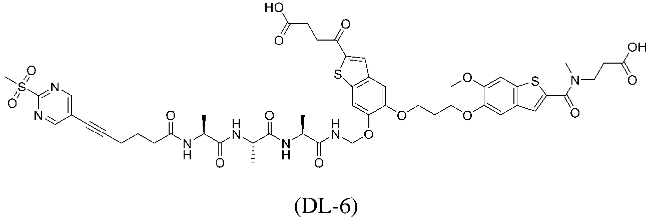

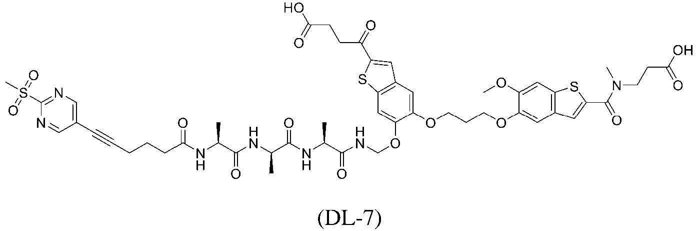

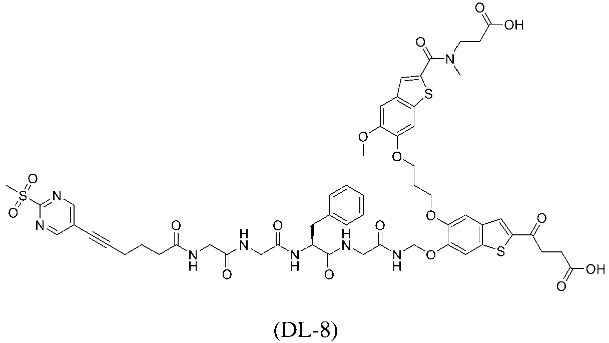

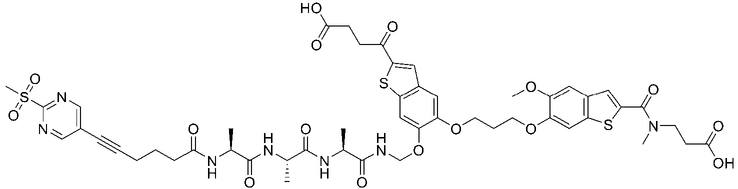

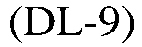

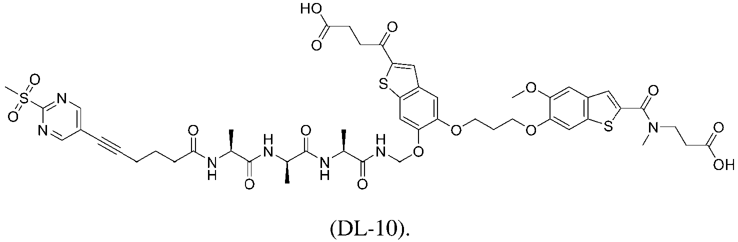

12. A compound represented by formula III-1 or III-2 or a pharmaceutically acceptable salt, ester, stereoisomer, tautomer, polymorph, solvate, *N*-oxide, isotopically labeled compound, metabolite, or prodrug thereof: 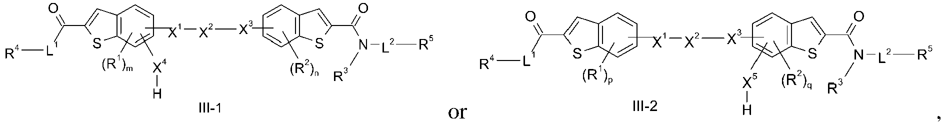
wherein R¹, R², R³, R⁴, R⁵, L¹, L², X¹, X², X³, X⁴, X⁵, m, n, p, and q are as defined in any one of claims 1-9;
preferably, the compound is selected from the following structures: 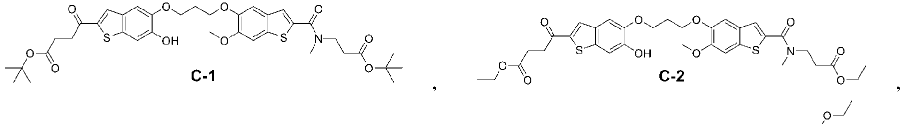 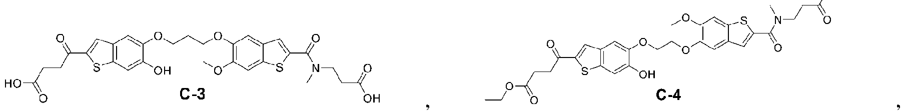 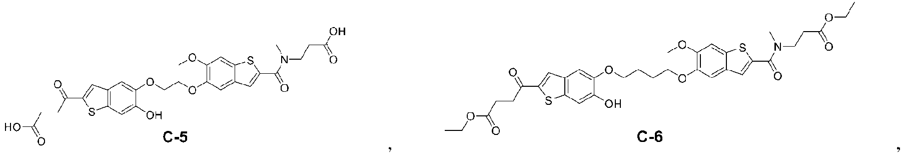 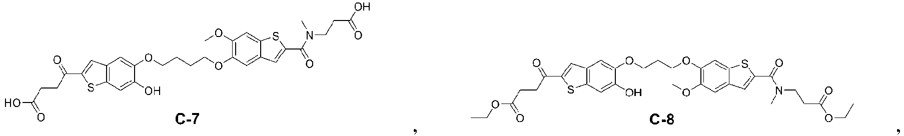 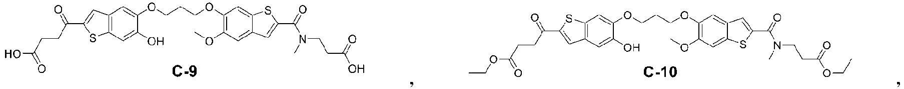 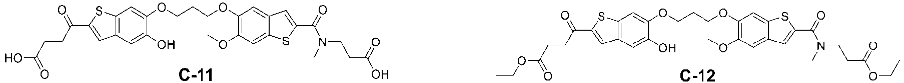 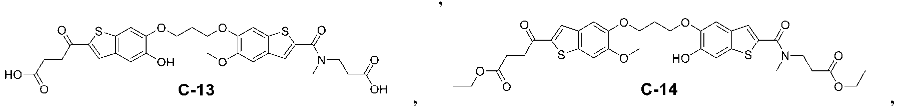 , and

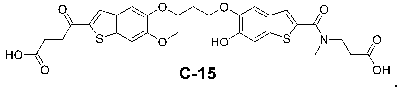

13. A compound represented by formula IV-1 or IV-2 or a pharmaceutically acceptable salt, ester, stereoisomer, tautomer, polymorph, solvate, N-oxide, isotopically labeled compound, metabolite, or prodrug thereof: 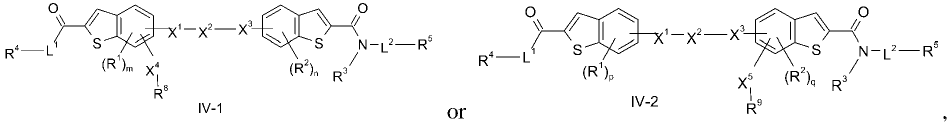
wherein R¹, R², R³, R⁴, R⁵, L¹, L², X¹, X², X³, X⁴, X⁵, m, n, p, and q are as defined in any one of claims 1-9;
R⁸ and R⁹ are each independently selected from -C₁₋₆ alkylene-NR¹²-C(=O)-C₁₋₆ alkylene-NR¹⁰R¹¹, - C(=O)O-C₆₋₁₀ aryl, -C(=O)NR¹²-C₁₋₆ alkylene-NR¹⁰R¹¹, -C₁₆ alkylene-O-C₁₋₆ alkyl, and -C₁₋₆ alkylene-C(=O)-C₆₋₁₀ aryl, wherein the aryl is optionally substituted with amino or nitro;
R¹⁰ and R¹¹ are each independently selected from H, C₁₋₆ alkyl, Fmoc, and Boc;
R¹² is selected from H and C₁₋₆ alkyl;
preferably, the compound is selected from the following structures: 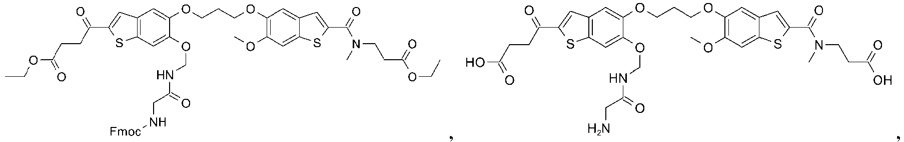 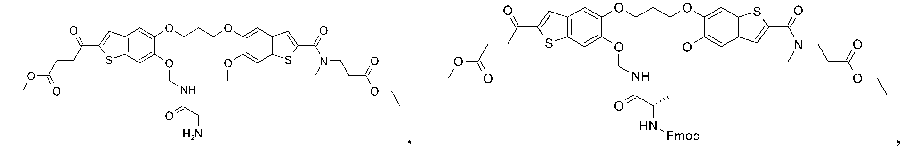 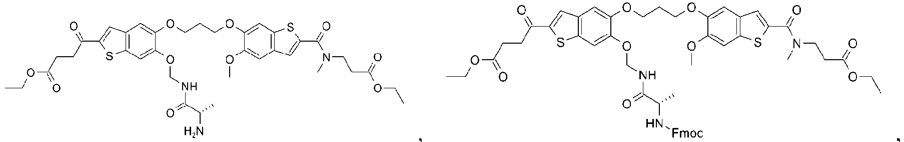 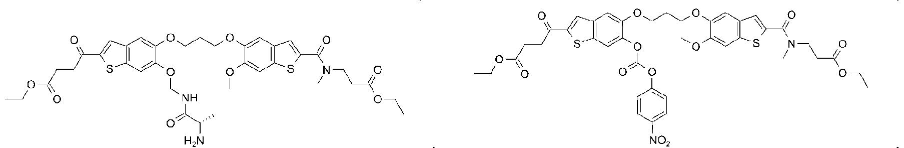 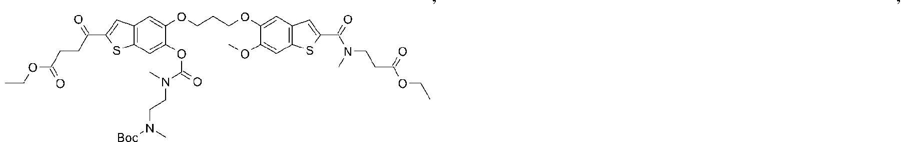 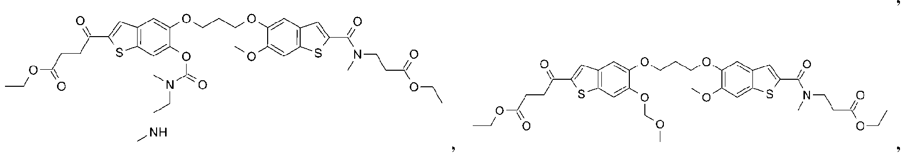 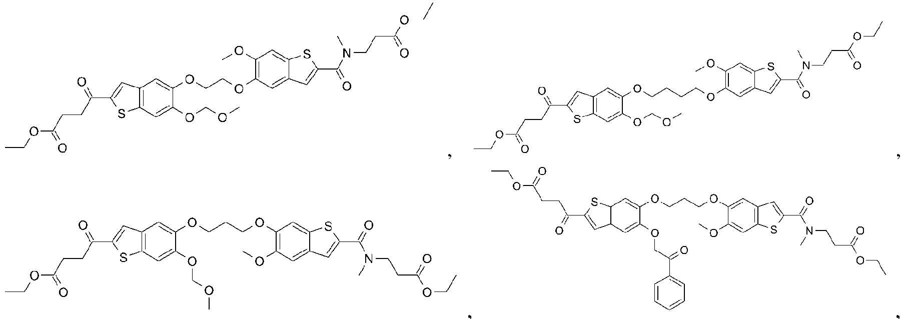 and

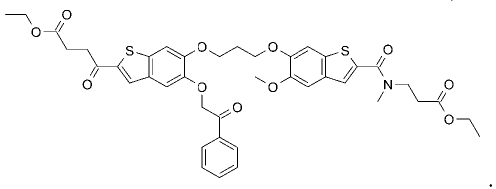

14. Use of the drug-linker compound or the pharmaceutically acceptable salt, ester, stereoisomer, tautomer, polymorph, solvate, N-oxide, isotopically labeled compound, metabolite, or prodrug thereof according to claim 10 or 11 or the compound or the pharmaceutically acceptable salt, ester, stereoisomer, tautomer, polymorph, solvate, N-oxide, isotopically labeled compound, metabolite, or prodrug thereof according to claim 12 or 13 in the preparation of an immune-stimulating antibody conjugate, wherein:
preferably, the immune-stimulating antibody conjugate is as described in any one of claims 1-9.

15. A pharmaceutical composition, comprising the immune-stimulating antibody conjugate according to any one of claims 1-9, wherein:
preferably, the pharmaceutical composition has a DAR value of 1.0-10.0, e.g., 6.0-9.0;
preferably, the pharmaceutical composition further comprises one or more pharmaceutically acceptable auxiliary materials.

16. A pharmaceutical composition, comprising the immune-stimulating antibody conjugate according to any one of claims 1-9, the drug-linker compound or the pharmaceutically acceptable salt, ester, stereoisomer, tautomer, polymorph, solvate, *N*-oxide, isotopically labeled compound, metabolite, or prodrug thereof according to claim 10 or 11, the compound or the pharmaceutically acceptable salt, ester, stereoisomer, tautomer, polymorph, solvate, *N*-oxide, isotopically labeled compound, metabolite, or prodrug thereof according to claim 12 or 13, and one or more pharmaceutically acceptable auxiliary materials.

17. Use of the immune-stimulating antibody conjugate according to any one of claims 1-9, the drug-linker compound or the pharmaceutically acceptable salt, ester, stereoisomer, tautomer, polymorph, solvate, *N-*oxide, isotopically labeled compound, metabolite, or prodrug thereof according to claim 10 or 11, the compound or the pharmaceutically acceptable salt, ester, stereoisomer, tautomer, polymorph, solvate, *N-*oxide, isotopically labeled compound, metabolite, or prodrug thereof according to claim 11 or 12, or the pharmaceutical composition according to claim 15 or 16 in the preparation of a medicament for treating and/or preventing cancer (e.g., HER2-positive cancer, such as HER2-positive gastric cancer, breast cancer, or non-small cell lung cancer).

18. The immune-stimulating antibody conjugate according to any one of claims 1-9, the drug-linker compound or the pharmaceutically acceptable salt, ester, stereoisomer, tautomer, polymorph, solvate, *N-*oxide, isotopically labeled compound, metabolite, or prodrug thereof according to claim 10 or 11, the compound or the pharmaceutically acceptable salt, ester, stereoisomer, tautomer, polymorph, solvate, *N-*oxide, isotopically labeled compound, metabolite, or prodrug thereof according to claim 12 or 13, or the pharmaceutical composition according to claim 15 or 16, for use in the treatment and/or prevention of cancer (e.g., HER2-positive cancer, such as HER2-positive gastric cancer, breast cancer, or non-small cell lung cancer).

19. A method for treating and/or preventing cancer (e.g., HER2-positive cancer, such as HER2-positive gastric cancer, breast cancer, or non-small cell lung cancer), comprising administering to an individual in need thereof a therapeutically and/or prophylactically effective amount of the immune-stimulating antibody conjugate according to any one of claims 1-9, the drug-linker compound or the pharmaceutically acceptable salt, ester, stereoisomer, tautomer, polymorph, solvate, *N*-oxide, isotopically labeled compound, metabolite, or prodrug thereof according to claim 10 or 11, the compound or the pharmaceutically acceptable salt, ester, stereoisomer, tautomer, polymorph, solvate, *N*-oxide, isotopically labeled compound, metabolite, or prodrug thereof according to claim 12 or 13, or the pharmaceutical composition according to claim 15 or 16.
